# EUROPÄISCHE PATENTSCHRIFT

(11) **EP 2 307 520 B1**
(45) Veröffentlichungstag und Bekanntmachung des Hinweises auf die Patenterteilung: **11.11.2015**
(21) Anmeldenummer: 09776787.5
(22) Anmeldetag: 19.06.2009
(51) Int. Cl.: C09K 11/06, H01L 51/50, C07D 235/20, C07D 251/24, C07D 251/28, C07D 519/00, C07D 209/86

(54) **MATERIALIEN FÜR ORGANISCHE ELEKTROLUMINESZENZVORRICHTUNGEN**
MATERIALS FOR ORGANIC ELECTROLUMINESCENCE DEVICES
MATÉRIAUX POUR DISPOSITIFS ÉLECTROLUMINESCENTS ORGANIQUES

(30) Priorität: 18.07.2008 DE 102008033943
(43) Veröffentlichungstag der Anmeldung: 13.04.2011
(73) Patentinhaber: Merck Patent GmbH, 64293 Darmstadt (DE)
(72) Erfinder: STOESSEL, Philipp, 60487 Frankfurt (DE); HEIL, Holger, 60389 Frankfurt (DE); JOOSTEN, Dominik, 60487 Frankfurt (DE); PFLUMM, Christof, 60316 Frankfurt (DE); GERHARD, Anja, 63329 Egelsbach (DE); BUESING, Arne, 65929 Frankfurt am Main (DE); KAISER, Joachim, 64293 Darmstadt (DE)
(86) Internationale Anmeldenummer: PCT/EP2009/004448
(87) Internationale Veröffentlichungsnummer: WO 2010/006680

(56) Entgegenhaltungen:
- WO-A-2004/093207
- CN-A- 101 096 357
- JP-A- 2002 093 606
- US-A1- 2007 077 453

## Beschreibung

Die vorliegende Erfindung betrifft organische Halbleiter und deren Verwendung in organischen elektronischen Vorrichtungen.

Organische Halbleiter werden für eine Reihe verschiedenartiger elektronischer Anwendungen entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings sind für die Verwendung dieser Verbindungen in hochwertigen und langlebigen Displays noch weitere Verbesserungen wünschenswert. So gibt es insbesondere bei der Lebensdauer und der Effizienz organischer Elektrolumineszenzvorrichtungen derzeit noch Verbesserungsbedarf. Weiterhin ist es erforderlich, dass die Verbindungen eine hohe thermische Stabilität und eine hohe Glasübergangstemperatur aufweisen und sich unzersetzt sublimieren lassen.

Gerade auch bei phosphoreszierenden Elektrolumineszenzvorrichtungen sind noch deutliche Verbesserungen der Eigenschaften, insbesondere der Lebensdauer wünschenswert.

Es besteht daher weiterhin Bedarf an verbesserten Materialien, beispielsweise Hostmaterialien für fluoreszierende und phosphoreszierende Emitter, aber auch bei Ladungstransportmaterialien, also Loch- und Elektronentransportmaterialien, und Ladungsblockiermaterialien sind weitere Verbesserungen erforderlich. Gerade die Eigenschaften dieser Materialien sind häufig für die Lebensdauer und Effizienz der organischen Elektrolumineszenzvorrichtung verantwortlich. Insbesondere auch im Bereich der phosphoreszierenden OLEDs besteht noch deutlicher Verbesserungsbedarf.

Überraschend wurde gefunden, dass Benzophenonderivate und Diphenylmethanderivate sowie entsprechende heterocyclische Derivate, welche an den Phenylgruppen bzw. den entsprechenden heterocyclischen Gruppen jeweils in 3,5-Position durch ausgesuchte Substituenten substituiert sind, sich sehr gut für die Verwendung in organischen Elektrolumineszenzvorrichtungen eignen und dort zu deutlichen Verbesserungen gegenüber dem Stand der Technik führen. Diese Verbindungen und deren Anwendung in organischen elektronischen Vorrichtungen sind daher der Gegenstand der vorliegenden Erfindung. Je nach Substitution an den Phenylgruppen und je nachdem, ob es sich um ein Benzophenonderivat oder ein Diphenylmethanderivat handelt, eignen sich die erfindungsgemäßen Verbindungen insbesondere als Lochtransportmaterialien, Elektronen- bzw. Exzitonenblockiermaterialien, Matrixmaterialien für fluoreszierende oder phosphoreszierende Verbindungen, Lochblockiermaterialien oder Elektronentransportmaterialien. Mit den erfindungsgemäßen Materialien ist eine deutliche Steigerung der Lebensdauer und eine leichte Verbesserung der Effizienz der organischen elektronischen Vorrichtung im Vergleich zu Materialien gemäß dem Stand der Technik möglich. Weiterhin weisen diese Verbindungen eine hohe thermische Stabilität auf.

In der WO 04/093207 werden Diarylketonderivate als Matrixmaterialien für phosphoreszierende Elektrolumineszenzvorrichtungen offenbart. Darin werden als besonders bevorzugte Materialien Ketoverbindungen, welche mit Spirobifluoren substituiert sind, genannt. Benzophenonderivate, welche jeweils in 3,5-Position an den Phenylgruppen substituiert sind, oder die entsprechenden heterocyclischen Verbindungen sind nicht offenbart. Es hat sich jedoch gezeigt, dass gerade dieses Substitutionsmuster bei Anwendung in organischen elektronischen Vorrichtungen zu besonders guten Ergebnissen führt.

US 2007/077453 offenbart Carbonylverbindungen für die Verwendung in organischen Elektrolumineszenzvorrichtungen. Unter anderem wird als Beispielstruktur (56) ein substituiertes Bispyridylketon offenbart, wobei jeweils eine Phenylgruppe und eine Triphenylsilylgruppe als Substituenten am Pyridin vorhanden sind. US 2007/077453 offenbart keine Verbindungen, in denen die Gruppen, die direkt an die Carbonylgruppe gebunden sind, Phenylgruppen oder Triazinylgruppen sind.

Gegenstand der Erfindung sind somit Verbindungen gemäß Formel (1), 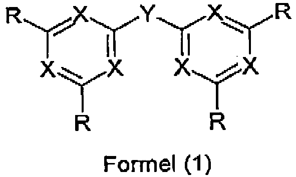 wobei für die verwendeten Symbole gilt:
- Y: ist C=O oder C(R¹)₂;
- X: ist bei jedem Auftreten gleich oder verschieden CR² oder N, wobei alle Symbole X in einem Cyclus entweder für CR² stehen oder wobei alle Symbole X in einem Cyclus für N stehen;
- R: ist bei jedem Auftreten gleich oder verschieden ein aromatisches oder heteroaromatisches Ringsystem mit 5 bis 30 aromatischen Ringatomen, welches durch einen oder mehrere Reste R³ substituiert sein kann, oder eine Gruppe N(Ar)₂, C(=O)Ar oder P(O)(Ar)2;
- Ar: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R³ substituiert sein kann; dabei können auch zwei Reste Ar, welche an dasselbe Stickstoff- oder Phosphoratom binden, durch eine Einfachbindung oder eine Brücke, ausgewählt aus B(R⁴), C(R⁴)₂, Si(R⁴)₂, C=O, C=NR⁴, C=C(R⁴)₂, O, S, S=O, SO₂, N(R⁴), P(R⁴) und P(=O)R⁴, miteinander verknüpft sein;
- R¹: ist bei jedem Auftreten gleich oder verschieden H oder eine lineare Alkylgruppe mit 1 bis 20 C-Atomen oder eine verzweigte oder cyclische Alkylgruppe mit 3 bis 20 C-Atomen; dabei können mehrere Reste R¹ miteinander ein Ringsystem bilden;
- R²: ist H;
- R³: ist bei jedem Auftreten gleich oder verschieden H, D, F, Cl, Br, I, CHO, N(Ar)₂, C(=O)Ar, P(=O)(Ar)₂, S(=O)Aᵣ, S(=O)₂Ar, CR²=CR²Ar, CN, NO₂, Si(R⁴)₃, B(OR⁴)₂, B(R⁴)₂, B(N(R⁴)₂)₂, OSO₂R⁴, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R⁴ substituiert sein kann, wobei eine oder mehrere nicht benachbarte CH₂-Gruppen durch R⁴C=CR⁴, C=C , Si(R⁴)₂, Ge(R⁴)₂, Sn(R⁴)₂, C=O, C=S, C=Se, C=NR⁴, P(=O)(R⁴), SO, SO₂, NR⁴, O, S oder CONR⁴ ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 60 aromatischen Ringatomen, die durch einen oder mehrere Reste R⁴ substituiert sein kann, oder eine Kombination dieser Systeme; dabei können zwei oder mehrere benachbarte Substituenten R³ auch miteinander ein mono- oder polycyclisches, aliphatisches oder aromatisches Ringsystem bilden;
- R⁴: ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R⁴ auch miteinander ein mono- oder polycyclisches, aliphatisches oder aromatisches Ringsystem bilden.
dabei sind die folgenden Verbindungen von der Erfindung ausgenommen: 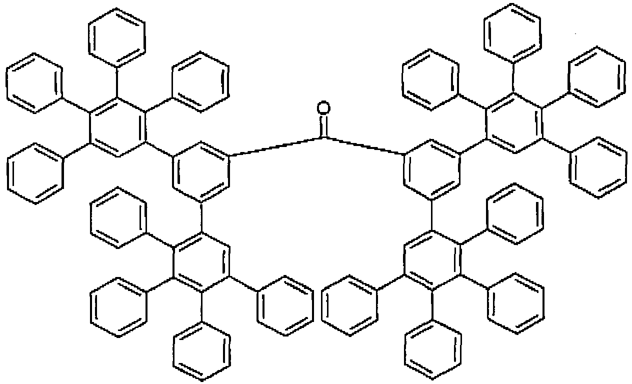 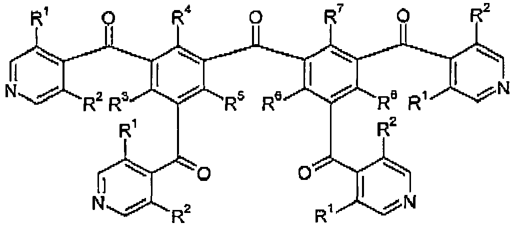 wobei für die verwendeten Symbole R¹ bis R⁸ gilt: R¹ bis R⁸ sind unabhängig voneinander und stehen für ein Wasserstoffatom, eine Methylgruppe, eine Ethylgruppe, eine n-Propylgruppe, eine Isopropylgruppe, eine n-Butylgruppe, eine Isobutylgruppe, eine sekundäre Butylgruppe oder eine tertiäre Butylgruppe.

Bevorzugt weisen die Verbindungen gemäß Formel (1) eine Glasübergangstemperatur T_{G} von größer als 70 °C auf, besonders bevorzugt größer als 90 °C.

Eine Arylgruppe im Sinne dieser Erfindung enthält mindestens 6 C-Atome; eine Heteroarylgruppe im Sinne dieser Erfindung enthält mindestens 2 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S, Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, etc., verstanden.

Ein aromatisches Ringsystem im Sinne dieser Erfindung enthält mindestens 6 C-Atome im Ringsystem. Ein heteroaromatisches Ringsystem im Sinne dieser Erfindung enthält mindestens 2 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, Benzophenon, etc. als aromatische Ringsysteme im Sinne dieser Erfindung verstanden werden. Ebenso werden unter einem aromatischen bzw. heteroaromatischen Ringsystem Systeme verstanden, in denen mehrere Aryl- bzw. Heteroarylgruppen durch Einfachbindungen miteinander verknüpft sind, beispielsweise Biphenyl, Terphenyl oder Bipyridin.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, tert-Pentyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Benzanthracen, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Benzofluoren, Dibenzofluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetra-azaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausführungsform der Erfindung stehen alle Symbole X in einem Cyclus entweder für CR², oder alle Symbole X in einem Cyclus stehen für N. Jeder der beiden aromatischen Cyclen in Formel (1) steht also bevorzugt entweder für eine 3,5-substituierte Phenylgruppe oder für eine 4,6-substituierte Triazingruppe. Besonders bevorzugt stehen alle Symbole X für CR².

In einer weiteren bevorzugten Ausführungsform der Erfindung steht das Symbol Y für C=O oder C(R¹)₂ und R¹ steht gleich oder verschieden bei jedem Auftreten für H, F, eine lineare Alkylgruppe mit 1 bis 10 C-Atomen, insbesondere mit 1 bis 6 C-Atomen, oder eine verzweigte oder cyclische Alkylgruppe mit 3 bis 10 C-Atomen, insbesondere mit 3 bis 6 C-Atomen; dabei können zwei Alkylreste R¹ miteinander-ein Ringsystem bilden. Besonders bevorzugt steht das Symbol Y für C=O oder C(R¹)₂ und R¹ steht für H, D, F oder Methyl. Dabei gilt, dass wenn eine oder mehrere Gruppen R, insbesondere alle Gruppen R, für ein aromatisches oder heteroaromatisches Ringsystem stehen, R¹ besonders bevorzugt für C=O steht. Wenn eine oder mehrere Gruppen R, insbesondere alle Gruppen R, für N(Ar)₂ stehen, steht Y besonders bevorzugt für C(R¹)₂, insbesondere für CH₂ oder C(CH₃)₂.

In einer weiteren bevorzugten Ausführungsform der Erfindung steht das Symbol R gleich oder verschieden bei jedem Auftreten für ein aromatisches oder heteroaromatisches Ringsystem mit 5 bis 30 aromatischen Ringatomen, insbesondere ein aromatisches Ringsystem mit 6 bis 30 aromatischen Ringatomen, welches jeweils durch einen oder mehrere Reste R³ substituiert sein kann, oder für eine Gruppe N(Ar)₂, C(=O)Ar oder P(=O)Ar₂. Ganz besonders bevorzugt steht R für ein aromatisches Ringsystem mit 6 bis 30 aromatischen Ringatomen, welches durch einen oder mehrere Reste R³ substituiert sein kann.

Wenn die Gruppe R für ein aromatisches oder heteroaromatisches Ringsystem steht, so ist dieses bevorzugt ausgewählt aus den Gruppen Phenyl, o-Biphenyl, m-Biphenyl, p-Biphenyl, o-Terphenyl, m-Terphenyl, p-Terphenyl, 3,5-(Diphenyl)phenyl, m-Quarterphenyl, 2-Fluorenyl, 2-Spirobifluorenyl, 1-Naphthyl, 2-Naphthyl, 1-, 2- oder 9-Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, 2-, 4- oder 5-Pyrimidinyl, 1,3,5-Triazinyl, insbesondere substituiert mit aromatischen Gruppen, N-Benzimidazolyl, Phenyl-N-benzimidazolyl, N-Phenylbenzimidazolyl, Phenyl-N-phenylbenzimidazolyl, Thiophen, Oxazol, Oxadiazol, Thiadiazol oder Benzthiazol. Dabei können diese Gruppen jeweils durch eine oder mehrere Substituenten R³ substituiert sein. Besonders bevorzugte aromatische oder heteroaromatische Ringsysteme R sind ausgewählt aus Strukturen der folgenden Formeln (2) bis (11), wobei die gestrichelte Bindung jeweils die Verknüpfung dieser Einheit andeutet und wobei diese Gruppen jeweils durch einen oder mehrere Reste R³ substituiert sein können:

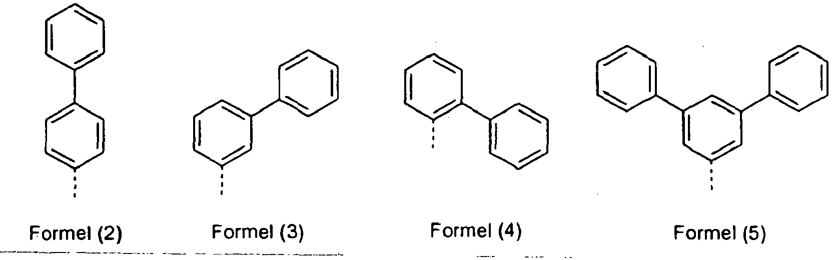

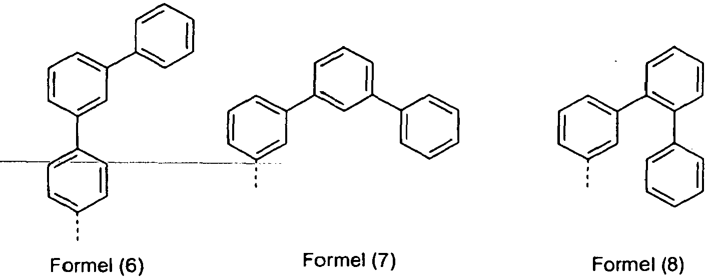

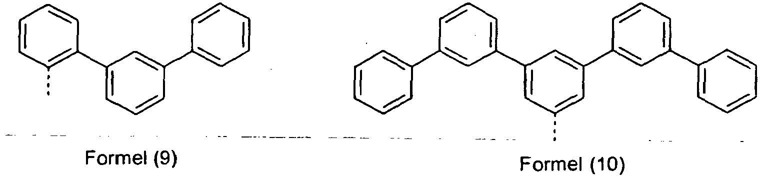

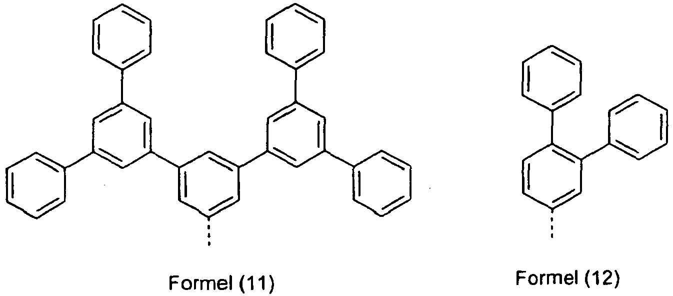

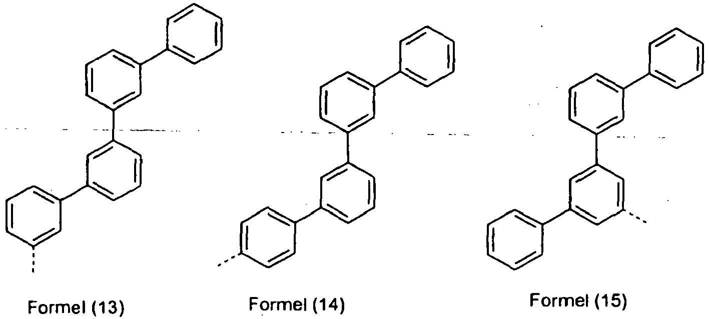

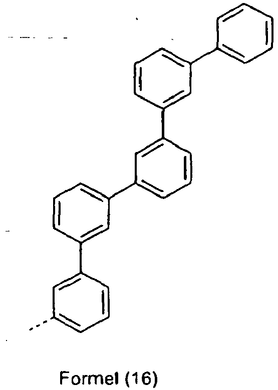

Unter den Strukturen der Formeln (2) bis (16) sind die Strukturen der Formeln (3), (5), (7), (10), (11), (13) und (15) ganz besonders bevorzugt.

Wenn der Rest R für eine Gruppe N(Ar)₂ steht, so ist diese Gruppe bevorzugt ausgewählt aus den Gruppen der Formel (17) oder der Formel (18), 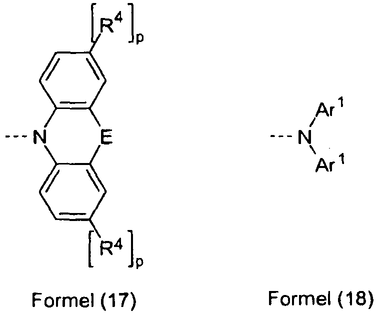 wobei R⁴ die oben aufgeführte Bedeutung hat und weiterhin gilt:
- E: steht für eine Einfachbindung, O, S, N(R⁴) oder C(R⁴)₂;
- Ar¹: ist gleich oder verschieden bei jedem Auftreten ein aromatisches oder heteroaromatisches Ringsystem mit 5 bis 24 aromatischen Ringatomen oder eine Triarylamingruppe mit 15 bis 30 aromatischen Ringatomen, welche jeweils mit einem oder mehreren Resten R⁴ substituiert sein kann, bevorzugt eine Aryl- oder Heteroarylgruppe mit 6 bis 14 aromatischen Ringatomen oder eine Triarylamingruppe mit 18 bis 30 aromatischen Ringatomen, bevorzugt mit 18 bis 22 aromatischen Ringatomen, welche jeweils mit einem oder mehreren Resten R⁴ substituiert sein kann;
- p: ist bei jedem Auftreten gleich oder verschieden 0 oder 1.
Besonders bevorzugt steht Ar¹ gleich oder verschieden bei jedem Auftreten für Phenyl, Biphenyl, 1-Naphthyl, 2-Naphthyl, 2-Spirobifluorenyl, 2-, 3- oder 4-Triphenylamin, 1- oder 2-Naphthyldiphenylamin, welches jeweils über die Naphthyl- oder die Phenylgruppe gebunden sein kann, 1- oder 2-Dinaphthyl-phenylamin, welches Jeweils über die Naphthyl- oder die Phenylgruppe gebunden sein kann, N-Carbazolyl, N-Phenyl-2-carbazolyl oder N-Phenyl-3-carbazolyl. Diese Gruppen können jeweils durch eine oder mehrere Alkylgruppen mit 1 bis 4 C-Atomen oder durch Fluor substituiert sein.

In einer bevorzugten Ausführungsform der Erfindung sind alle Symbole R in Verbindungen der Formel (1) gleich gewählt. In einer weiteren bevorzugten Ausführungsform der Erfindung sind beide Substituenten R, welche an denselben Ring binden, jeweils gleich gewählt, unterscheiden sich jedoch von den Substituenten R am anderen Ring.

Besonders bevorzugt weisen die Verbindungen gemäß Formel (1) die oben ausgeführten Bevorzugungen gleichzeitig auf. Besonders bevorzugte Ausführungsformen der Verbindungen gemäß Formel (1) sind daher die Verbindungen der Formeln (19), (20) und (21), 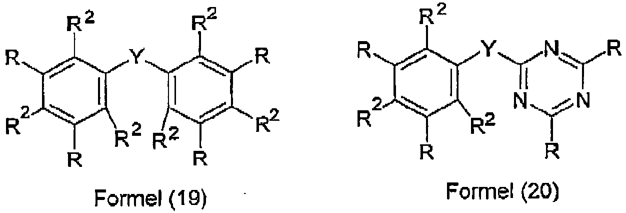 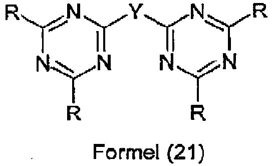 wobei Ar, R³ und R⁴ wie oben definiert sind und für die weiteren verwendeten Symbole gilt:
- Y: ist C=O oder C(R¹)₂;
- R: ist bei jedem Auftreten gleich oder verschieden ein aromatisches oder heteroaromatisches Ringsystem ausgewählt aus der Gruppe bestehend aus Phenyl, o-Biphenyl, m-Biphenyl, p-Biphenyl, o-Terphenyl, m-Terphenyl, p-Terphenyl, 3,5-(Diphenyl)phenyl, m-Quarterphenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Benzimidazolyl, Phenyl-N-benzimidazolyl, N-Phenylbenzimidazolyl oder Phenyl-N-phenylbenz-imidazolyl, insbesondere ausgewählt aus den oben abgebildeten Formeln (2) bis (16) oder eine Gruppe N(Ar)₂, bevorzugt ausgewählt aus den oben abgebildeten Formeln (17) oder (18) oder C(=O)Ar oder P(=O)Ar₂;
- R¹: ist bei jedem Auftreten gleich oder verschieden H, eine lineare Alkylgruppe mit 1 bis 10 C-Atomen, bevorzugt mit 1 bis 6 C-Atomen, insbesondere Methyl, oder eine verzweigte oder cyclische Alkylgruppe mit 3 bis 10 C-Atomen, bevorzugt mit 3 bis 6 C-Atomen; dabei können mehrere Reste R¹ miteinander ein Ringsystem bilden
- R²: ist H.

Eine besonders bevorzugte Ausführungsform der Verbindungen gemäß Formel (1) sind die Verbindungen gemäß Formel (22), 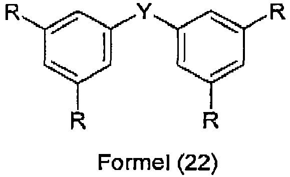 wobei Ar, R³ und R⁴ wie oben definiert sind und für die weiteren verwendeten Symbole gilt:
- Y: ist C=O, CH₂ oder C(Alkyl)₂, wobei Alkyl eine Alkylgruppe mit 1 bis 6 C-Atomen darstellt, insbesondere Methyl;
- R: ist bei jedem Auftreten gleich oder verschieden ein aromatisches Ringsystem ausgewählt aus der Gruppe bestehend aus Phenyl, o-Biphenyl, m-Biphenyl, p-Biphenyl, o-Terphenyl, m-Terphenyl, o-Terphenyl, 3,5-(Diphenyl)phenyl, m-Quarterphenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Benzimidazolyl, Phenyl-N-benzimidazolyl, N-Phenylbenzimidazolyl oder Phenyl-N-phenylbenzimidazolyl, insbesondere Gruppen der oben abgebildeten Formeln (2) bis (16) oder eine Gruppe N(Ar)₂, bevorzugt ausgewählt aus den oben abgebildeten Formeln (17) oder (18), oder C(=O)Ar oder P(=O)Ar₂.

In einer weiteren bevorzugten Ausführungsform der Erfindung steht das Symbol Ar in Verbindungen der Formel (1) bzw. der Formeln (19) bis (22) gleich oder verschieden bei jedem Auftreten für ein aromatisches Ringsystem mit 6 bis 30 aromatischen Ringatomen, insbesondere ausgewählt aus der Gruppe bestehend aus Phenyl, o-Biphenyl, m-Biphenyl, p-Biphenyl, o-Terphenyl, m-Terphenyl, p-Terphenyl, 3,5-(Diphenyl)phenyl, m-Quarterphenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Benzimidazolyl, Phenyl-N-benzimidazolyl, N-Phenylbenzimidazolyl oder Phenyl-N-phenylbenzimidazolyl.

In einer weiteren bevorzugten Ausführungsform der Erfindung steht das Symbol R³ in Verbindungen der Formel (1) bzw. der Formeln (19) bis (22) gleich oder verschieden bei jedem Auftreten für H, F, N(Ar)₂, C(=O)Ar, P(=O)(Ar)₂, S(=O)Ar, S(=O)₂Ar, CR²=CR²Ar, Si(R⁴)₃, B(OR⁴)₂, B(N(R⁴)₂)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, die jeweils mit einem oder mehreren Resten R⁴ substituiert sein kann, wobei eine oder mehrere nicht benachbarte CH₂-Gruppen durch R⁴C=CR⁴ oder C=O, NR⁴, O oder S ersetzt sein können und wobei ein oder mehrere H-Atome durch F ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, oder eine Kombination dieser Systeme; dabei können zwei oder mehrere benachbarte Substituenten R³ auch miteinander ein mono- oder polycyclisches, aliphatisches oder aromatisches Ringsystem bilden. Besonders bevorzugt steht R³ gleich oder verschieden bei jedem Auftreten für H, F, N(Ar)₂, C(=O)Ar, P(=O)(Ar)₂, Si(R⁴)₃, B(OR⁴)₂, B(N(R⁴)₂)₂, eine geradkettige Alkylgruppe mit 1 bis 6 C-Atomen, insbesondere Methyl oder Ethyl, oder eine verzweigte oder cyclische Alkylgruppe mit 3 bis 6 C-Atomen, die jeweils mit einem oder mehreren Resten R⁴ substituiert sein kann, wobei ein oder mehrere H-Atome durch F ersetzt sein können, insbesondere iso-Propyl oder tert-Butyl, oder ein aromatisches oder heteroaromatisches Ringsystem mit 5 bis 20 aromatischen Ringatomen, das jeweils durch einen oder mehrere Reste R⁴ substituiert sein kann, oder eine Kombination dieser Systeme; dabei können zwei oder mehrere benachbarte Substituenten R³ auch miteinander ein mono- oder polycyclisches, aliphatisches oder aromatisches Ringsystem bilden. Bei Verbindungen, die aus Lösung verarbeitet werden, sind auch lineare oder verzweigte Alkylgruppen mit bis zu 10 C-Atomen bevorzugt.

Beispiele für bevorzugte Verbindungen gemäß den Formeln (1) und (19) bis (22) sind die im Folgenden abgebildeten Strukturen (1) bis (250).

| | |
|---|---|
| 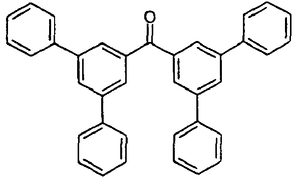 | 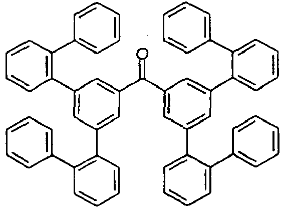 |
| (1) | (2) |
| 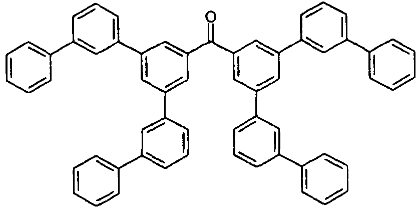 | 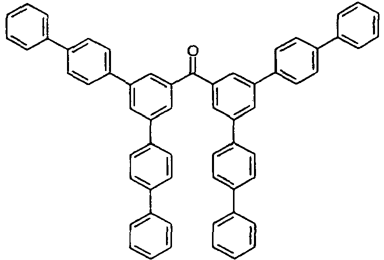 |
| (3) | (4) |
| 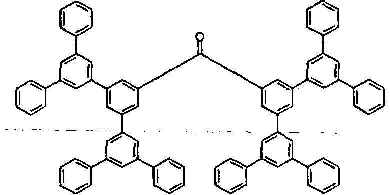 | 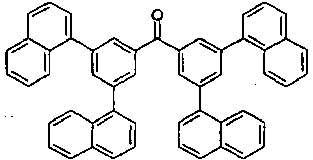 |
| (5) | (6) |
| 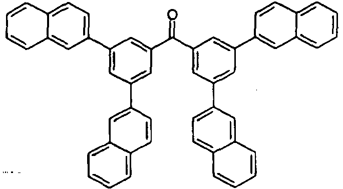 | 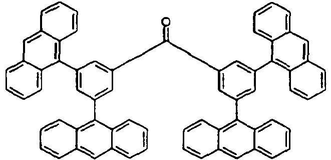 |
| (7) | (8) |
| 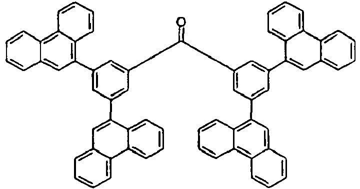 | 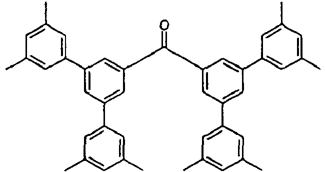 |
| (9) | (10) |
| 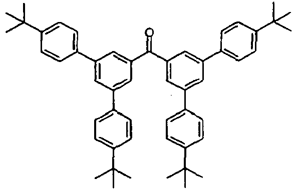 | 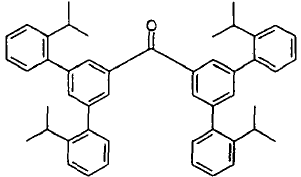 |
| (11) | (12) |
| 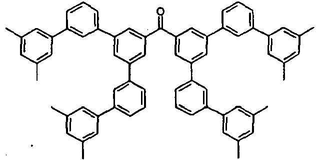 | 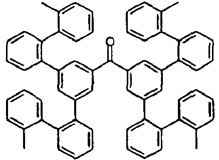 |
| (13) | (14) |
| 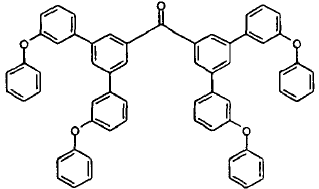 | 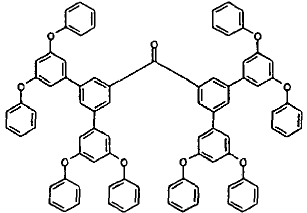 |
| (15) | (16) |
| 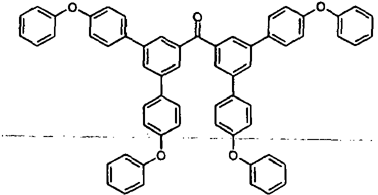 | 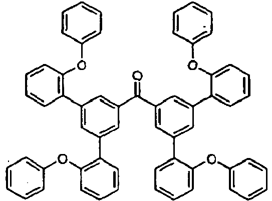 |
| (17) | (18) |
| 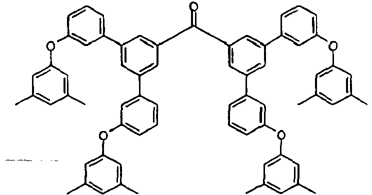 | 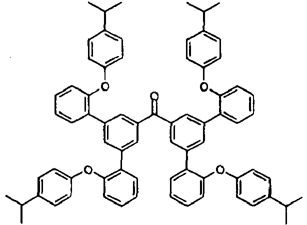 |
| (19) | (20) |
| 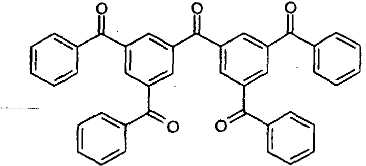 | 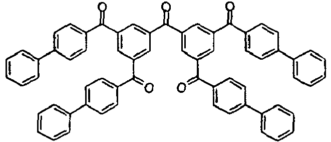 |
| (21) | (22) |
| 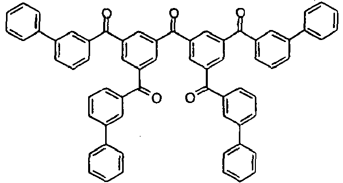 | 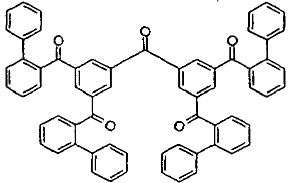 |
| (23) | (24) |
| 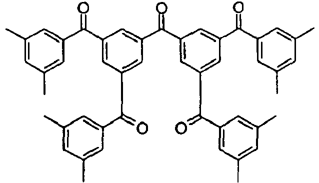 | 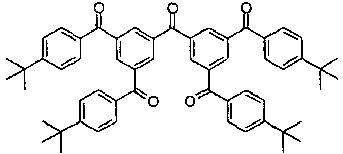 |
| (25) | (26) |
| 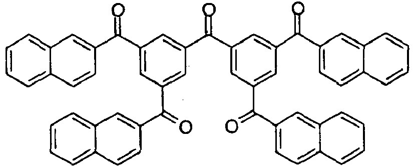 | 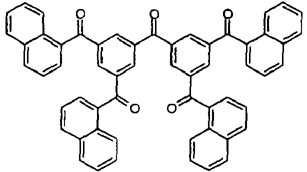 |
| (27) | (28) |
| 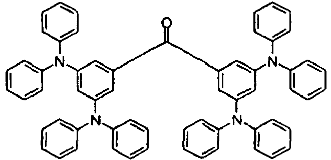 | 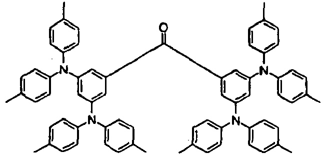 |
| (29) | (30) |
| 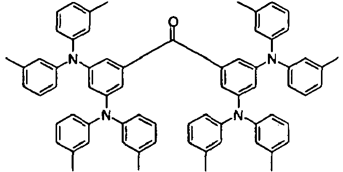 | 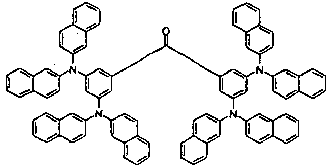 |
| (31) | (32) |
| 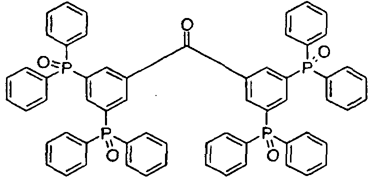 | 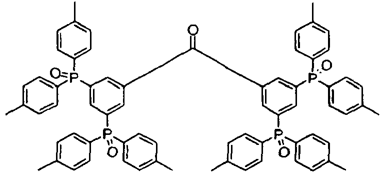 |
| (33) | (34) |
| 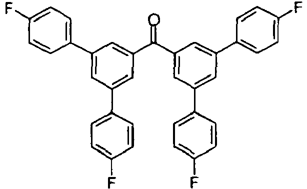 | 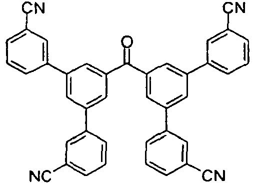 |
| (35) | (36) |
| 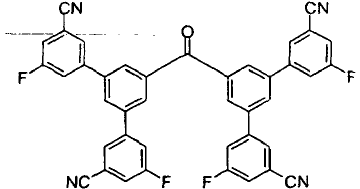 | 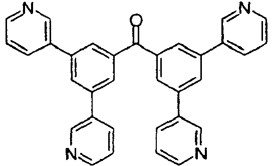 |
| (37) | (38) |
| 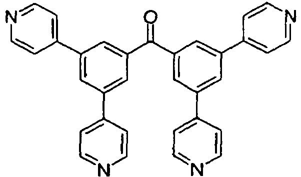 | 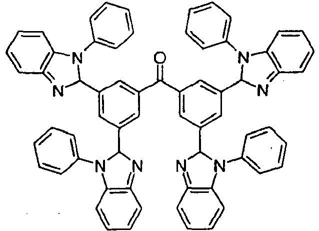 |
| (39) | (40) |
| 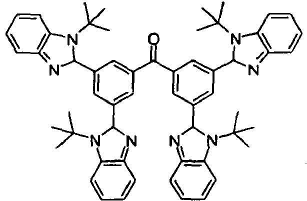 | 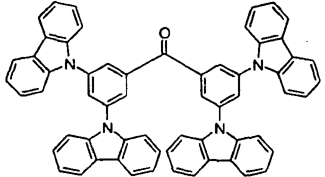 |
| (41) | (42) |
| 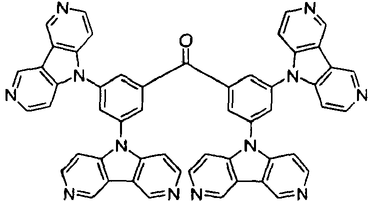 | 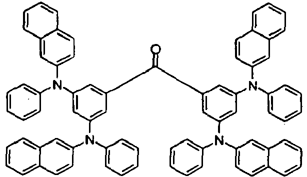 |
| (43) | (44) |
| 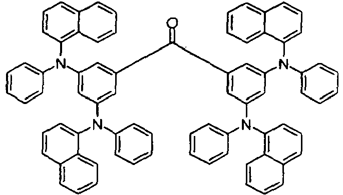 | 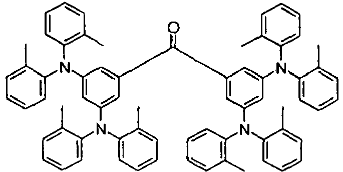 |
| (45) | (46) |
| 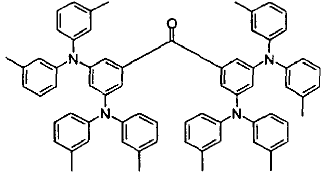 | 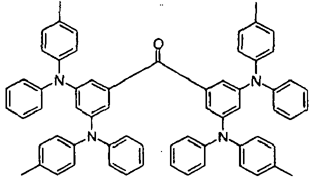 |
| (47) | (48) |
| 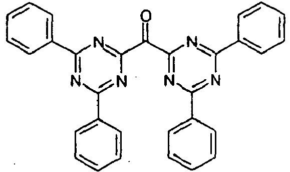 | 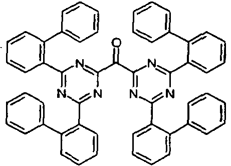 |
| (49) | (50) |
| 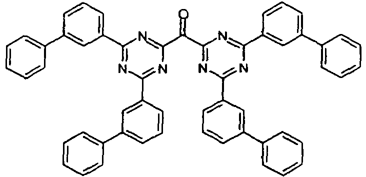 | 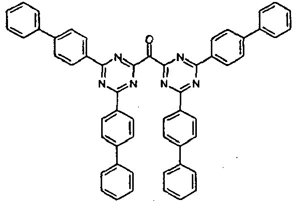 |
| (51) | (52) |
| 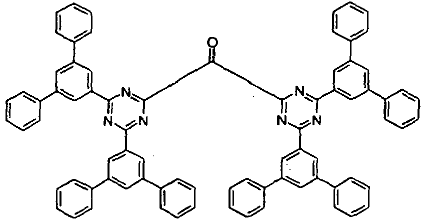 | 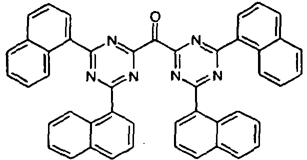 |
| (53) | (54) |
| 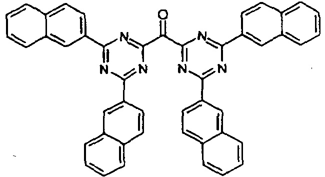 | 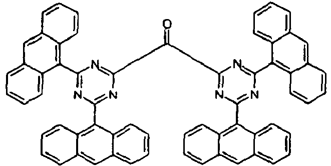 |
| (55) | (56) |
| 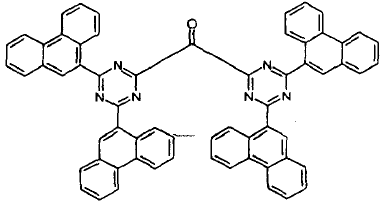 | 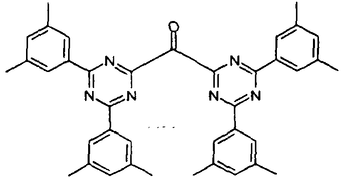 |
| (57) | (58) |
| 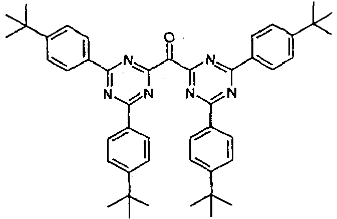 | 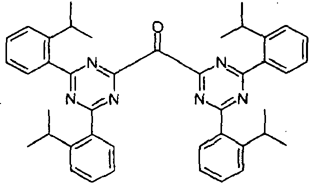 |
| (59) | (60) |
| 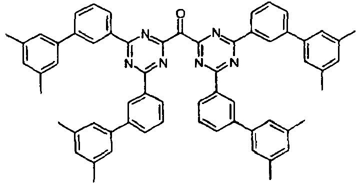 | 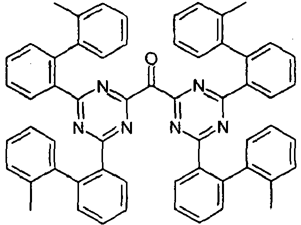 |
| (61) | (62) |
| 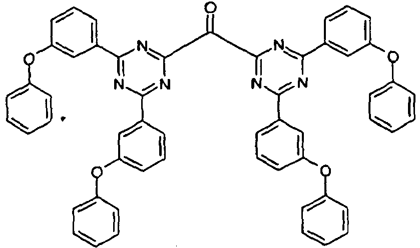 | 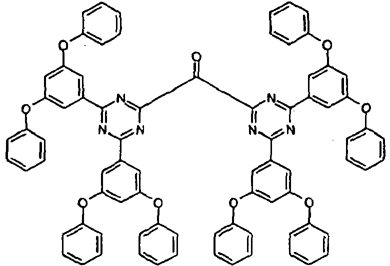 |
| (63) | (64) |
| 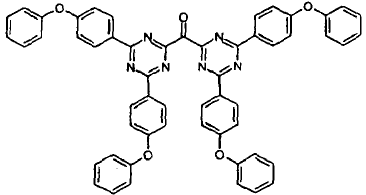 | 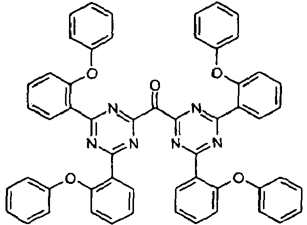 |
| (65) | (66) |
| 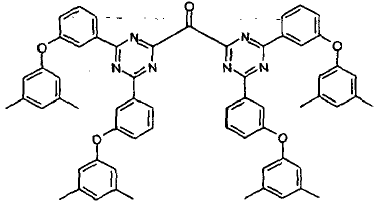 | 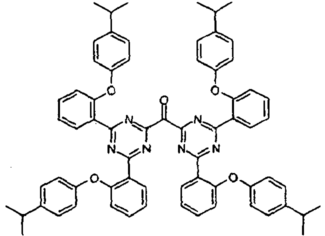 |
| (67) | (68) |
| 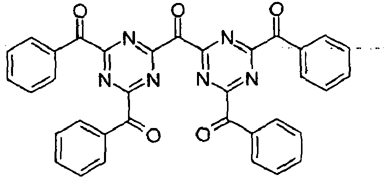 | 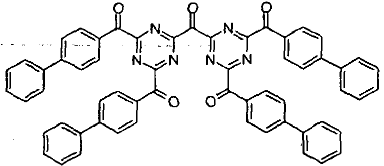 |
| (69) | (70) |
| 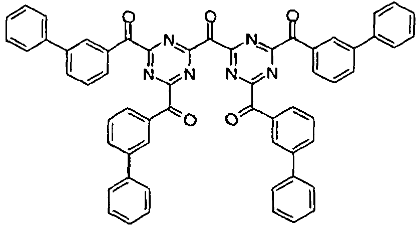 | 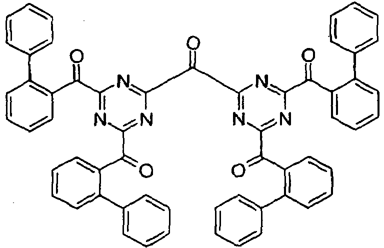 |
| (71) | (72) |
| 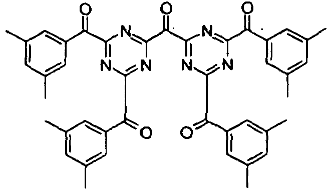 | 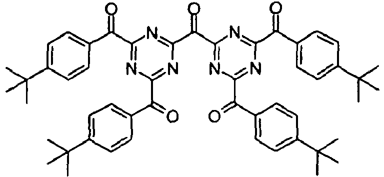 |
| (73) | (74) |
| 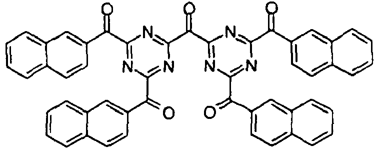 | 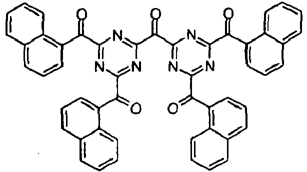 |
| (75) | (76) |
| 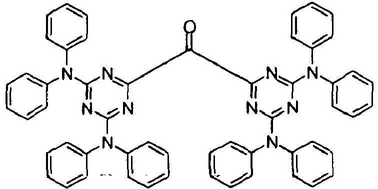 | 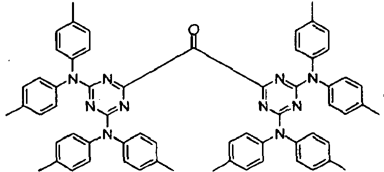 |
| (77) | (78) |
| 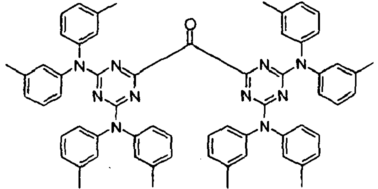 | 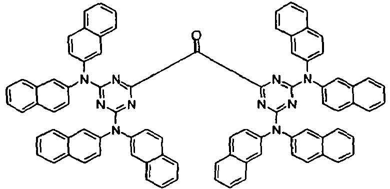 |
| (79) | (80) |
| 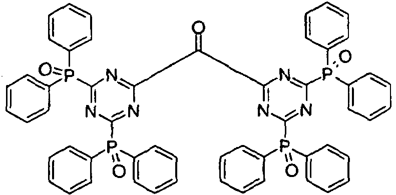 | 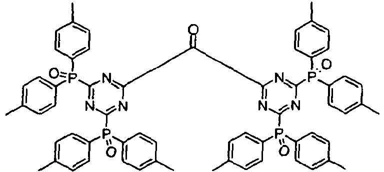 |
| (81) | (82) |
| 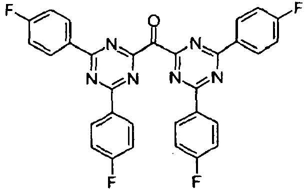 | 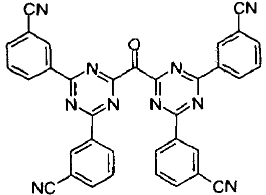 |
| (83) | (84) |
| 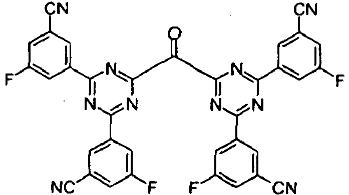 | 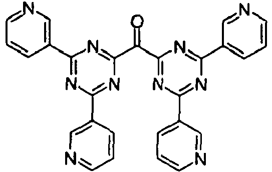 |
| (85) | (86) |
| 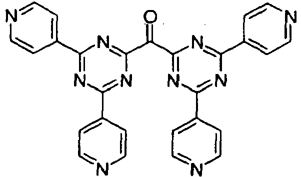 | 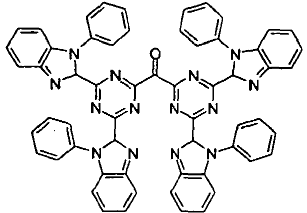 |
| (87) | (88) |
| 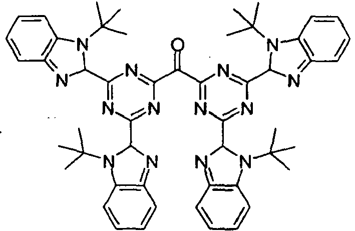 | 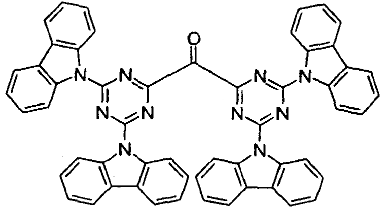 |
| (89) | (90) |
| 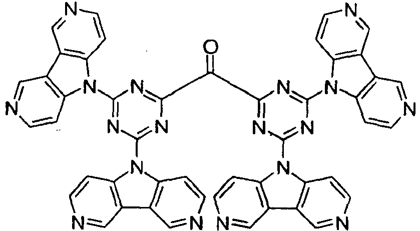 | 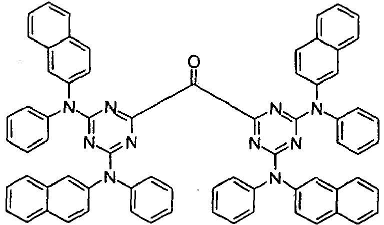 |
| (91) | (92) |
| 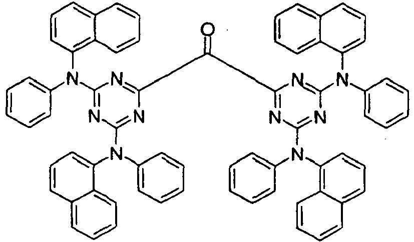 | 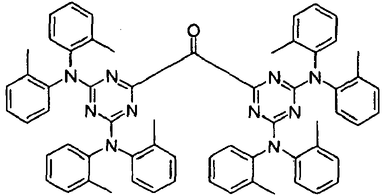 |
| (93) | (94) |
| 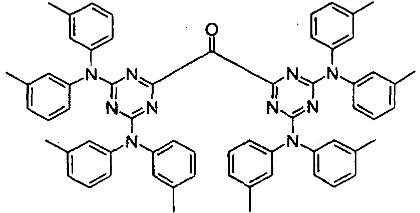 | 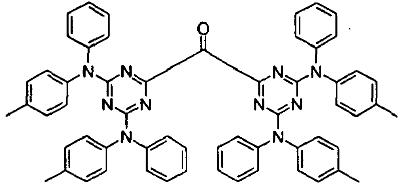 |
| (95) | (96) |
| 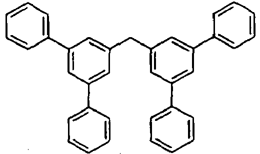 | 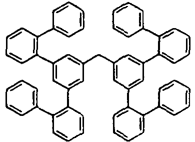 |
| (97) | (98) |
| 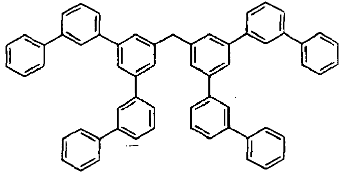 | 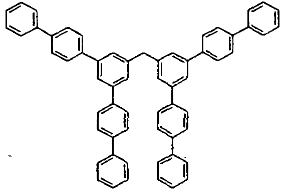 |
| (99) | (100) |
| 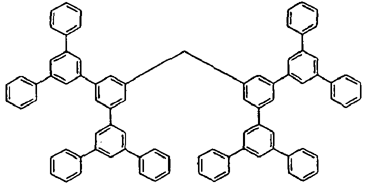 | 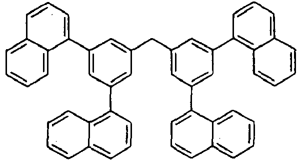 |
| (101) | (102) |
| 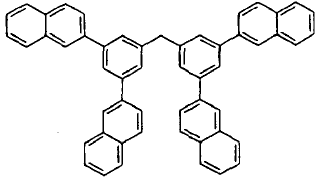 | 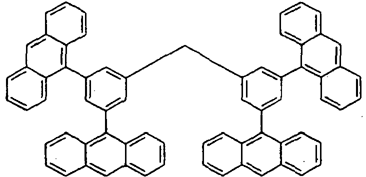 |
| (103) | (104) |
| 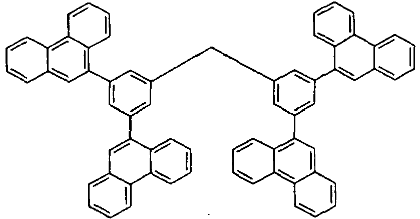 | 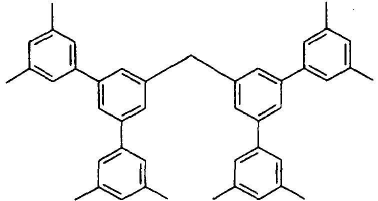 |
| (105) | (106) |
| 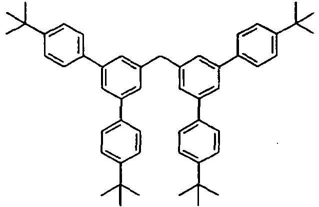 | 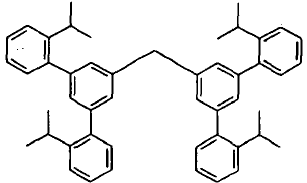 |
| (107) | (108) |
| 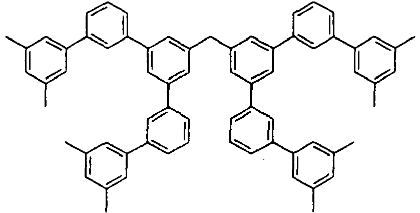 | 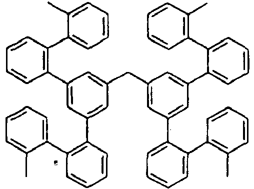 |
| (109) | (110) |
| 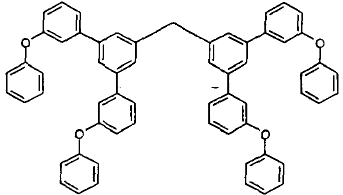 | 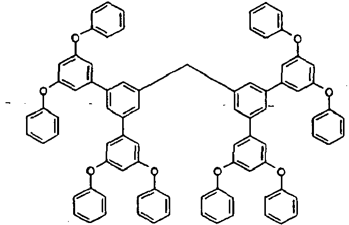 |
| (111) | (112) |
| 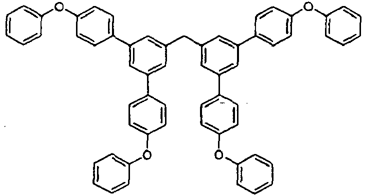 | 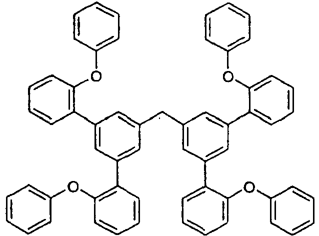 |
| (113) | (114) |
| 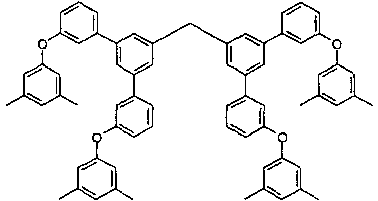 | 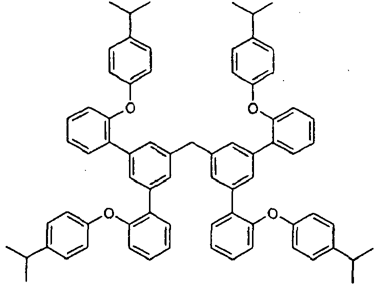 |
| (115) | (116) |
| 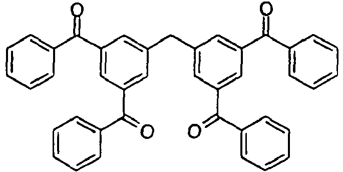 | 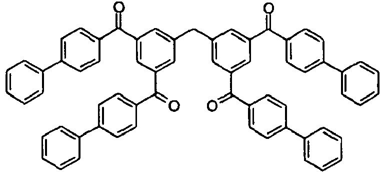 |
| (117) | (118) |
| 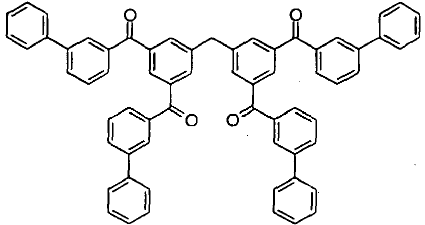 | 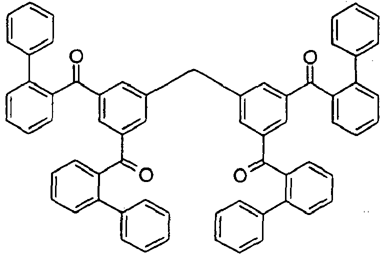 |
| (119) | (120) |
| 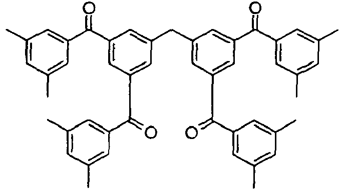 | 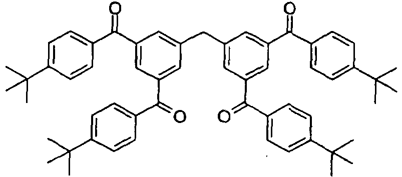 |
| (121) | (122) |
| 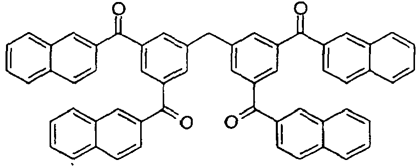 | 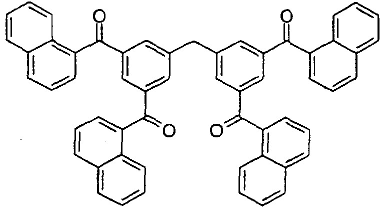 |
| (123) | (124) |
| 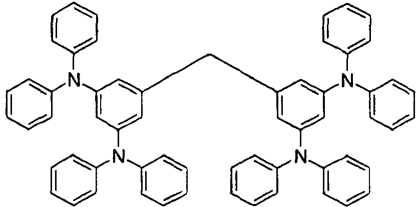 | 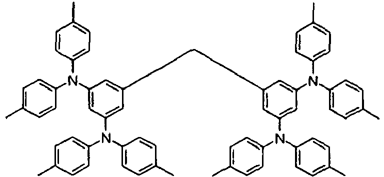 |
| (125) | (126) |
| 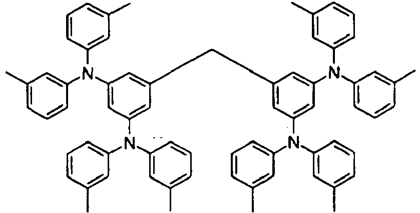 | 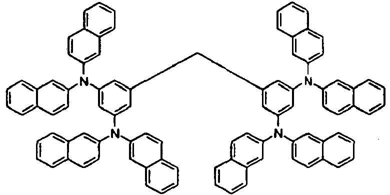 |
| (127) | (128) |
| 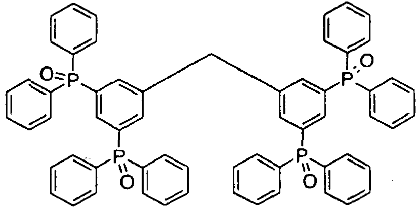 | 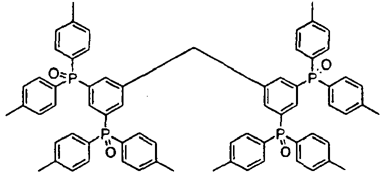 |
| (129) | (130) |
| 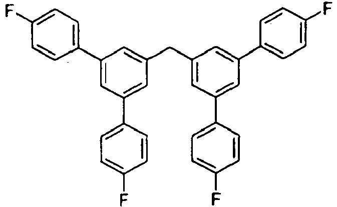 | 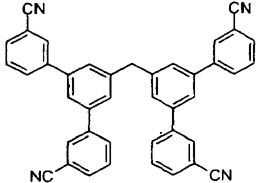 |
| (131) | (132) |
| 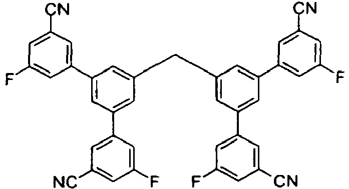 | 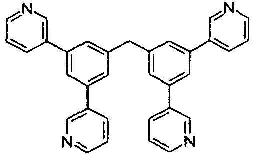 |
| (133) | (134) |
| 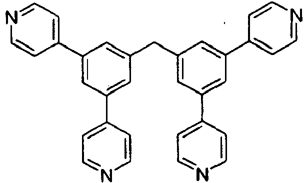 | 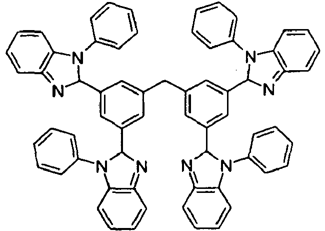 |
| (135) | (136) |
| 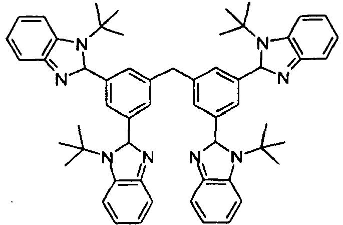 | 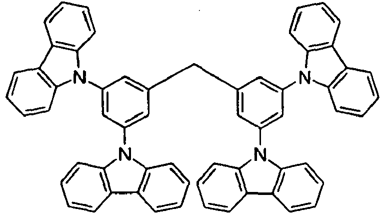 |
| (137) | (138) |
| 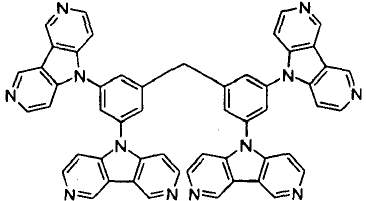 | 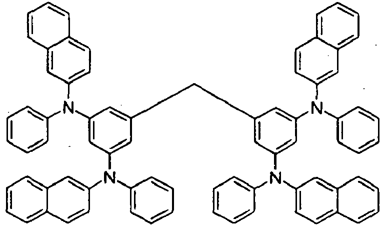 |
| (139) | (140) |
| 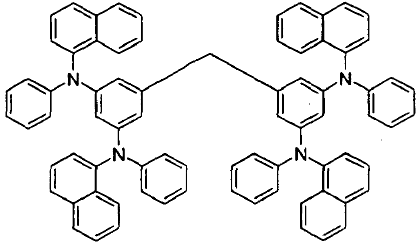 | 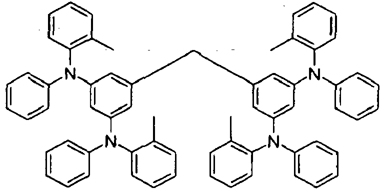 |
| (141) | (142) |
| 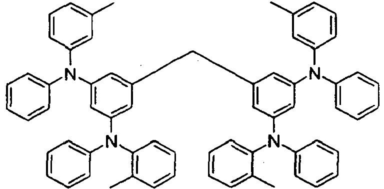 | 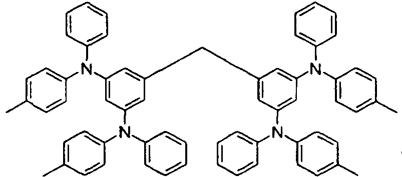 |
| (143) | (144) |
| 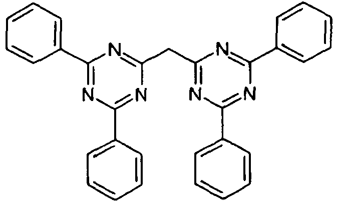 | 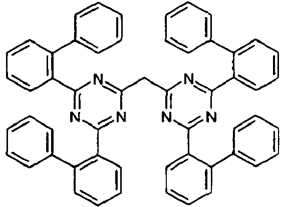 |
| (145) | (146) |
| 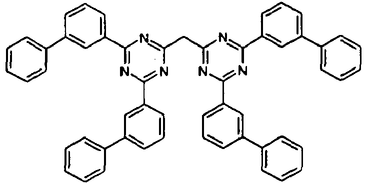 | 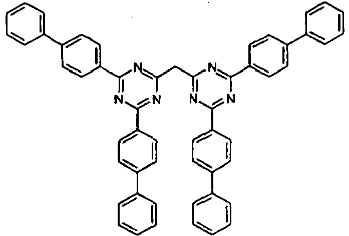 |
| (147) | (148) |
| 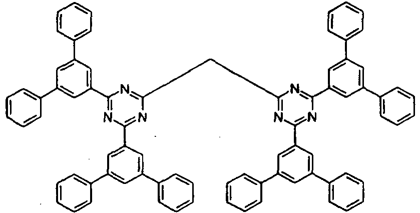 | 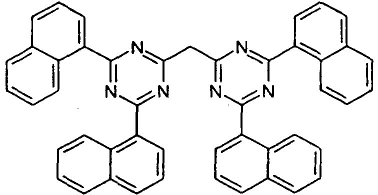 |
| (149) | (150) |
| 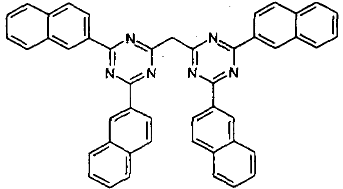 | 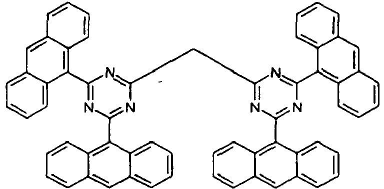 |
| (151) | (152) |
| 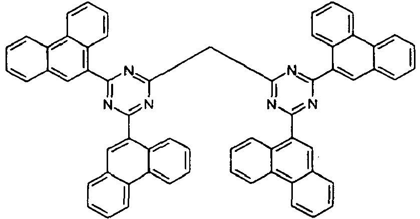 | 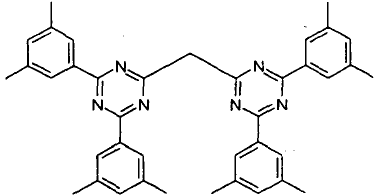 |
| (153) | (154) |
| 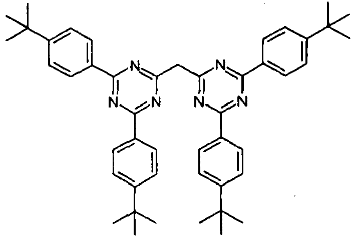 | 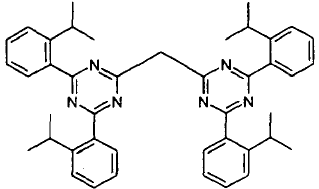 |
| (155) | (156) |
| 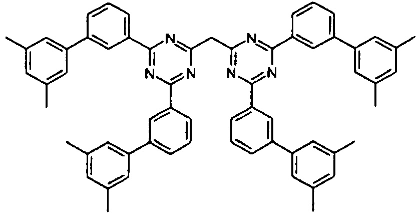 | 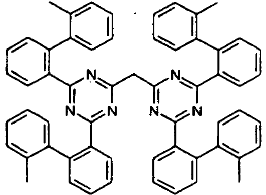 |
| (157) | (158) |
| 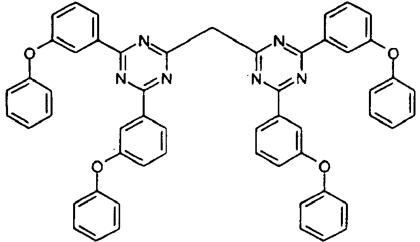 | 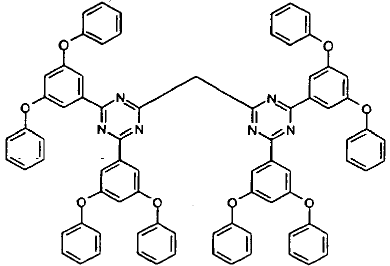 |
| (159) | (160) |
| 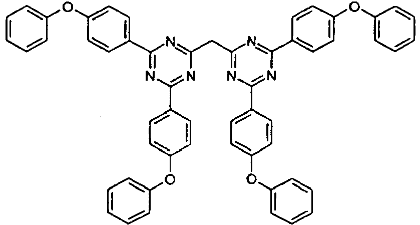 | 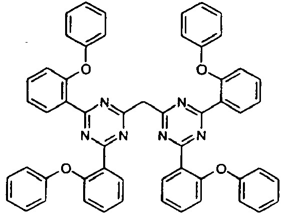 |
| (161) | (162) |
| 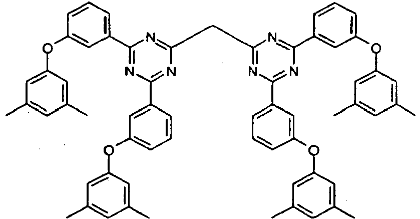 | 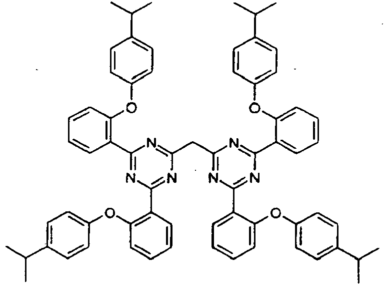 |
| (163) | (164) |
| 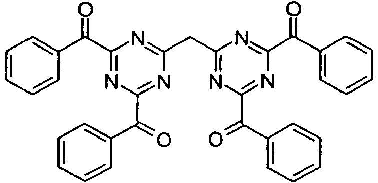 | 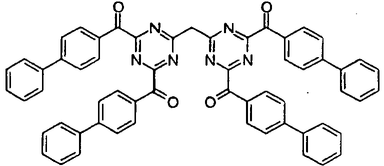 |
| (165) | (166) |
| 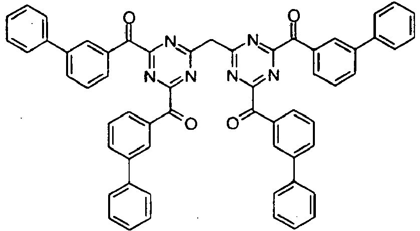 | 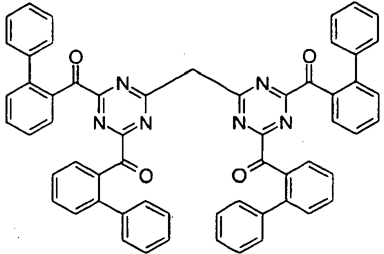 |
| (167) | (168) |
| 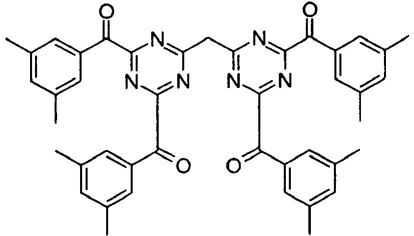 | 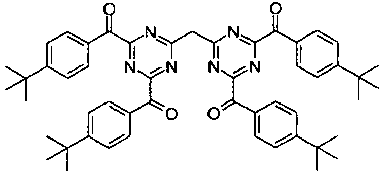 |
| (169) | (170) |
| 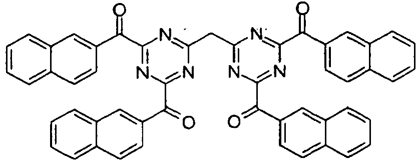 | 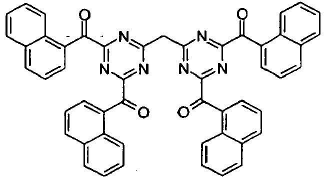 |
| (171) | (172) |
| 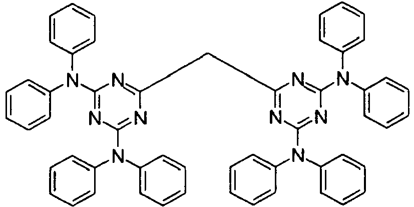 | 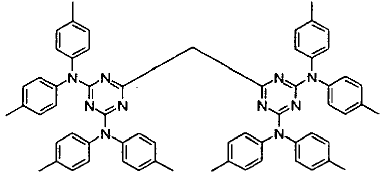 |
| (173) | (174) |
| 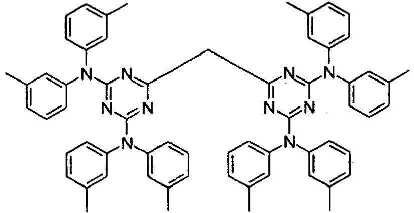 | 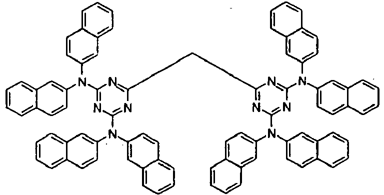 |
| (175) | (176) |
| 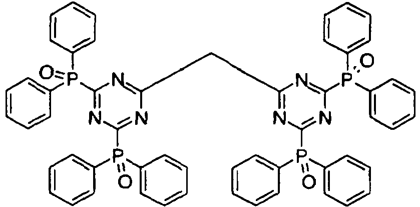 | 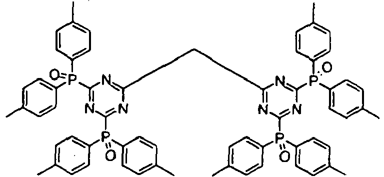 |
| (177) | (178) |
| 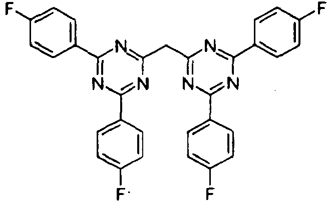 | 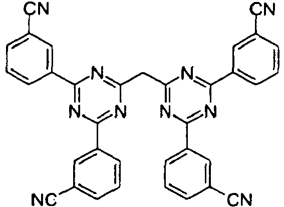 |
| (179) | (180) |
| 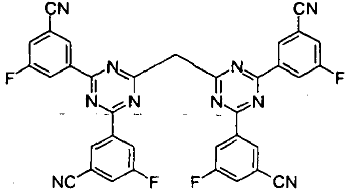 | 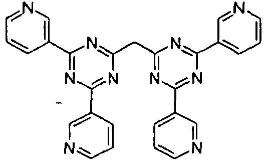 |
| (181) | (182) |
| 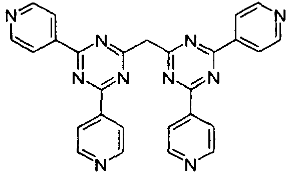 | 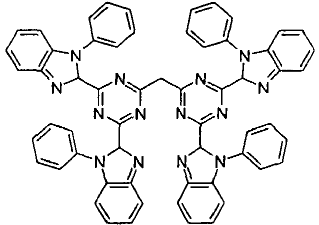 |
| (183) | (184) |
| 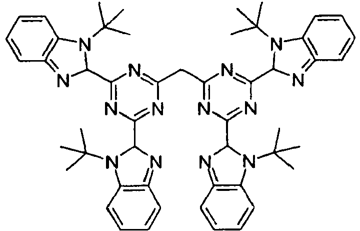 | 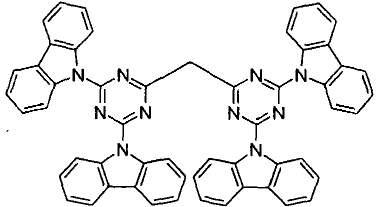 |
| (185) | (186) |
| 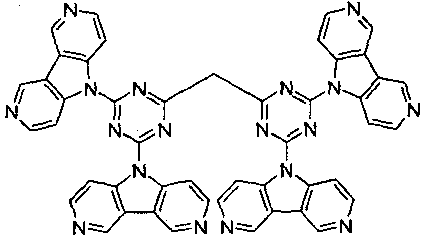 | 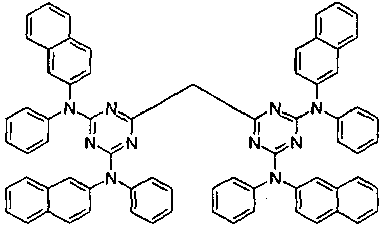 |
| (187) | (188) |
| 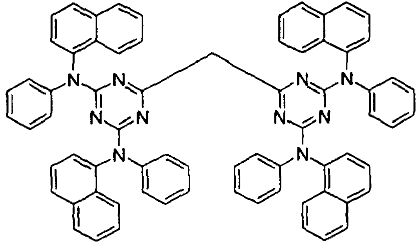 | 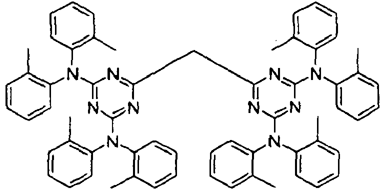 |
| (189) | (190) |
| 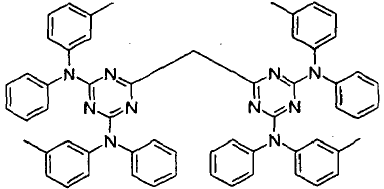 | 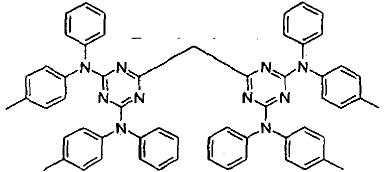 |
| (191) | (192) |
| 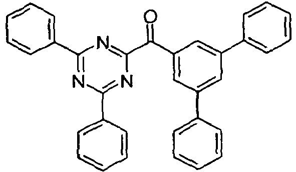 | 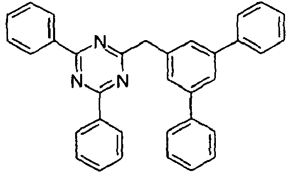 |
| (193) | (194) |
| 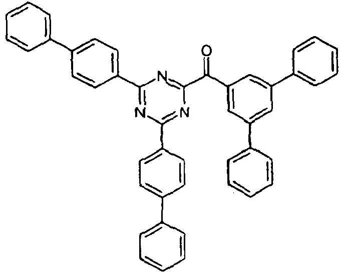 | 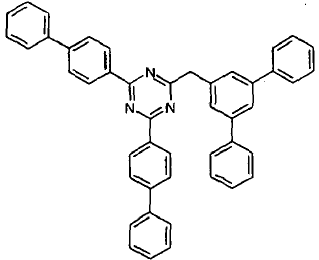 |
| (195) | (196) |
| 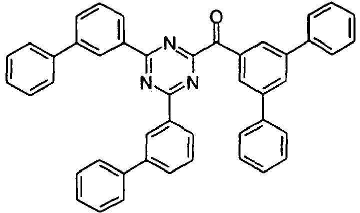 | 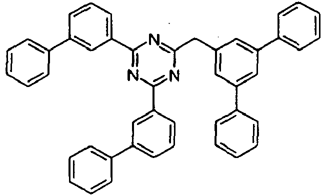 |
| (197) | (198) |
| 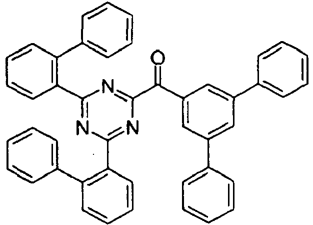 | 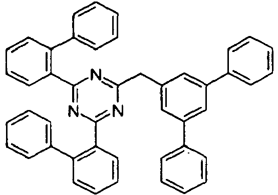 |
| (199) | (200) |
| 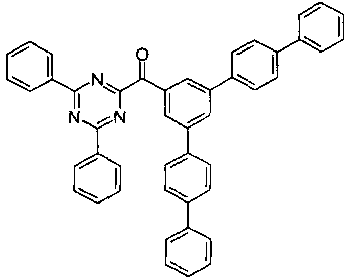 | 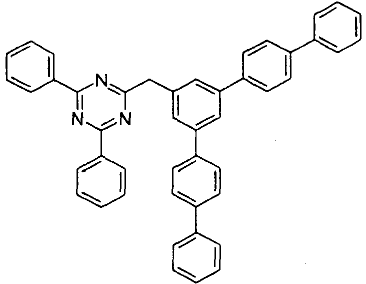 |
| (201) | (202) |
| 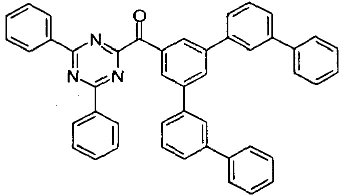 | 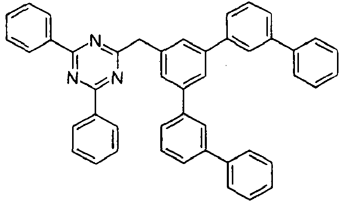 |
| (203) | (204) |
| 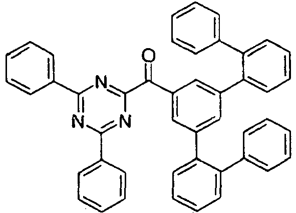 | 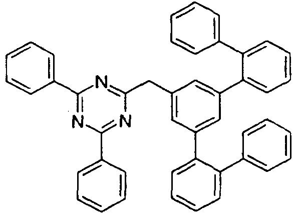 |
| (205) | (206) |
| 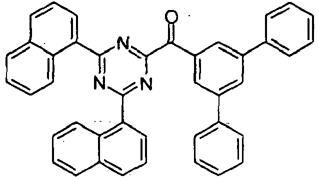 | 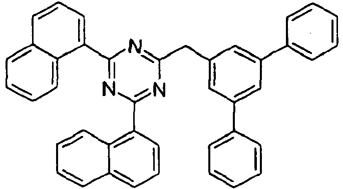 |
| (207) | (208) |
| 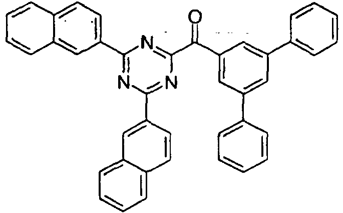 | 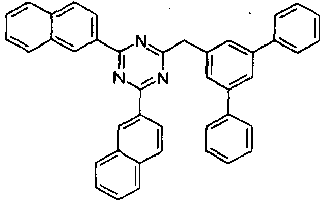 |
| (209) | (210) |
| 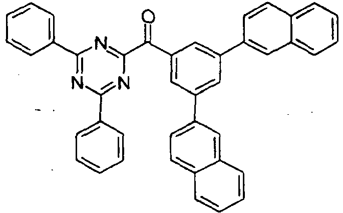 | 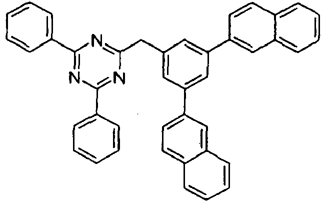 |
| (211) | (212) |
| 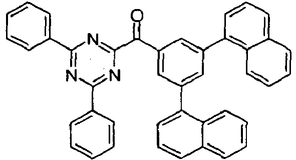 | 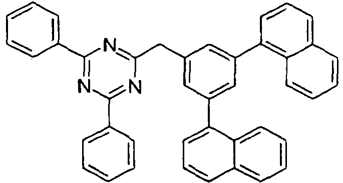 |
| (213) | (214) |
| 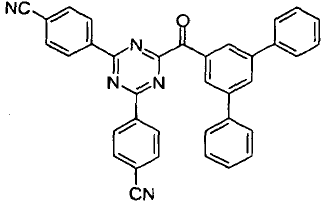 | 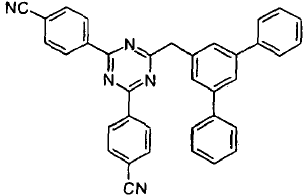 |
| (215) | (216) |
| 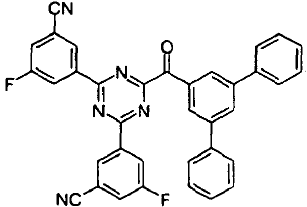 | 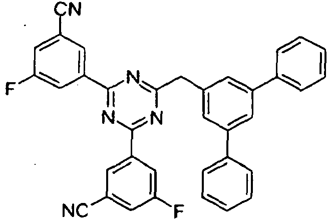 |
| (217) | (218) |
| 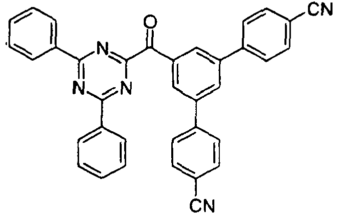 | 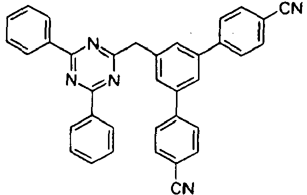 |
| (219) | (220) |
| 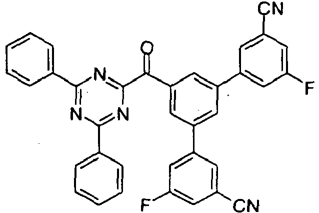 | 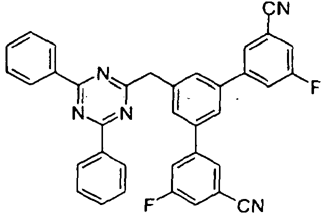 |
| (221) | (222) |
| 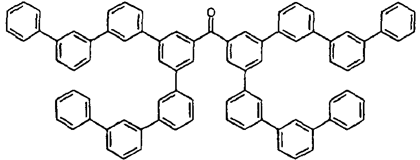 | 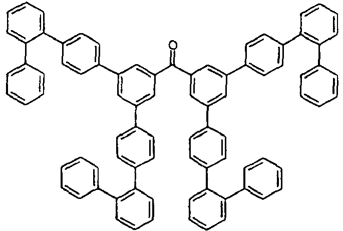 |
| (223) | (224) |
| 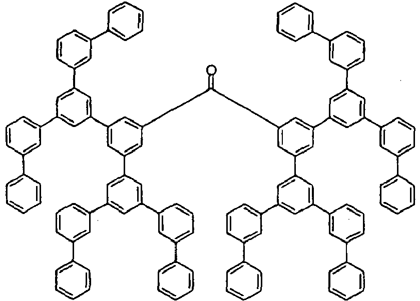 | 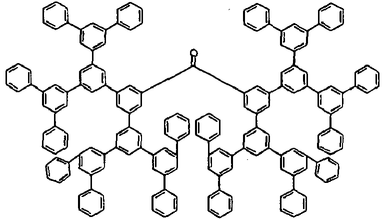 |
| (225) | (226) |
| 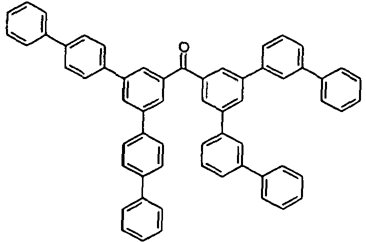 | 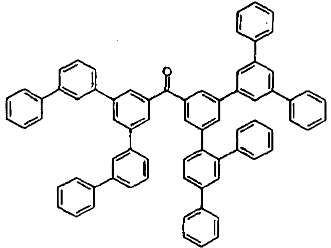 |
| (227) | (228) |
| 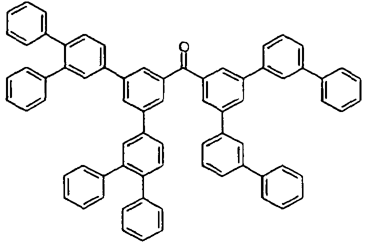 | 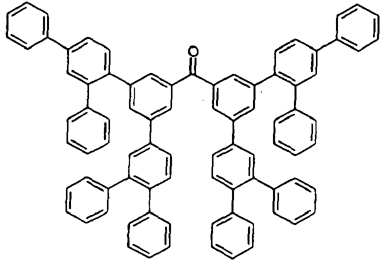 |
| (229) | (230) |
| 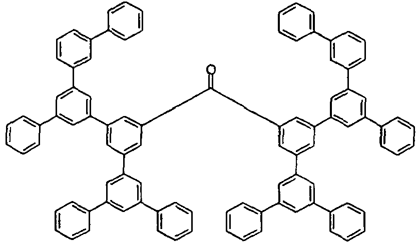 | 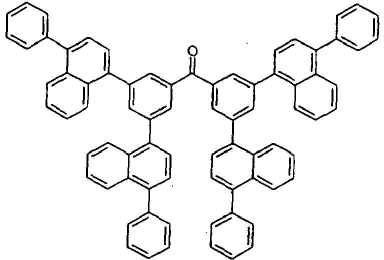 |
| (231) | (232) |
| 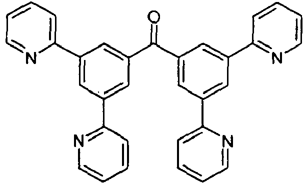 | 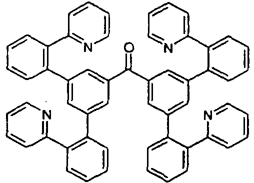 |
| (233) | (234) |
| 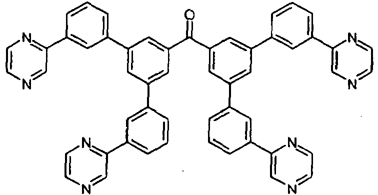 | 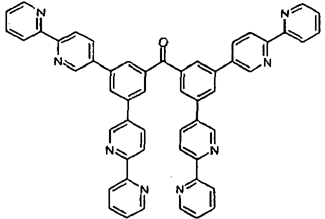 |
| (235) | (236) |
| 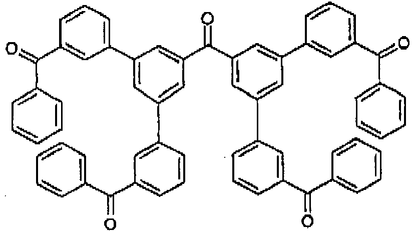 | 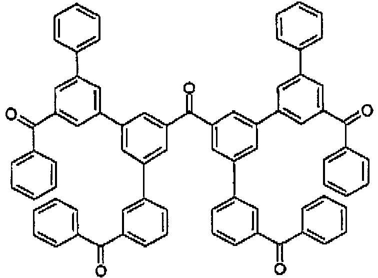 |
| (237) | (238) |
| 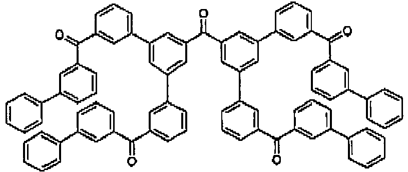 | 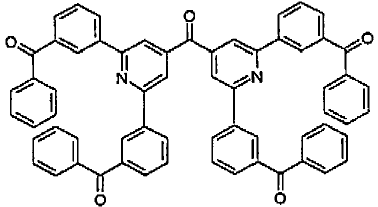 |
| (239) | (240) |
| 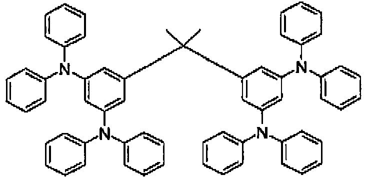 | 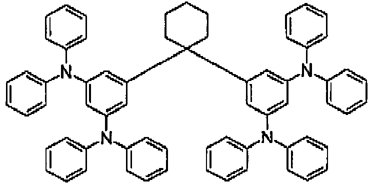 |
| (243) | (244) |
| 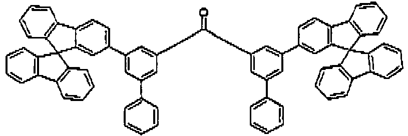 | 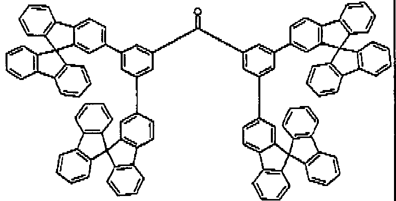 |
| 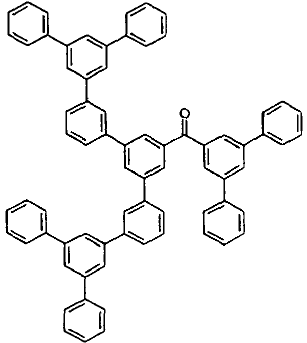 | 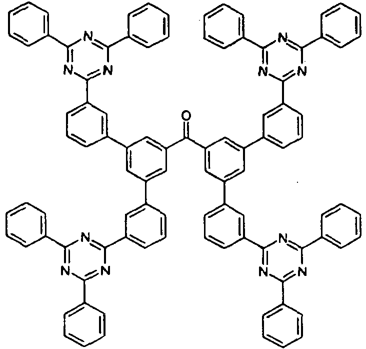 |
| (247) | (248) |
| 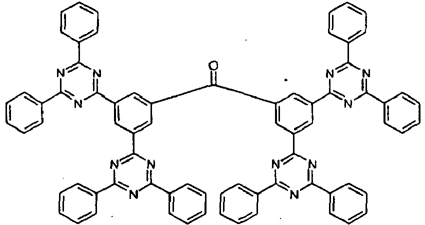 | 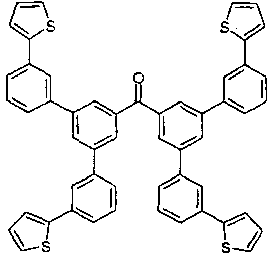 |
| (249) | (250) |

Die erfindungsgemäßen Verbindungen gemäß Formel (1) bzw. gemäß Formel (19) bis (22) können nach den im Folgenden beschriebenen, dem Fachmann allgemein bekannten Syntheseschritten dargestellt werden. Als Ausgangsverbindung zu symmetrisch substituierten erfindungsgemäßen Verbindungen können z. B: 3,3',5,5'-Tetrabrombenzophenon (Eur. J. Org. Chem. 2006, 2523-2529) oder Bis(3,5-dibromphenyl)methan (J. Org. Chem. 1994, 59, 7701-7703) dienen. Diese Tetrabromide können nach dem Fachmann bekannten Methoden umgewandelt werden. Die palladiumkatalysierte Umsetzung mit Boronsäuren bzw. Boronsäurederivaten (Suzuki-Kupplung) oder die palladiumkatalysierte Umsetzung mit Organozinkverbindungen (Negishi-Kupplung) führt zu erfindungsgemäßen aromatischen oder heteroaromatischen Verbindungen (Schema 1). Ein weiteres Verfahren zur Herstellung von 3,3',5,5'-Tetrabrombenzophenon besteht in der Monolithiierung von 1,3,5-Tribrombenzol, gefolgt von Umsetzung mit N,N-Dimethylcarbamoylchlorid, wie hinten für die Vorstufe 1 im Detail beschrieben ist.

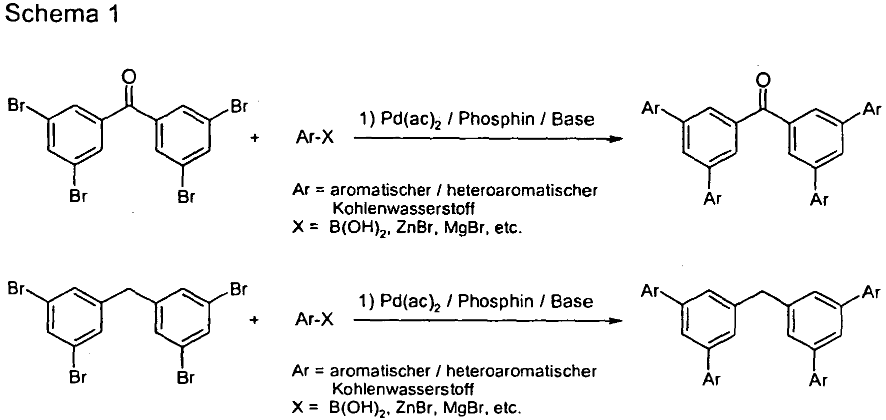

Die palladiumkatalysierte Umsetzung mit Aminen (Hartwig-Buchwald-Kupplung) führt zu erfindungsgemäßen aromatischen oder heteroaromatischen Aminen (Schema 2).

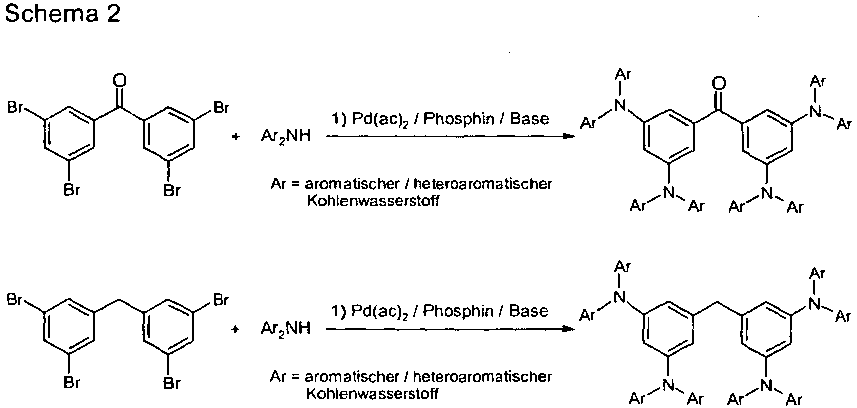

Die Bromfunktion kann durch Transmetallierung mit Organolithiumverbindungen bzw. Grignard-Verbindungen in eine elektrophile Gruppe überführt werden, die dann mit einer Vielzahl von Elektrophilen, wie z. B. Aryl-Bor-Halogeniden, Aldehyden, Ketonen, Nitrilen, Estern, Halogenestern, Kohlendioxid, Arylphosphinhalogeniden, Halogensulfinsäuren, Halogenarylsulfonsäuren, etc., gekoppelt werden, wobei die so erhaltenen Verbindungen erfindungsgemäße Endprodukte oder aber Intermediate sein können, die weiter umgesetzt werden können. Dies ist exemplarisch am Beispiel der Herstellung eines erfindungsgemäßen Ketons und eines Phosphinoxids verdeutlicht (Schema 3).

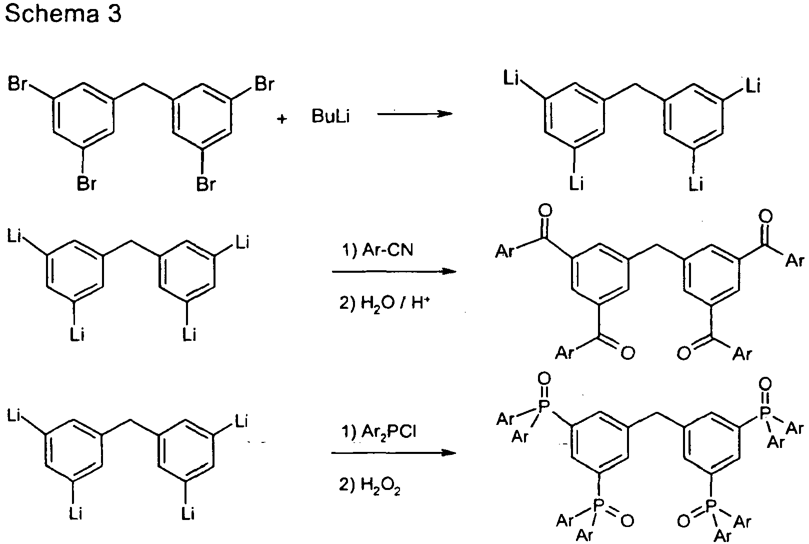

In Verbindungen, in denen die Gruppe Y für C=O steht, muss zunächst vor der Umsetzung mit einer Organolithiumverbindung oder einer Grignard-Verbindung eine Schutzgruppe eingeführt werden, die am Ende der Reaktion wieder abgespalten werden kann. Geeignete Schutzgruppen für Carbonylfunktionen sind dem Fachmann der organischen Synthese bekannt. Eine geeignete Schutzgruppe ist beispielsweise 1,3-Dioxolan (Schema 4), welches durch Umsetzung mit 2-Chlorethanol und Kalium*tert*-butanolat in Dimethylformamid eingeführt werden kann.

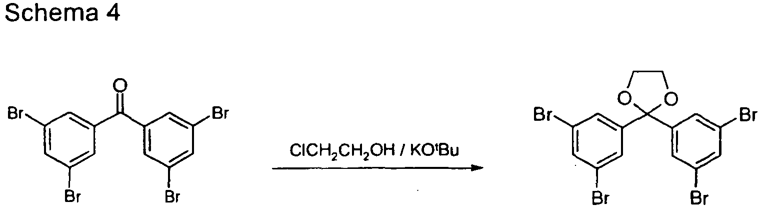

Die Bromfunktion kann durch Einwirkung eines Cyanids, beispielsweise Zinkcyanid in Anwesenheit von Zink und Tetrakis(triphenylphosphin)-palladium in Dimethylacetamid, in eine Nitrilfunktion überführt werden (Schema 5).

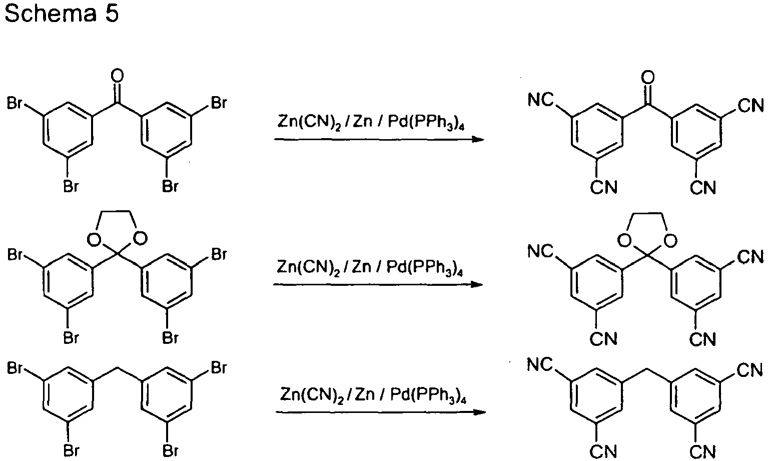

Durch Umsetzung der Nitrilfunktion mit Grignard-Verbindungen und anschließende saure Hydrolyse können daraus erfindungsgemäße Ketone hergestellt werden (Schema 6).

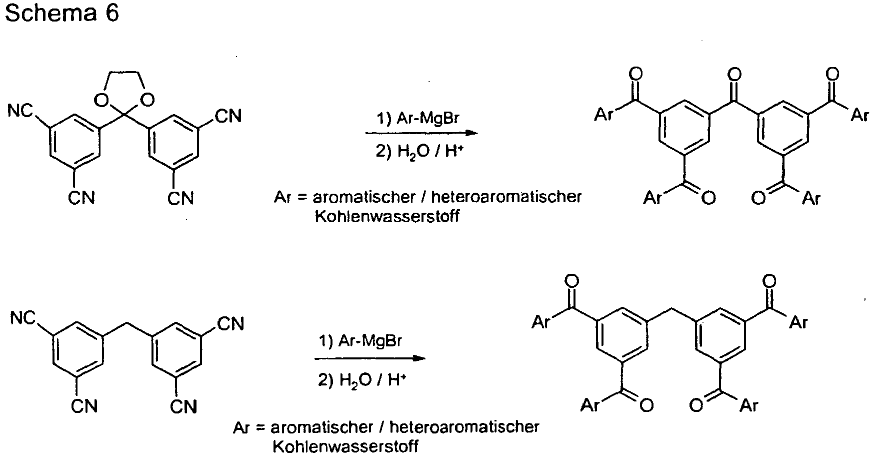

Eine weitere Möglichkeit ist die basische Hydrolyse der Nitrilfunktion zur Carbonsäure, welche durch weitere Umsetzung mit Thionylchlorid in das entsprechende Säurechlorid und durch weitere Umsetzung mit geeigneten Diaminen in erfindungsgemäße Benzimidazolderivate überführt werden kann (Schema 7).

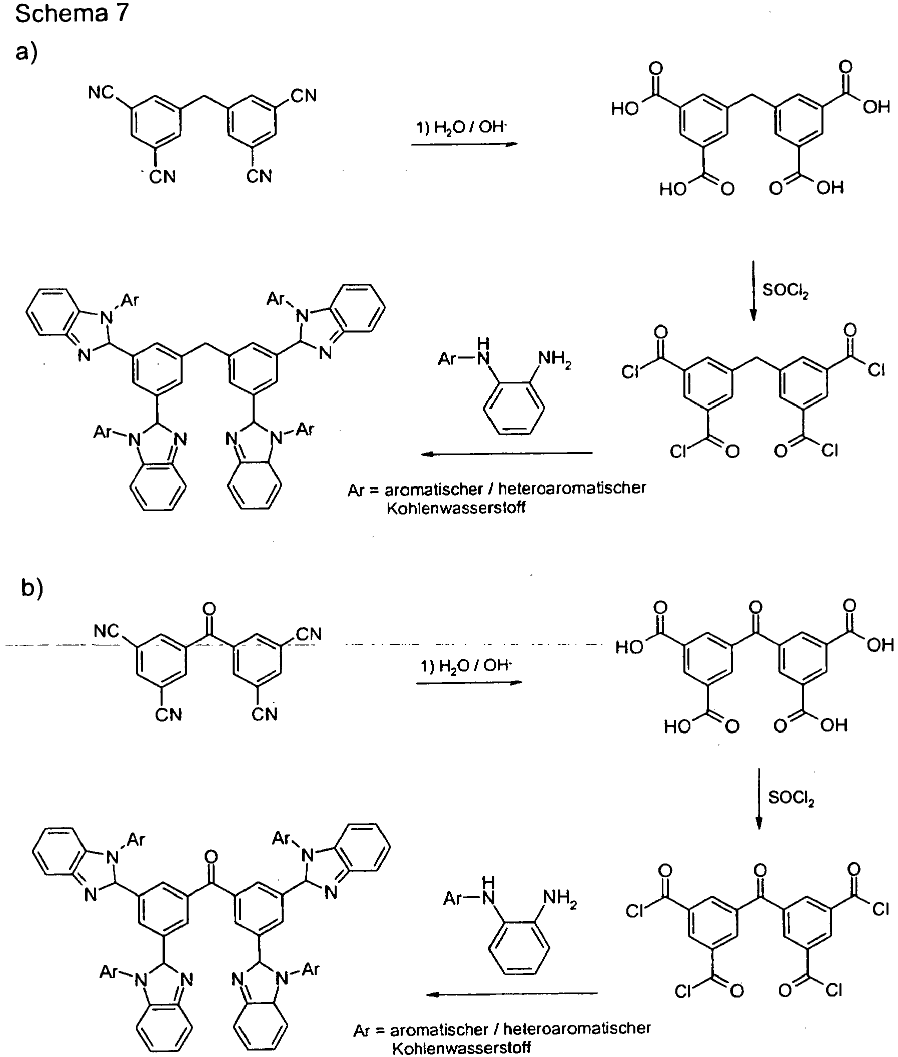

Die Darstellung von Triazin-Aryl- bzw. Bis-Triazinketonen kann z. B. ausgehend von Cyanurchlorid erfolgen, welches in Anlehnung an EP 810453 in das 1-Chlor-3,5-diaryltriazin überführt werden kann, das konsekutiv gemäß US 2959589 durch Einwirkung von Magnesium in THF in das 3,5-Diaryltriazin-1-magnesiumchlorid überführt werden kann, welches mit geeigneten Elektrophilen wie Nitrilen bzw. Carbamidsäurechloriden umgesetzt werden kann, wobei aus den so erhaltenen Additionsverbindungen durch saure Hydrolyse die gewünschten Ketone hervorgehen (Schema 8).

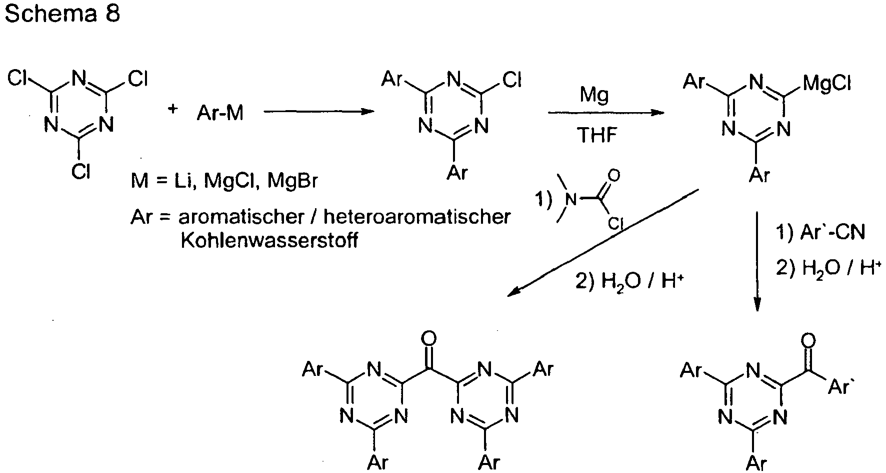

Ein weiterer Gegenstand der Erfindung ist ein Verfahren zur Herstellung der Verbindungen gemäß Formel (1) bzw. gemäß Formeln (19) bis (22) umfassend die Kupplung von einem substituierten oder unsubstituierten Bis(3,5-dibrombenzophenon) mit einer aromatischen oder heteroaromatischen Boronsäure oder einem entsprechenden Boronsäurederivat unter Metallkatalyse oder mit einem primären oder sekundären aromatischen Amin unter Metallkatalyse oder mit einem Metallcyanid unter Metallkatalyse.

Die oben beschriebenen erfindungsgemäßen Verbindungen, insbesondere Verbindungen, welche mit reaktiven Abgangsgruppen, wie Brom, Iod, Triflat, Tosylat, Boronsäure oder Boronsäureester, substituiert sind, können als Monomere zur Erzeugung entsprechender Dimere, Trimere, Tetramere, Pentamere, Oligomere, Polymere oder als Kern von Dendrimeren Verwendung finden. Die Oligomerisation bzw. Polymerisation erfolgt dabei bevorzugt über die Halogenfunktionalität bzw. die Boronsäurefunktionalität.

Ein weiterer Gegenstand der vorliegenden Erfindung sind Mischungen enthaltend mindestens eine Verbindung gemäß Formel (1) bzw. Formel (19) bis (22) und mindestens eine weitere Verbindung. Die weitere Verbindung kann beispielsweise ein fluoreszierender oder phosphoreszierender Dotand sein, wenn die Verbindung der Formel (1) bzw. Formel (19) bis (22) als Matrixmaterial verwendet wird. Geeignete fluoreszierende und phosphoreszierende Dotanden sind unten im Zusammenhang mit den organischen Elektrolumineszenzvorrichtungen aufgeführt und sind auch für die erfindungsgemäßen Mischungen bevorzugt. Die weitere Verbindung kann auch ein Dotierstoff sein, wenn die Verbindung gemäß Formel (1) bzw. Formel (19) bis (22) eine Lochtransport- oder Elektronentransportverbindung ist. Geeignete Dotierstoffe sind unten im Zusammenhang mit den organischen Elektrolumineszenzvorrichtungen aufgeführt.

Nochmals ein weiterer Gegenstand der vorliegenden Erfindung sind Lösungen enthaltend mindestens eine Verbindung gemäß Formel (1) und mindestens ein organisches Lösemittel. Derartige Lösungen sind erforderlich für die Herstellung der organischen elektronischen Vorrichtung aus Lösung, beispielsweise durch Spin-Coating oder durch Druckverfahren.

Die erfindungsgemäßen Verbindungen gemäß Formel (1) bzw. Formel (19) bis (22) eignen sich für den Einsatz in elektronischen Vorrichtungen, insbesondere in organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

Abhängig von der Substitution werden die Verbindungen in unterschiedlichen Funktionen und Schichten eingesetzt.

Ein weiterer Gegenstand der Erfindung ist daher die Verwendung von Verbindungen gemäß Formel (1) bzw. Formel (19) bis (22) in elektronischen Vorrichtungen, insbesondere in organischen Elektrolumineszenzvorrichtungen. Dabei gelten die oben genannten bevorzugten Ausführungsformen auch für die Verwendung der Verbindungen in organischen elektronischen Vorrichtungen.

Nochmals ein weiterer Gegenstand der Erfindung sind elektronische Vorrichtungen, insbesondere die oben genannten elektronischen Vorrichtungen, enthaltend mindestens eine Verbindung gemäß Formel (1) bzw. Formel (19) bis (22), insbesondere organische Elektrolumineszenzvorrichtungen, enthaltend Anode, Kathode und mindestens eine emittierende Schicht, dadurch gekennzeichnet, dass mindestens eine organische Schicht, die eine emittierende Schicht oder eine andere Schicht sein kann, mindestens eine Verbindung gemäß Formel (1) bzw. Formel (19) bis (22) enthält. Dabei gelten die oben genannten bevorzugten Ausführungsformen auch für die organischen elektronischen Vorrichtungen.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Exzitonenblockierschichten, Ladungserzeugungsschichten (Charge-Generation Layers) und/oder organischen oder anorganischen p/n-Übergängen. Weiterhin können die Schichten, insbesondere die Ladungstransportschichten, auch dotiert sein. Die Dotierung der Schichten kann für einen verbesserten Ladungstransport vorteilhaft sein. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

In einer weiteren bevorzugten Ausführungsform der Erfindung enthält die organische Elektrolumineszenzvorrichtung mehrere emittierende Schichten, wobei mindestens eine organische Schicht mindestens eine Verbindung gemäß Formel (1) bzw. Formel (19) bis (22) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (1) bzw. Formel (19) bis (22) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

In einer bevorzugten Ausführungsform der Erfindung werden die Verbindungen gemäß Formel (1) bzw. Formel (19) bis (22) als Matrixmaterial für fluoreszierende oder phosphoreszierende Verbindungen in einer emittierenden Schicht eingesetzt. Bei einem Matrixmaterial für phosphoreszierende Verbindungen steht Y bevorzugt für C=O und/oder eine oder mehrere Gruppen R stehen für C(=O)Ar, P(=O)Ar₂ oder N-Carbazolyl. Insbesondere steht die Gruppe Y für C=O. Bei einem Matrixmaterial für fluoreszierende Verbindungen stehen bevorzugt eine oder mehrere Gruppen R für ein aromatisches oder heteroaromatisches Ringsystem, insbesondere für ein aromatisches Ringsystem enthaltend Anthracen. Die Gruppe Y steht dann bevorzugt für C(R¹)₂.

Unter einem Matrixmaterial wird in einem System aus Matrix und Dotand diejenige Komponente verstanden, die in dem System im höheren Anteil vorliegt. Bei einem System aus einer Matrix und mehreren Dotanden wird als Matrix diejenige Komponente verstanden, deren Anteil der höchste in der Mischung ist. Es kann auch eine Mischung aus mehreren Matrixmaterialien verwendet werden.

Wenn die Verbindung gemäß Formel (1) bzw. Formel (19) bis (22) als Matrixmaterial für eine emittierende Verbindung in einer emittierenden Schicht eingesetzt wird, kann sie in Kombination mit einem oder mehreren phosphoreszierenden Materialien (Triplettemitter) eingesetzt werden. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne der vorliegenden Erfindung sollen insbesondere alle lumineszierenden Iridium- und Platinkomplexe als phosphoreszierende Verbindungen verstanden werden. Die Mischung aus der Verbindung gemäß Formel (1) und der emittierenden Verbindung enthält dann zwischen 99 und 50 Vol.-%, vorzugsweise zwischen 98 und 50 Vol.-%, besonders bevorzugt zwischen 97 und 60 Vol.-%, insbesondere zwischen 95 und 85 Vol.-% der Verbindung gemäß Formel (1) bezogen auf die Gesamtmischung aus Emitter und Matrixmaterial. Entsprechend enthält die Mischung zwischen 1 und 50 Vol.-%, vorzugsweise zwischen 2 und 50 Vol.-%, besonders bevorzugt zwischen 3 und 40 Vol.-%, insbesondere zwischen 5 und 15 Vol.-% des Emitters bezogen auf die Gesamtmischung aus Emitter und Matrixmaterial.

Bevorzugt ist auch die Verwendung mehrerer Matrixmaterialien als Mischung. Dabei ist insbesondere eine Komponente dieser Mischung eine lochtransportierende Verbindung und eine weitere Komponente der Mischung eine elektronentransportierende Verbindung. Wenn die Verbindung gemäß Formel (1) bzw. Formel (19) bis (22) eine elektronentransportierende Verbindung ist, wenn also beispielsweise die Gruppe Y für C=O und/oder die Gruppen R für C(=O)Ar oder für aromatische oder heteroaromatische Ringsysteme stehen, wird diese Verbindung bevorzugt in Kombination mit einem lochtransportierenden Matrixmaterial verwendet. Bevorzugte lochleitende Matrixmaterialien sind Triarylamine und Carbazolderivate. Wenn die Verbindung gemäß Formel (1) bzw. Formel (19) bis (22) eine lochleitende Verbindung ist, wenn also beispielsweise die Gruppe Y für C(R¹)₂ und die Gruppen R für N(Ar)₂ stehen, wird diese Verbindung bevorzugt in Kombination mit einem elektronentransportierenden Matrixmaterial verwendet. Bevorzugte elektronentransportierende Matrixmaterialien sind aromatische Ketone, aromatische Phosphinoxide, aromatische Sulfoxide, aromatische Sulfone und Triazinderivate.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 01/41512, WO 02/02714, WO 02/15645, EP 1191613, EP 1191612, EP 1191614 und WO 05/033244 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Wenn die Verbindung gemäß Formel (1) bzw. gemäß Formel (19) bis (22) als Matrixmaterial für fluoreszierende Verbindungen eingesetzt wird, beträgt der Anteil des Matrixmaterials in der emittierenden Schicht zwischen 50.0 und 99.9 Vol.-%, bevorzugt zwischen 80.0 und 99.5 Vol.-%, besonders bevorzugt zwischen 90.0 und 99.0 Vol.-%. Entsprechend beträgt der Anteil des Dotanden zwischen 0.1 und 50.0 Vol.-%, bevorzugt zwischen 0.1 und 20.0 Vol.-%, besonders bevorzugt zwischen 0.5 und 15 Vol.-%, ganz besonders bevorzugt zwischen 1.0 und 10.0 Vol.-%.

Bevorzugte Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 2-Position oder in 9-Position. Unter einem aromatischen Anthracendiamin wird eine Verbindung verstanden, in der zwei Diarylaminogruppen direkt an eine Anthracengruppe gebunden sind, vorzugsweise in 2,6-Position oder in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind gewählt aus Indenofluorenaminen bzw. -diaminen, beispielsweise gemäß WO 06/122630, Benzoindenofluorenaminen bzw. -diaminen, beispielsweise gemäß WO 08/006449, und Dibenzoindenofluorenaminen bzw. -diaminen, beispielsweise gemäß WO 07/140847. Beispiele für Dotanden aus der Klasse der Styrylamine sind substituierte oder unsubstituierte Tristilbenamine oder die Dotanden, die in WO 06/000388, WO 06/058737, WO 06/000389, WO 07/065549 und WO 07/115610 beschrieben sind.

In einer weiteren Ausführungsform der Erfindung werden die Verbindungen gemäß Formel (1) bzw. gemäß Formel (19) bis (22) als Lochtransportmaterial bzw. als Lochinjektionsmaterial bzw. als Elektronenblockiermaterial bzw. als Exzitonenblockiermaterial eingesetzt. Die Gruppe Y steht dann bevorzugt für C(R¹)₂. Die Verbindungen sind dann bevorzugt mit mindestens einer Gruppe N(Ar)₂ substituiert, bevorzugt mit mindestens zwei Gruppen N(Ar)₂ und/oder sie enthalten weitere Gruppen, die den Lochtransport verbessern. Besonders bevorzugt stehen hier alle Gruppen R für N(Ar)₂. Die Gruppen N(Ar)₂ sind bevorzugt ausgewählt aus den oben beschriebenen Formeln (17) oder (18). Weitere bevorzugte Gruppen, die den Lochtransport verbessern, sind beispielsweise die elektronenreiche Heteroaromaten, insbesondere Thiophen, Pyrrol oder Furan als Gruppe R. Die Verbindung wird bevorzugt in einer Lochtransport- bzw. in einer Lochinjektions- bzw. in einer Elektronenblockier- bzw. in einer Excitonenblockierschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Eine Elektronenblockier- bzw. Exzitonenblockierschicht im Sinne dieser Erfindung ist eine Schicht, die auf Anodenseite direkt an eine emittierende Schicht angrenzt. Wenn die Verbindungen gemäß Formel (1) als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben.

In nochmals einer weiteren Ausführungsform der Erfindung werden die Verbindungen gemäß Formel (1) bzw. Formel (19) bis (22) als Elektronentransportmaterial bzw. als Lochblockiermaterial in einer Elektronentransportschicht bzw. einer Lochblockierschicht eingesetzt. Hier ist es bevorzugt, wenn die Gruppe Y für C=O steht und/oder wenn mindestens eine der aromatischen Gruppen ein Triazin darstellt, also Verbindungen der Formel (20) und (21), und/oder wenn mindestens einer der Substituenten R für eine Heteroarylgruppe, welche einen elektronenarmen Heterocyclus darstellt, wie beispielsweise Imidazol, Pyrazol, Thiazol, Benzimidazol, Benzothiazol, Triazol, Oxadiazol, Benzothiadiazol, Pyridin, Pyrazin, Pyrimidin, Pyridazin, Triazin, Bipyridin, Chinolin, Isochinolin, Chinoxalin, Phenanthrolin, etc., oder für C(=O)Ar, P(=O)Ar₂, S(=O)Ar oder S(O)₂Ar steht. Weiterhin kann es bevorzugt sein, wenn die Verbindung in der Elektronentransportschicht dotiert ist, beispielsweise mit Elektronendonorverbindungen oder Lithiumsalzen, wie z. B. Liq. Eine Lochblockierschicht im Sinne dieser Erfindung ist eine Schicht, die zwischen einer emittierenden Schicht und einer Elektronentransportschicht liegt und direkt an die emittierende Schicht angrenzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es sei jedoch angemerkt, dass der Druck auch noch geringer sein kann, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen. Dabei können nicht nur Lösungen aus einzelnen Materialien aufgebracht werden, sondern auch Lösungen, die mehrere Verbindungen enthalten, beispielsweise Matrixmaterialien und Dotanden.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. Da die Verbindungen gemäß Formel (1) bzw. Formel (19) bis (22) im Allgemeinen eine hohe Löslichkeit in organischen Lösemitteln aufweisen, können diese Verbindungen gut aus Lösung verarbeitet werden. So ist es beispielsweise möglich, eine emittierende Schicht enthaltend eine Verbindung gemäß Formel (1) bzw. Formel (19) bis (22) und einen fluoreszierenden oder phosphoreszierenden Dotanden aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend Verbindungen gemäß Formel (1) bzw. gemäß Formel (19) bis (22) bzw. die oben aufgeführten bevorzugten Ausführungsformen angewandt werden.

Die erfindungsgemäßen Verbindungen weisen bei Verwendung in organischen Elektrolumineszenzvorrichtungen folgende überraschende Vorteile gegenüber dem Stand der Technik auf:
1. Die erfindungsgemäßen Verbindungen weisen eine hohe thermische Stabilität auf und lassen sich unzersetzt sublimieren.
2. Die erfindungsgemäßen Verbindungen, in denen Y für C=O steht, insbesondere solche, in denen die Reste R jeweils für ein aromatisches oder heteroaromatisches Ringsystem stehen, führen bei Verwendung als Matrixmaterial für phosphoreszierende Emitter zu einer erheblichen Verbesserung der Lebensdauer und einer leichten Verbesserung der Effizienz der organischen Elektrolumineszenzvorrichtung.
3. Die erfindungsgemäßen Verbindungen, insbesondere solche, die Diarylamino-Substituenten als Gruppen R enthalten, führen bei Verwendung in einer Elektronen-/Exzitonenblockierschicht in einer phosphoreszierenden Elektrolumineszenzvorrichtung zu einer erheblichen Verbesserung der Effizienz gegenüber Materialien gemäß dem Stand der Technik. Dies gilt insbesondere für Verbindungen, in denen Y für C(R¹)₂ steht.
4. Die erfindungsgemäßen Verbindungen, insbesondere solche, welche mit Diarylaminogruppen als Gruppen R substituiert sind und/oder welche mit elektronenreichen Heteroaromaten substituiert sind, eignen sich sehr gut für die Verwendung als Lochinjektions- und Lochtransportmaterial und führen zu einer Verringerung der Betriebsspannung.

Die Erfindung wird durch die nachfolgenden Beispiele genauer beschrieben, ohne sie dadurch einschränken zu wollen. Der Fachmann kann,ohne erfinderisch tätig zu werden, weitere erfindungsgemäße Verbindungen herstellen und diese in organischen elektronischen Vorrichtungen verwenden.

### Beispiele:

Die nachfolgenden Synthesen werden - sofern nicht anders angegeben - unter einer Schutzgasatmosphäre in getrockneten Lösungsmitteln durchgeführt. Lösungsmittel und Reagenzien können von den Firmen ALDRICH bzw. ABCR bezogen werden.

Die Vorstufen können wie folgt hergestellt werden (Vorstufen 1-13):

### Vorstufe 1: Synthese von Bis(3,5-dibromphenyl)keton

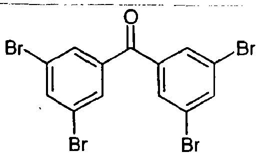

Eine Suspension von 252.0 g (801 mmol) 1,3,5-Tribrombenzol in 4000 ml Diethylether wird auf -72 °C gekühlt. 320 ml (800 mmol) einer Lösung von *n*-Butyllithium (2.5 M in Hexan) werden so zugetropft, dass die Temperatur -60 °C nicht übersteigt, dann wird eine Stunde nachgerührt. Anschließend wird eine Mischung aus 38.8 ml (399 mmol) *N,N*-Dimethylcarbamoylchlorid und 80 ml Diethylether zugegeben und eine Stunde nachgerührt, dann wird die Kühlung entfernt. Ab einer Innentemperatur von 0 °C wird ein Gemisch aus 1000 ml Wasser und 80 ml Essigsäure innerhalb einer Stunde zugetropft. Es wird zwei Stunden nachgerührt und über Nacht stehen gelassen, wobei sich ein Feststoff absetzt. 3000 ml Diethylether werden abgesaugt, dann wird der Feststoff abgesaugt, einmal mit 100 ml Ethanol, zweimal mit 100 ml Ethanol/Wasser (1:1), zweimal mit 100 ml Ethanol gewaschen und im Vakuum getrocknet. Ausbeute: 168.5 g (338 mmol), 84.9%, Reinheit ca. 98.5% (HPLC).

### Vorstufe 2: Synthese von Bis(3,5-dibromophenyl)methanol

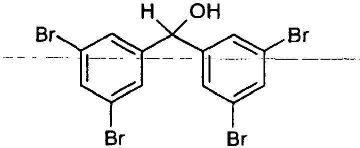

Bis(3,5-dibromophenyl)methanol wird dargestellt, wie in J. Org. Chem. 1994, 59, 7701-7703 beschrieben.

### Vorstufe 3: Synthese von Bis(3,5-dibromophenyl)methan

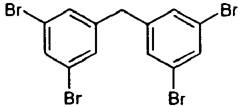

Bis(3,5-dibromophenyl)methan wird dargestellt wie in J. Org. Chem. 1994, 59, 7701-7703 beschrieben.

### Vorstufe 4: Synthese von Bis(3,5-dicyanophenyl)keton

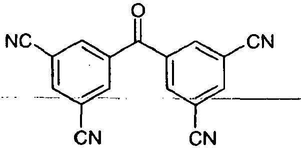

49.7 g (100 mmol) Bis(3,5-dibromphenyl)keton, 29.4 g (250 mmol) Zinkcyanid, 2.0 g (31 mmol) Zinkstaub und 8.1 g (7 mmol) Tetrakis(triphenylphosphin)palladium werden in 900 ml Dimethylacetamid für sieben Tage unter kräftigem Rühren auf 135 °C Innentemperatur erhitzt. Nach Abkühlen wird die Mischung mit 500 ml Wasser und 500 ml Ammoniaklösung (25%) versetzt und zwei Stunden gerührt. Der sich bildende Feststoff wird abgesaugt, fünfmal mit 200 ml Wasser, fünfmal mit 100 ml Methanol gewaschen und anschließend in Dichlormethan aufgenommen. Die Lösung wird über Natriumsulfat getrocknet, über Kieselgel filtriert und im Vakuum stark eingeengt. Durch Zugabe von 200 ml Ethanol wird ein Feststoff ausgefällt, der abgesaugt und mit Ethanol gewaschen wird. Nach viermaligem Umkristallisieren aus Dimethylformamid bleibt ein farbloser Feststoff. Ausbeute 19.4 g (69 mmol), 68.7%, Reinheit ca. 99% (HPLC).

### Vorstufe 5: Synthese von Bis(3,5-dicyanophenyl)methan

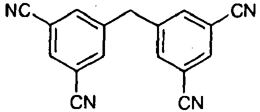

Bis(3,5-dicyanophenyl)methan wird analog zu Vorstufe 4 aus 38.7 g (80 mmol) Bis(3,5-dibromophenyl)methan dargestellt. Ausbeute: 15.2 g (57 mmol), 71.0%, Reinheit ca. 99.5% (HPLC).

### Vorstufe 6: Synthese von 3,3',5,5'-Benzophenontetracarbonsäure

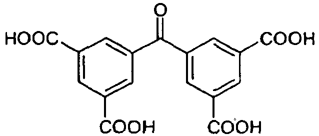

28.2 g (100 mmol) Bis(3,5-dicyanophenyl)keton werden in 500 ml-Ethanol suspendiert, langsam mit 40.0 g (1 mol) Natriumhydroxid in 500 ml Wasser versetzt und für 24 Stunden unter Rückfluß erhitzt. Nach Abkühlen wird die Mischung auf 1000 ml wässrige Salzsäure (5 M) gegossen. Der sich bildende Niederschlag wird abgesaugt, bis zum Neutralpunkt mit Wasser und anschließend zweimal mit 150 ml Ethanol gewaschen. Ausbeute: 33.5 g (93.5 mmol), 93.5%, Reinheit ca. 99.5% (HPLC).

### Vorstufe 7: Synthese von 3,3',5,5'-Diphenylmethantetracarbonsäure

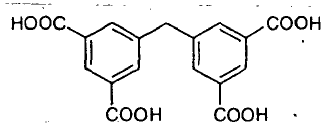

3,3',5,5'-Diphenylmethantetracarbonsäure wird analog zu Vorstufe 6 aus 16.1 g (60 mmol) Bis(3,5-dicyanophenyl)methan dargestellt. Ausbeute: 20.0 g (58 mmol), 96.8%, Reinheit ca. 99.5% (HPLC).

### Vorstufe 8: Synthese von 3,3',5,5'-Benzophenontetracarbonsäurechlorid

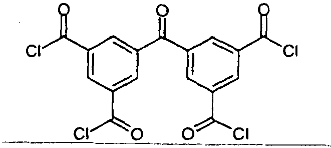

28.7 g (80 mmol) 3,3',5,5'-Benzophenontetracarbonsäure werden mit 150 ml Thionylchlorid vier Stunden unter Rückfluss erhitzt. Nach Abkühlen wird überschüssiges Thionylchlorid im Vakuum entfernt. Ausbeute: 34.2 g (79 mmol) 98.8%, Reinheit ca. 99.5% (HPLC).

### Vorstufe 9: Synthese von 3,3',5,5'-Diphenylmethantetracarbonsäurechlorid

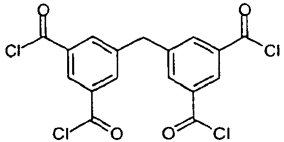

3,3',5,5'-Diphenylmethantetracarbonsäurechlorid wird analog zu Vorstufe 8 aus 17.2 g (50 mmol) 3,3',5,5'-Diphenylmethantetracarbonsäure dargestellt. Ausbeute: 20.6 g (49 mmol), 98.6%, Reinheit ca. 99.5% (HPLC).

### Vorstufe 10: Synthese von 2,2-Bis(3,5-dibromophenyl)-1,3-dioxolan

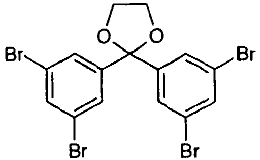

99.6 g (200 mmol) Bis(3,5-dibromophenyl)keton werden vorgelegt und langsam mit 20.1 ml (300 mmol) 2-Chlorethanol versetzt. Dann werden 300 ml Dimethylformamid zugegeben, und die Mischung wird auf -63 °C gekühlt. Ein Gemisch aus 33.7 g (112 mmol) Kalium-*tert*-butanolat und 250 ml Dimethylformamid wird innerhalb von 40 Minuten zugetropft, wobei eine hellbraune Suspension entsteht. Nach sechs Stunden wird die Kühlung entfernt. Bei etwa -45 °C Innentemperatur wird ein Gemisch aus 200 ml gesättigter Ammoniumchloridlösung und 20 ml Ammoniaklösung (25%) zugegeben. Nach Aufwärmen auf Raumtemperatur wird der Feststoff abgesaugt, einmal mit Wasser und dreimal mit Ethanol/Wasser (1:1) gewaschen. Der Rückstand wird dreimal am Rotationsverdampfer mit Toluol azeotrop getrocknet, in 300 ml n-Heptan aufgenommen, abfiltriert, mit n-Heptan gewaschen und im Vakuum getrocknet. Ausbeute: 100.0 g (179 mmol), 89.5%, Reinheit ca. 97% (HPLC).

### Vorstufe 11: Synthese von m-Terphenyl-3-boronsäure

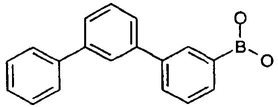

*m*-Terphenyl-3-boronsäure wird dargestellt wie in WO 07/043357 beschrieben.

### Vorstufe 12: Synthese von m-Quaterphenyl-3-boronsäure

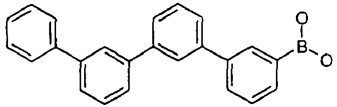

*m*-Quaterphenyl-3-boronsäure wird in Anlehnung an *m*-Terphenyl-3-boronsäure wie in WO 07/043357 beschrieben dargestellt

### Vorstufe 13: 2-Chlor-4,6-diphenyl-1,3,5-triazin

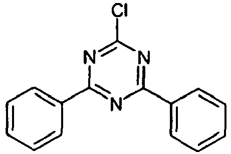

Darstellung nach EP 810453. Analoge 2-Chlor-4,6-diaryl-1,3,5-triazine (Aryl = 4-Biphenyl, 3-Biphenyl, 3-*m*-Terphenyl, 3,5-Diphenylphen-1-yl) werden ebenfalls nach diesem Verfahren gewonnen.

### Beispiel 1: Synthese von Bis(3,5-diphenylphenyl)keton

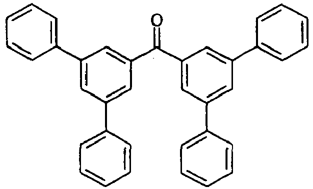

74.7 g (150 mmol) Bis(3,5-dibromphenyl)keton, 109.7 g (900 mmol) Phenylboronsäure, 267.5 g (1162 mmol) Trikaliumphosphat-Monohydrat, 5.5 g (18 mmol) Tri-*o*-tolylphosphin und 673.5 mg (3 mmol) Palladium(II)-acetat werden in einem Gemisch von 600 ml Toluol, 300 ml Dioxan und 750 ml Wasser suspendiert und für 72 h zum Rückfluß erhitzt. Nach Abkühlen wird die organische Phase abgetrennt, dreimal mit Wasser gewaschen und über Natriumsulfat getrocknet. Anschließend wird über Aluminiumoxid filtriert, auf etwa 200 ml eingeengt und mit 500 ml Ethanol versetzt, wobei das Rohprodukt ausfällt. Der Feststoff wird abgesaugt und mit 100 ml Ethanol gewaschen, dann wird fünfmal in siedendem Toluol gelöst und durch Zugabe von Ethanol in der Hitze wieder ausgefällt. Ausbeute: 44.0 g (90 mmol), 60.2%, Reinheit ca. 99.9% (HPLC).

Analog zu Beispiel 1 werden aus Bis(3,5-dibromophenyl)keton und entsprechenden Boronsäuren folgende erfindungsgemäße Verbindungen erhalten (Beispiele 2-8):

| Bsp. | Boronsäure | Produkt | Ausbeute |
|---|---|---|---|
| 2 | 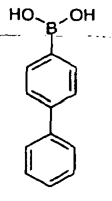 | 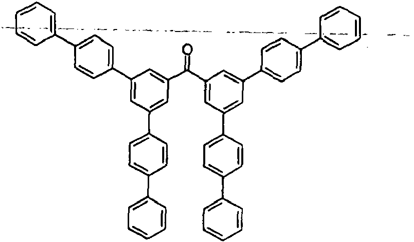 | 61,3% |
| 3 | 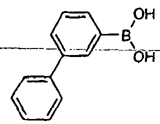 | 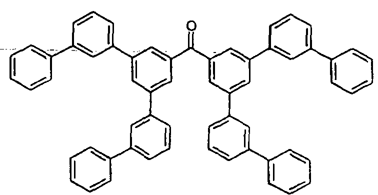 | 64.1% |
| 4 | 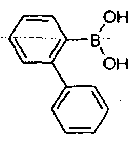 | 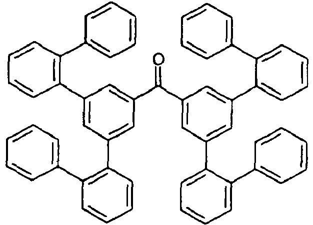 | 68.7% |
| 5 | 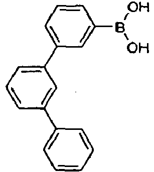 | 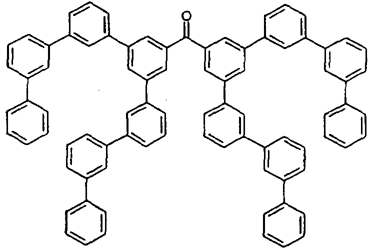 | 33.3% |
| 6 | 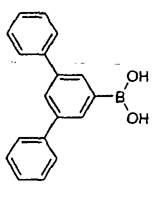 | 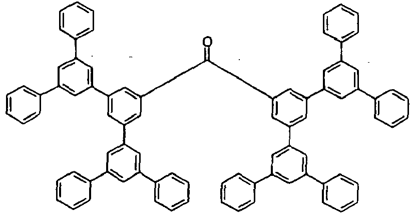 | 35.6% |
| 7 | 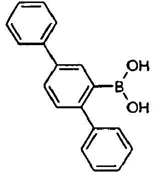 | 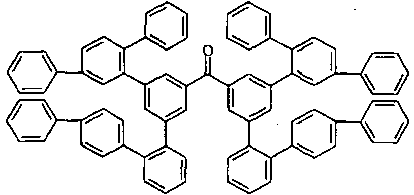 | 28.3% |
| 8 | 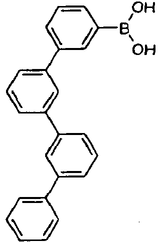 | 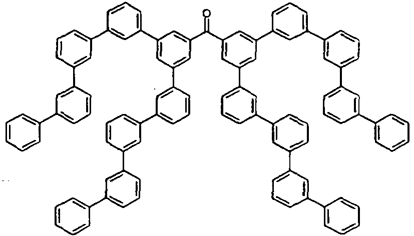 | 30.5% |
| 9 | 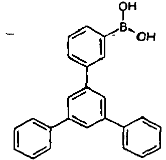 | 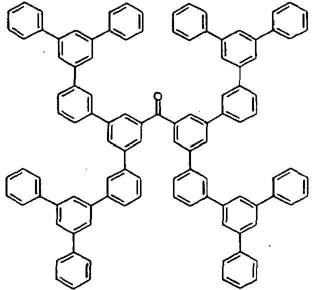 | 82.0% |
| 10 | 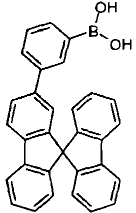 | 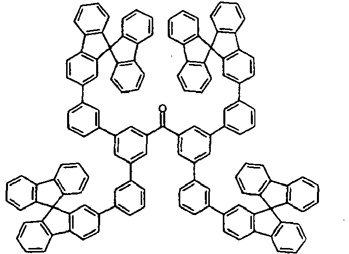 | 80.0% |
| 11 | 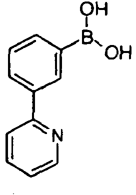 | 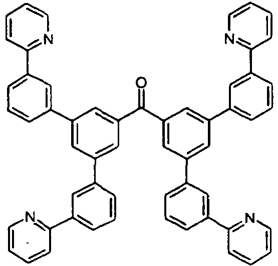 | 34.5% |

### Beispiel 12: Synthese von Bis(3,5-diphenylphenyl)methan

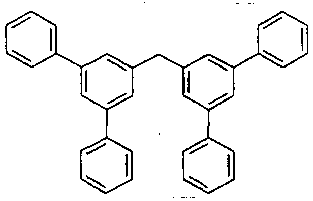

13.9 g (28 mmol) Bis(3,5-diphenylphenyl)keton, 6.8 g (121 mmol) Kaliumhydroxid und 4.9 ml (100 mmol) Hydrazinhydrat werden in 450 ml Diethylenglycol suspendiert und für fünf Stunden auf 175 °C Innentemperatur erhitzt, wobei eine klare Lösung entsteht; sich bildendes Wasser wird dabei über einen Wasserabscheider aus der Mischung entfernt. Beim Abkühlen auf Raumtemperatur fällt ein Feststoff aus. Es werden 400 ml Wasser zugegeben, der Feststoff wird abgesaugt, mit Methanol gewaschen, zweimal aus Toluol und dreimal aus Dimethylformamid umkristallisiert und anschließend im Vakuum getrocknet. Ausbeute: 10.7 g (23 mmol), 80.9%, Reinheit ca. 99.8% (HPLC).

Analog zu Beispiel 12 werden aus entsprechenden Ketonen folgende erfindungsgemäße Verbindungen erhalten (Beispiele 13-17):

| Bsp. | Keton | Produkt | Ausbeute |
|---|---|---|---|
| 13 | 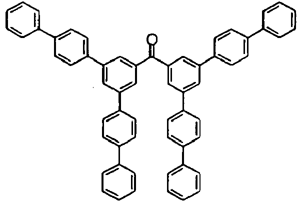 | 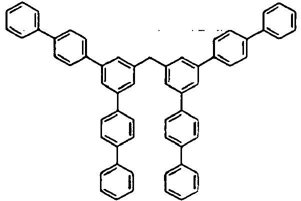 | 76.4% |
| 14 | 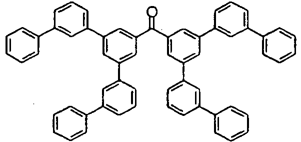 | 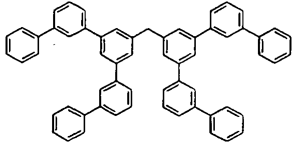 | 82.3% |
| 15 | 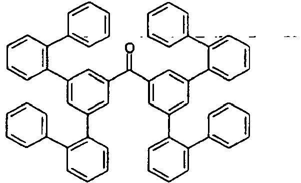 | 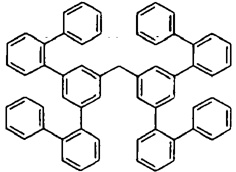 | 79.7% |
| 16 | 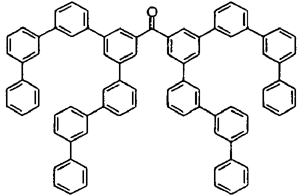 | 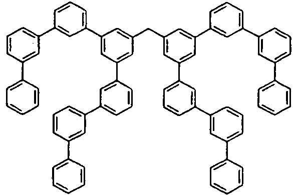 | 64.2% |
| 17 | 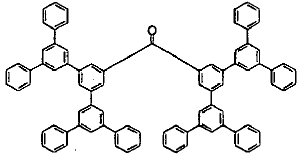 | 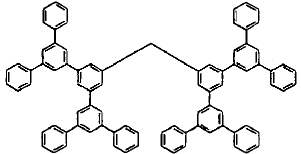 | 69.2% |

### Beispiel 18: Synthese von 3,3',5,5'-Tetrabenzoylbenzophenon

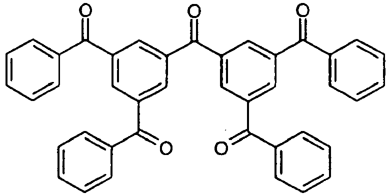

Eine Lösung von 16.3 g (30 mmol) 2,2-Bis(3,5-dibromophenyl)-1,3-dioxolan in 250 ml Tetrahydrofuran wird auf -62 °C gebracht. 52 ml (130 mmol) *n*-Butyllithium werden langsam zugetropft und die Mischung zwei Stunden nachgerührt. Dann werden 16.8 ml (165 mmol) Benzonitril in 20 ml Tetrahydrofuran zugetropft, die Mischung wird zwei Stunden nachgerührt und dann auf Raumtemperatur aufwärmen gelassen. Man versetzt mit 50 ml 1 N wässriger Salzsäure, kocht 16 h unter Rückfluss, entfernt das Lösemittel im Vakuum, nimmt den Rückstand in 300 ml Dichlormethan auf, wäscht mit gesättigter Natriumhydrogencarbonat-Lösung neutral, trocknet die organische Phase über Magnesiumsulfat. Nach Entfernen des Dichlormethans im Vakuum wird der Rückstand dreimal an Kieselgel chromatographiert (Laufmittel Heptan:Essigsäureethylester 3:1 > 1:1). Ausbeute: 7.7 g (13 mmol), 42.9 %, Reinheit ca. 99.8% (HPLC).

### Beispiel 19: Synthese von 3,3',5,5'-Tetrakis(1-phenyl-benzimidazol-2-yl)benzophenon

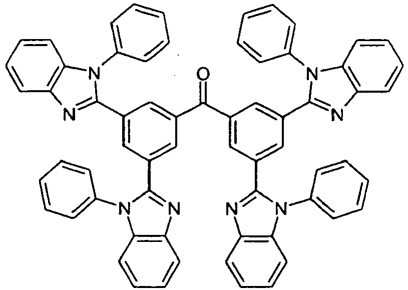

21.6 g (50 mmol) 3,3',5,5'-Benzophenontetracarbonsäurechlorid und 46.1 g (250 mmol) 2-Aminodiphenylamin werden in 300 ml Dichlormethan gelöst. 78 ml (560 mmol) Triethylamin werden langsam zugetropft und das Gemisch für 24 Stunden gerührt. Anschließend wird die organische Phase abgetrennt, zweimal mit 100 ml Wasser gewaschen, über Magnesiumsulfat getrocknet und im Vakuum vom Lösungsmittel befreit. Der Rückstand wird mit 350 ml Ethanol aufgenommen und eine Stunde gerührt. Der Feststoff wird abgesaugt und viermal aus Dimethylformamid umkristallisiert. Ausbeute: 19.9 g (21 mmol), 41.8%, Reinheit ca. 99.5% (HPLC).

### Beispiel 20: Synthese von 3,3',5,5'-Tetrakis(1-phenyl-benzimidazol-2-yl)diphenylmethan

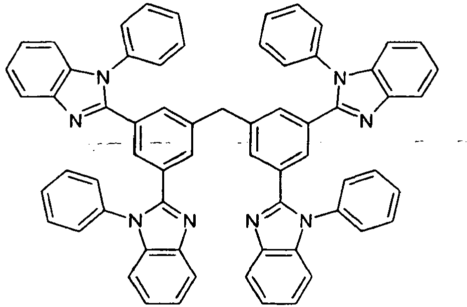

Analog zu Beispiel 16 wird 3,3',5,5'-Tetrakis(1-phenyl-benzimidazol-2-yl)-diphenylmethan aus 16.7 g (40 mmol) 3,3',5,5'-Diphenylmethantetracarbonsäurechlorid hergestellt. Ausbeute: 17.8 g (19 mmol), 47.5%, Reinheit ca. 99.7% (HPLC).

### Beispiel 21: Synthese von Bis(4,6-diphenyl-1,3,5-triazin-2-yl)keton

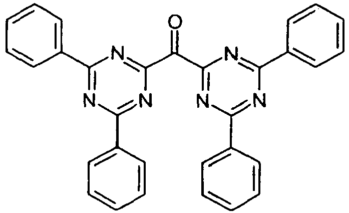

Aus 53.5 g (200 mmol) 2-Chlor-4,6-diphenyl-1,3,5-triazin und 4.9 g (200 mmol) Magnesium, aktiviert mit einem Körnchen lod, wird unter Kochen in 700 ml THF die entsprechende Grignard-Verbindung dargestellt. Die Grignard-Lösung wird auf -78° C abgekühlt und unter gutem Rühren tropfenweise mit einem Gemisch aus 9.7 g (90 mmol) *N,N*-Dimethyl-carbamidsäurechlorid in 100 ml THF versetzt. Nach vollendeter Zugabe wird 1 h bei -78 °C nachgerührt, dann wird die Kühlung entfernt. Ab einer Innentemperatur von 0 °C wird ein Gemisch aus 500 ml Wasser und 30 ml Essigsäure innerhalb einer Stunde zugetropft. Es wird zwei Stunden nachgerührt und über Nacht stehengelassen. Die Wasserphase wird abgetrennt, die organische Phase wird im Vakuum getrocknet, der Rückstand wird mit 300 ml Ethanol versetzt und eine Stunde mit Ethanol ausgerührt. Dann wird der Feststoff abgesaugt, einmal mit 100 ml Ethanol, zweimal mit 100 ml Ethanol / Wasser (1:1), zweimal mit 100 ml Ethanol gewaschen, im Vakuum getrocknet und abschließend fünfmal aus DMF / BuOH umkristallisiert Ausbeute: 22.0 g (45 mmol), 49.6 %, Reinheit ca. 99.9% (HPLC).

Analog zu Beispiel 21 werden aus und entsprechenden 2-Chlor-4,6-diaryl-1,3,5-triazinen folgende erfindungsgemäße Verbindungen erhalten (Beispiele 22-25):

| Bsp. | Triazin | Produkt | Ausbeute |
|---|---|---|---|
| 22 | 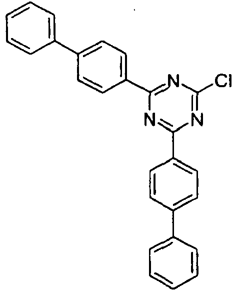 | 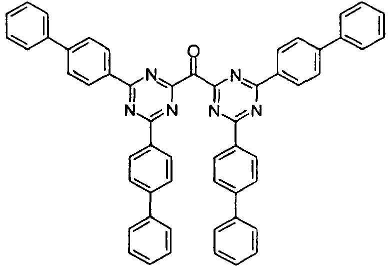 | 59.4% |
| 23 | 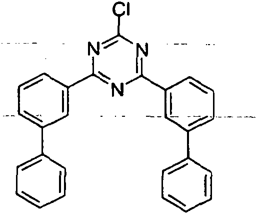 | 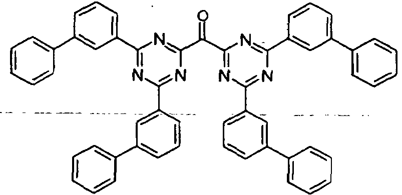 | 51.1% |
| 24 | 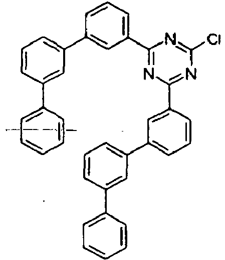 | 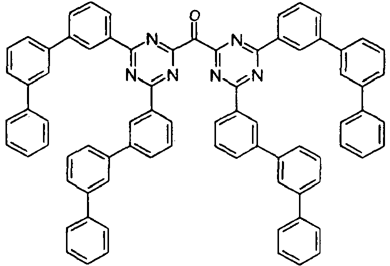 | 35.0% |
| 25 | 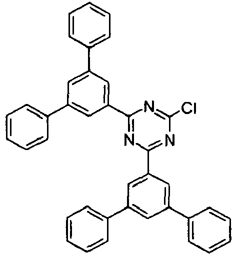 | 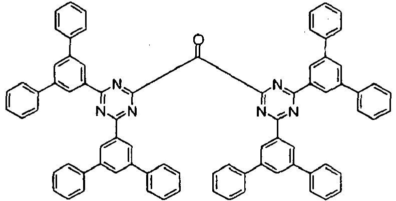 | 39.1% |

### Beispiel 26: Synthese von (4,6-Diphenyl-1,3,5-triazin-2-yl)-(3,5-diphenylphen-1-yl)keton

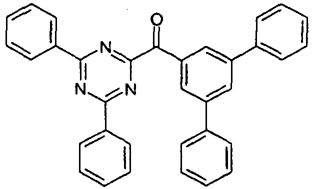

Aus 53.5 g (200 mmol) 2-Chlor-4,6-diphenyl-1,3,5-triazin und 4.9 g (200 mmol) Magnesium, aktiviert mit einem Körnchen lod, wird unter Kochen in 700 ml THF die entsprechende Grignard-Verbindung dargestellt. Die Grignard-Lösung wird auf 0° C abgekühlt und unter gutem Rühren tropfenweise mit einer Suspension von 48.5 g (190 mmol) 1-Cyano-3,5-diphenyl-benzol in 250 ml THF versetzt. Nach vollendeter Zugabe wird eine Stunde bei 0 °C nachgerührt, dann wird die Kühlung entfernt und sechs Stunden bei Raumtemperatur und abschließend 3 Stunden unter Rückfluss gerührt. Nach Zugabe eines Gemischs aus 200 ml Wasser und 100 ml Essigsäure wird sechs Stunden unter Rückfluss erhitzt. Nach Erkalten wird die Wasserphase abgetrennt, die organische Phase wird in Vakuum entfernt, der Rückstand wird mit 300 ml Ethanol versetzt und eine Stunde mit Ethanol ausgerührt. Dann wird der Feststoff abgesaugt, einmal mit 100 ml Ethanol, zweimal mit 400 ml Ethanol/Wasser (1:1), zweimal mit 100 ml Ethanol gewaschen, im Vakuum getrocknet und abschließend fünfmal aus Aceton / EtOH umkristallisiert. Ausbeute: 55.3 g (113 mmol), 59.5 %, Reinheit ca. 99.9% (HPLC).

Analog zu Beispiel 26 werden aus und entsprechenden 2-Chlor-4,6-diaryl-1,3,5-triazinen folgende erfindungsgemäße Verbindungen erhalten (Beispiele 27-30):

| Bsp. | Triazin | Produkt | Ausbeute |
|---|---|---|---|
| 27 | 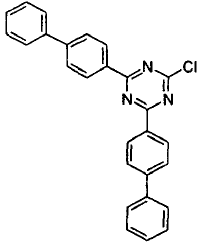 | 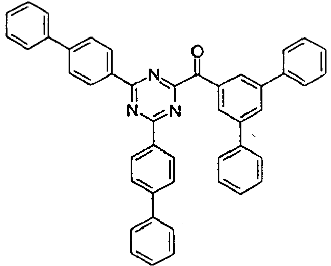 | 41.7% |
| 28 | 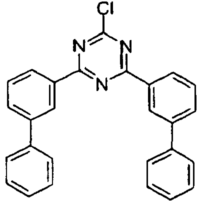 | 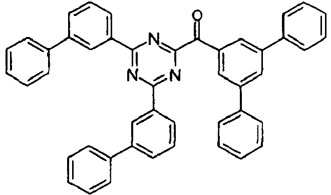 | 33.2% |
| 29 | 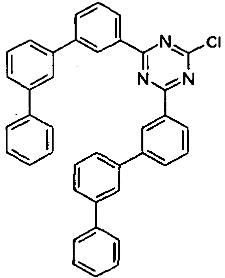 | 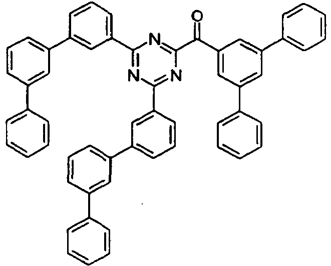 | 27.0% |
| 30 | 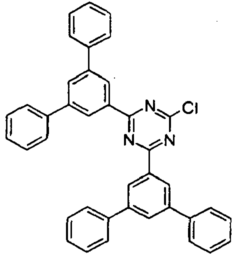 | 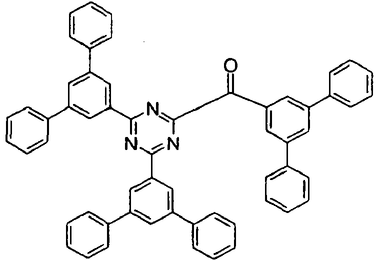 | 35.8% |

### Beispiel 31: Synthese von Bis-1,3-(diphenylamino-phenyl)keton

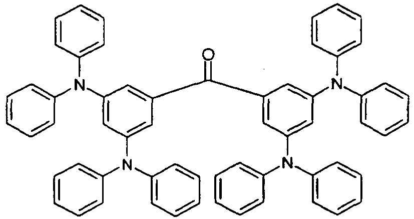

Eine Suspension vom 37.3 g (75 mmol) Bis(3,5-dibromphenyl)keton, 76.2 g (450 mmol) Diphenylamin und 49.0 g (510 mmol) Natrium-tert-butylat in 1000 ml Toluol wird mit 607 mg (3 mmol) Tri-tert-butylphosphio und dann mit 337 mg (1.5 mmol) Palladium(II)acetat versetzt und anschließend 3 h unter Rückfluss erhitzet. Nach Erkalten wird mit 500 ml Wasser versetzt, über eine kurze Säule aus Kieselgel filtriert, die organische Phase wird abgetrennt, dreimal mit 500 ml Wasser gewaschen, über Magnesiumsulfat getrocknet und anschließend im Vakuum fast bis zur Trockene eingeengt. Der Rückstand wird in 1000 ml warmem Ethanol aufgenommen. Nach Erkalten saugt man vom ausgefallenen Feststoff ab, trocknet diesen im Vakuum und kristallisiert fünfmal aus DMF um. Ausbeute: 23.8 g (28 mmol), 37.7 %, Reinheit ca. 99.9% (HPLC).

Analog zu Beispiel 31 werden aus und entsprechenden Amin folgende erfindungsgemäße Verbindungen erhalten (Beispiele 32-37):

| Bsp. | Amin | Produkt | Ausbeute |
|---|---|---|---|
| 32 | 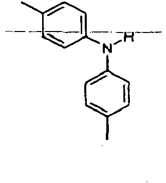 | 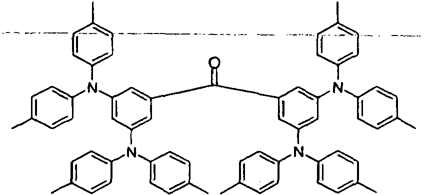 | 41.5 % |
| 33 | 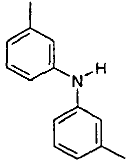 | 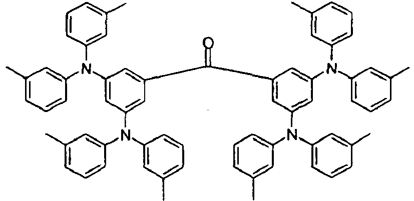 | 32.7 % |
| 34 | 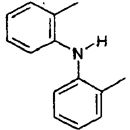 | 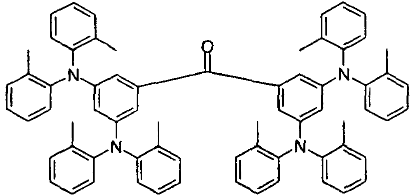 | 37.6 % |
| 35 | 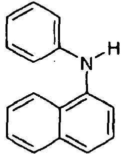 | 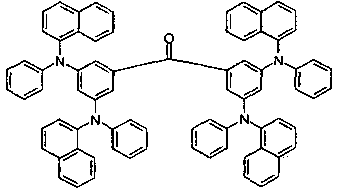 | 46.4 % |
| 36 | 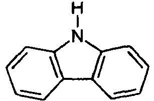 | 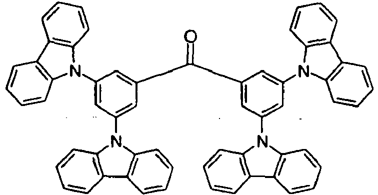 | 43.5 % |
| 37 | 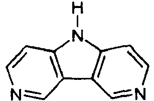 | 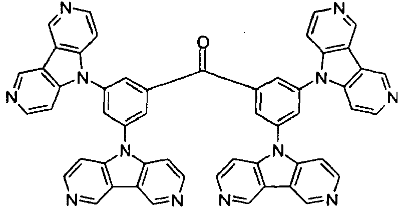 | 44.3 % |

### Beispiel 38: Herstellung und Charakterisierung von organischen Elektrolumineszenzvorrichtungen

Erfindungsgemäße Elektrolumineszenzvorrichtungen können, wie beispielsweise in WO 05/003253 beschrieben, dargestellt werden. Hier werden die Ergebnisse verschiedener OLEDs gegenübergestellt. Der grundlegende Aufbau, die verwendeten Materialien, der Dotierungsgrad und ihre Schichtdicken sind zur besseren Vergleichbarkeit identisch. Die ersten vier Devicebeispiele beschreiben Vergleichsstandards nach dem Stand der Technik, bei dem die Emissionsschicht aus dem Wirtsmaterial (bzw. Matrix) Bis(9,9'-spirobifluoren-2-yl)keton (SK) und verschiedenen Gastmaterialien (Dotanden) TEG für eine grüne Emission bzw. TER für eine rote Emission bzw. TEB für eine blaue Emission besteht. Des Weiteren werden OLEDs verschiedener Aufbauten beschrieben. Analog dem o. g. allgemeinen Verfahren werden OLEDs mit folgendem Aufbau erzeugt:

| | |
|---|---|
| Lochinjektionsschicht (HIL) | 20 nm 2,2',7,7'-Tetrakis(di-para-tolylamino)spiro-9,9'-bifluoren |
| Lochtransportschicht (HTL) | 20 nm NPB (N-Naphthyl-N-phenyl-4,4'-diaminobiphenyl). |
| Elektronenblockierschicht (EBL,optional) | 15 nm EBL-1 (9,9-Bis-(3,5-diphenylaminophenyl)fluoren) |
| Emissionsschicht (EML) | 40 nm Wirtsmaterial: Spiro-Keton (SK) (Bis(9,9'-spirobifluoren-2-yl)keton) als |
| | Vergleich oder erfindungsgemäße Verbindungen. Das Wirtsmaterial kann auch aus einer Mischung zweier Materialien bestehen. Dotand: 10 Vol.-% Dotierung; Verbindungen s. unten. |
| Lochblockierschicht (HBL, optional) | 10 nm erfindungsgemäße Verbindung (s. Tabelle 1) |
| Elektronenleiter (ETL) | 20 nm AlQ₃ (Tris(chinolinato)-aluminium(III)) als Vergleich. |
| Kathode | 1 nm LiF, darauf 100 nm Al. |

Die Strukturen von EBL-1, TEG-1 (synthetisiert nach WO 04/085449), TEG-2 und TEG-3 (jeweils synthetisiert nach US 2001/0019782), TER-1, TER-2, TEB-1, SK, LSK, CBZ und TCTA sind der Übersichtlichkeit halber im Folgenden abgebildet.

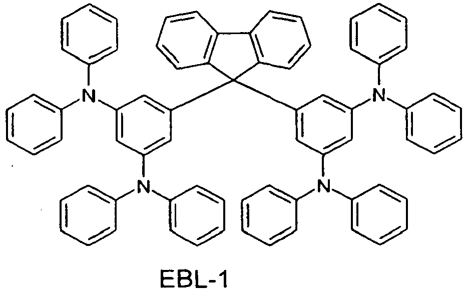

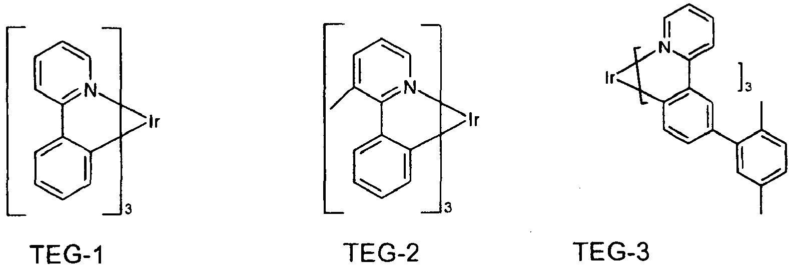

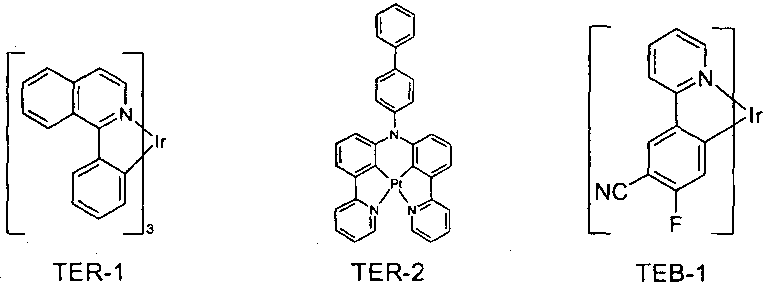

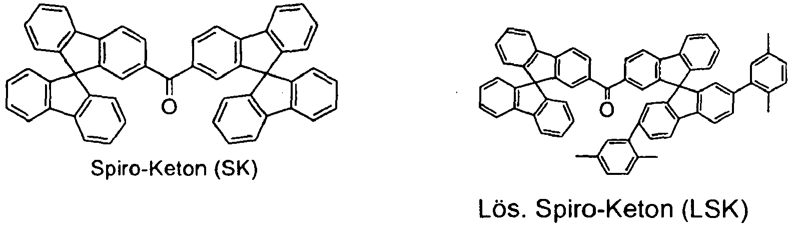

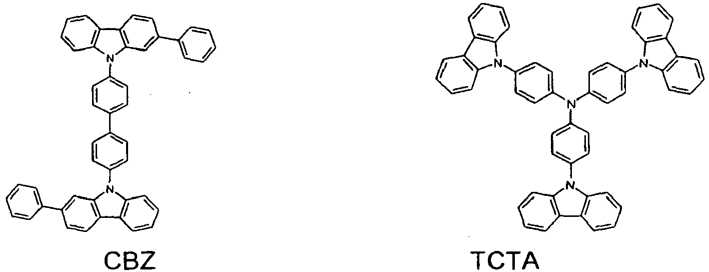

Die verwendeten erfindungsgemäßen Verbindungen 1 bis 6 sind im Folgenden abgebildet:

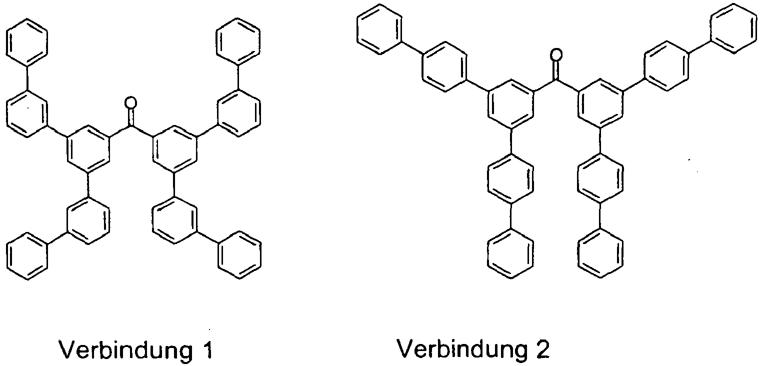

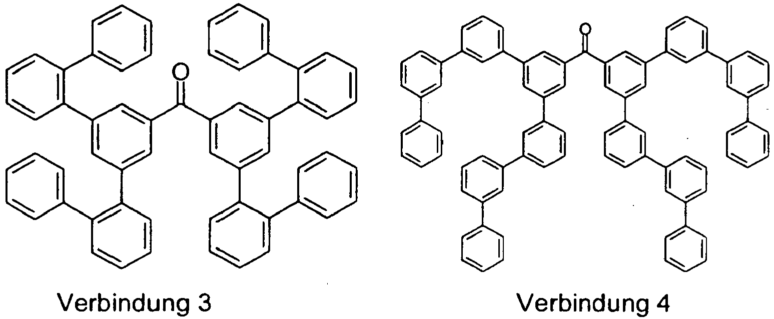

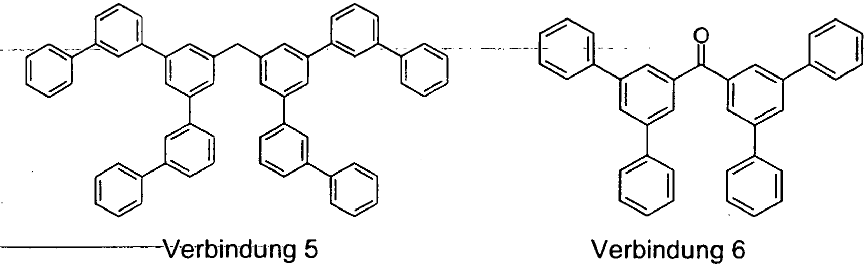

Diese noch nicht optimierten OLEDs werden standardmäßig charakterisiert; hierfür werden die Elektrolumineszenzspektren, die Effizienz (gemessen in cd/A) in Abhängigkeit von der Helligkeit, berechnet aus Strom-Spannungs-Helligkeit-Kennlinien (IUL-Kennlinien), und die Lebensdauer bestimmt.

Wie man Tabelle 1 entnehmen kann, zeigen Elektrolumineszenzvorrichtungen mit den grün bzw. rot emittierenden Dotanden (TEG und TER) in den gemessenen Effizienzen, Spannungen, Farbe und Lebensdauer ein überlegenes Verhalten gegenüber den Vergleichsdevices mit dem Wirtsmaterial SK.

Tabelle 2 führt entsprechende Werte für den blauen Dotanden TEB-1 auf. Das Wirtsmaterial SK ist für blaue Dotanden ungeeignet. Man beobachtet mit diesem Material eine äußerst schwache Elektrolumineszenz, so dass die Werte für die Effizienz und Spannung nicht sinnvoll bestimmt werden können.

**Tabelle 1: Device-Ergebnisse mit erfindungsgemäßen Verbindungen mit TEG-1, TEG-2, TER-1 und TER-2 als Dotanden**

| Bsp. | EML 40 nm | Max. Eff. [cd/A] | Spannung [V] bei 1000 cd/m² | CIE (x, y) | Lebensdauer [h], Anfangshelligkeit 1000 cd/m² |
|---|---|---|---|---|---|
| 39 Vergl. | SK : TEG-1 | 30 | 4.4 | 0.38/ 0.57 | 8000 |
| 40 Vergl. | SK : TEG-2 | 33 | 4.3 | 0.39/ 0.57 | 12000 |
| 41 Vergl. | SK : TER-1 | 6 | 4.2 | 0.69/0.31 | 30000 |
| 42 Vergl. | SK : TER-2 | 5.4 | 5.6 | 0.66/0.34 | 15000 |
| 43 | Verb. 1 : TEG-1 | 37 | 4.0 | 0.34/0.61 | 21000 |
| 44 | Verb. 1 : TEG-2 | 40 | 3.9 | 0.34/0.61 | 25000 |
| 45 | Verb. 1 : TER-1 | 6.1 | 4.1 | 0.69/0.31 | 40000 |
| 46 | Verb. 1 : TER-2 | 7 | 6.3 | 0.66/0.34 | 14000 |
| 47 | EBL-1 / Verb. 1 : TEG-1 | 49 | 4.4 | 0.34/0.61 | 22000 |
| 48 | EBL-1 / Verb. 1 : TEG-2 | 51 | 4.3 | 0.34/0.60 | 28000 |
| 49 | Verb. 2 : TER-2 | 10.5 | 6.6 | 0.66/0.34 | 17000 |
| 50 | Verb. 3 : TEG-1 | 38 | 4.4 | 0.32/0.62 | 12000 |
| 51 | Verb. 1 : TEG-1 30 nm / (HBL= Verb. 1 10 nm) | 37 | 4.0 | 0.34/0.61 | 22000 |
| 52 | Verb. 4 : TEG-1 30 nm / Verb. 4 : TEG-1 30 nm (HBL= Verb. 1 10 nm) | 35 | 4.5 | 0.32/0.61 | 12000 |
| 53 | EBL-1 / Verb. 1 : Verb. 5 : TEG-1 30 nm / (HBL= Verb. 1 10 nm) | 34.3 | 4.5 | 0.32/0.62 | 15000 |
| 54 Vergl. | SK : CBZ : TEG-1 | 36 | 4.7 | 0.34/0.60 | 14000 |
| 55 | Verb. 1 : CBZ : TEG-1 | 40 | 4.7 | 0.32/0.62 | 25000 |
| 56 Vergl. | SK : TCTA : TEG-1 | 38 | 3.7 | 0.35/0.60 | 3000 |
| 57 | Verb. 1 : TCTA : TEG-1 | 42 | 3.7 | 0.32/0.62 | 6000 |

**Tabelle 2: Device-Ergebnisse mit erfindungsgemäßen Verbindungen mit TEB-1 als Dotanden**

| Bsp. | EML 40 nm | Max. Eff. [cd/A] | Spannung [V] bei 1000 cd/m² | CIE (x, y) |
|---|---|---|---|---|
| 58 Vergl. | SK : TEB-1 | - | - | - |
| 59 | EBL-1 / Verb. 1 : TEB-1 | 13 | 8.2 | 0.17/0.28 |
| 60 | EBL-1 / Verb. 4 : TEB-1 | 16 | 9.6 | 0.16/0.27 |
| 61 | EBL-1 / Verb. 6 : TEB-1 | 17 | 8.5 | 0.17/0.27 |
| 62 | EBL-1 / Verb. 1 :Verb. 5 : TEB-1 (HBL= Verb. 1 10 nm) | 16 | 9.3 | 0.15/0.26 |

### Beispiel 63: Herstellung und Charakterisierung von organischen Elektrolumineszenzvorrichtungen aus Lösung

Die erfindungsgemäßen Materialien können auch aus Lösung verwendet werden und führen dort zu wesentlich einfacheren Devices mit weiterhin guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Im vorliegenden Fall werden die erfindungsgemäßen Verbindungen bzw. ebenfalls lösliche Vergleichsverbindungen (LSK) zusammen mit dem Triplettemitter TEG-3 in Toluol bzw. Chlorbenzol gelöst. Die Strukturen der verwendeten Materialien sind oben in Beispiel 38 abgebildet. Der typische Feststoffgehalt solcher Lösungen liegt zwischen 16 und 25 g/L, wenn, wie hier, die für eine Device typische Schichtdicke von 80 nm mittels Spincoating erzielt werden soll. Dabei weist die Vorrichtung den folgenden Aufbau auf:

ITO-Anode / 80 nm Pufferschicht aus PEDOT / 20 nm Interlayer / 80 nm emittierende Schicht enthaltend 17 Gew.-% TEG-3 im entsprechenden Matrixmaterial (siehe Tabelle 3) / Kathode aus 3 nm Ba und 150 nm Al. Strukturierte ITO-Substrate und das Material für die so genannte Pufferschicht (PEDOT, eigentlich PEDOT:PSS) sind käuflich erhältlich (ITO von Technoprint und anderen, PEDOT:PSS als wässrige Dispersion Clevios Baytron P von H.C. Starck). Die verwendete Interlayer dient der Lochinjektion; in diesem Fall wird HIL-012 von Merck verwendet. Die Emissionsschicht wird in einer Inertgasatmosphäre, im vorliegenden Fall Argon, aufgeschleudert und 10 min. bei 120 °C ausgeheizt. Zuletzt wird eine Kathode aus Barium und Aluminium im Vakuum aufgedampft. Zwischen die emittierende Schicht und die Kathode können auch die in den vorgenannten Beispielen verwendeten Lochblockier- bzw. Elektronentransportschichten per Bedampfung aufgebracht werden, auch kann die Interlayer durch eine oder mehrere Schichten ersetzt werden, die lediglich die Bedingung erfüllen müssen, durch den nachgelagerten Prozessierungsschritt der Abscheidung der emittierenden Schicht aus Lösung nicht wieder abgelöst zu werden.

Auch die lösungsprozessierten Devices werden standardmäßig charakterisiert. Tabelle 3 fasst die erhaltenen Daten zusammen. Auch im Bereich löslich prozessierter Devices zeigt sich hier, dass die erfindungsgemäßen Materialien den zuvor zur Verfügung stehenden in Effizienz und Lebensdauer überlegen sind.

**Tabelle 3: Ergebnisse mit aus Lösung prozessierten Materialien**

| Bsp. | EML 40 nm | Max. Eff. [cd/A] | Spannung [V] bei 1000 cd/m² | CIE (x, y) | Lebensdauer [h], Anfangshelligkeit 1000 cd/m² |
|---|---|---|---|---|---|
| 64 Vergl. | LSK : TEG-3 | 22 | 5.9 | 0.35/0.61 | 3000 |
| 65 | Verb.1 : TEG-3 | 28 | 6.8 | 0.35/0.61 | 3500 |
| 66 | Verb.3 : TEG-3 | 28 | 6.7 | 0.31/0.63 | NA |
| 67 | LSK : Verb.5 : TEG-3 | 31 | 5.5 | 0.33/0.62 | 3000 |
| 68 Vergl. | LSK : CBZ : TEG-3 | 29 | 6.0 | 0.35/0.61 | 9500 |
| 69 | Verb. 1 : CBZ : TEG-3 | 34 | 7.5 | 0.33/0.63 | 14000 |
| 70 Vergl. | LSK : TEG-3 (+ aufgedampfte ETL) | 21 | 7.4 | 0.36/061 | 100 |
| 71 | Verb. 1 : TEG-3 (+ aufgedampfte ETL) | 30 | 5.5 | 0.34/0.62 | 8000 |

## Patentansprüche

1. Verbindung gemäß Formel (1), 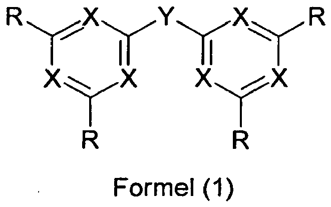 wobei für die verwendeten Symbole gilt:
Y ist C=O oder C(R¹)₂;
X ist bei jedem Auftreten gleich oder verschieden CR² oder N, wobei alle Symbole X in einem Cyclus entweder für CR² stehen oder wobei alle Symbole X in einem Cyclus für N stehen;
R ist bei jedem Auftreten gleich oder verschieden ein aromatisches oder heteroaromatisches Ringsystem mit 5 bis 30 aromatischen Ringatomen, welches durch einen oder mehrere Reste R³ substituiert sein kann, oder eine Gruppe N(Ar)₂, C(=O)Ar oder P(O)(Ar)₂;
Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R³ substituiert sein kann; dabei können auch zwei Reste Ar, welche an dasselbe Stickstoff- oder Phosphoratom binden, durch eine Einfachbindung oder eine Brücke, ausgewählt aus B(R⁴), C(R⁴)₂, Si(R⁴)₂, C=O, C=NR⁴, C=C(R⁴)₂, O, S, S=O, SO₂, N(R⁴), P(R⁴) und P(=O)R⁴, miteinander verknüpft sein;
R¹ ist bei jedem Auftreten gleich oder verschieden H oder eine lineare Alkylgruppe mit 1 bis 20 C-Atomen oder eine verzweigte oder cyclische Alkylgruppe mit 3 bis 20 C-Atomen; dabei können mehrere Reste R¹ miteinander ein Ringsystem bilden;
R² ist H;
R³ ist bei jedem Auftreten gleich oder verschieden H, D, F, Cl, Br, I, CHO, N(Ar)₂, C(=O)Ar, P(=O)(Ar)₂, S(=O)Ar, S(=O)₂Ar, CR²=CR²Ar, CN, NO₂, Si(R⁴)₃, B(OR⁴)₂, B(R⁴)₂, B(N(R⁴)₂)₂, OSO₂R⁴, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R⁴ substituiert sein kann, wobei eine oder mehrere nicht benachbarte CH₂-Gruppen durch R⁴C=CR⁴, C=C , Si(R⁴)₂, Ge(R⁴)₂, Sn(R⁴)₂, C=O, C=S, C=Se, C=NR⁴, P(=O)(R⁴), SO, SO₂, NR⁴, O, S oder CONR⁴ ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 60 aromatischen Ringatomen, die durch einen oder mehrere Reste R⁴ substituiert sein kann, oder eine Kombination dieser Systeme; dabei können zwei oder mehrere benachbarte Substituenten R³ auch miteinander ein mono- oder polycyclisches, aliphatisches oder aromatisches Ringsystem bilden;
R⁴ ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R⁴ auch miteinander ein mono- oder polycyclisches, aliphatisches oder aromatisches Ringsystem bilden.
dabei sind die folgenden Verbindungen von der Erfindung ausgenommen: 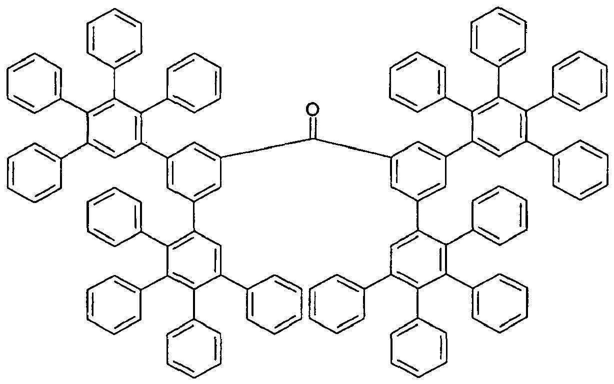 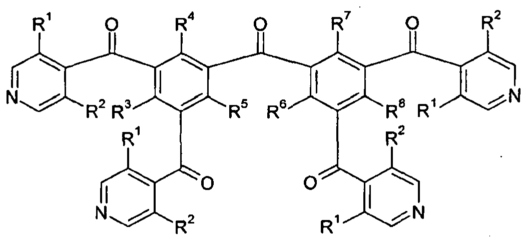 wobei für die verwendeten Symbole R¹ bis R⁸ gilt: R¹ bis R⁸ sind unabhängig voneinander und stehen für ein Wasserstoffatom, eine Methylgruppe, eine Ethylgruppe, eine n-Propylgruppe, eine Isopropylgruppe, eine n-Butylgruppe, eine Isobutylgruppe, eine sekundäre Butylgruppe oder eine tertiäre Butylgruppe.

2. Verbindung nach Anspruch 1, **dadurch gekennzeichnet, dass** die Gruppe R, wenn sie für ein aromatisches oder heteroaromatisches Ringsystem steht, ausgewählt ist aus den Gruppen Phenyl, o-Biphenyl, m-Biphenyl, p-Biphenyl, o-Terphenyl, m-Terphenyl, p-Terphenyl, 3,5-(Diphenyl)phenyl, m-Quarterphenyl, 2-Fluorenyl, 2-Spirobifluorenyl, 1-Naphthyl, 2-Naphthyl, 1-, 2- oder 9-Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, 2-, 4- oder 5-Pyrimidinyl, 1,3,5-Triazinyl, insbesondere substituiert mit aromatischen Gruppen, N-Benzimidazolyl, Phenyl-N-benzimidazolyl, N-Phenylbenzimidazolyl, Phenyl-N-phenylbenzimidazolyl, Thiophen, Oxazol, Oxadiazol, Thiadiazol oder Benzthiazol, wobei diese Gruppen jeweils durch einen oder mehrere Substituenten R³ substituiert sein können; bevorzugt ist R ausgewählt aus Strukturen der Formeln (2) bis (16), wobei die gestrichelte Bindung jeweils die Verknüpfung dieser Einheit andeutet und wobei die Gruppen jeweils durch einen oder mehrere Reste R³ substituiert sein können:

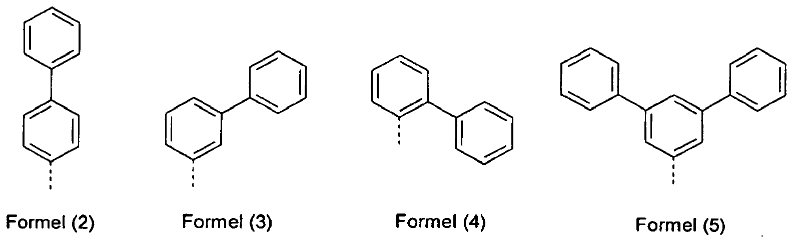

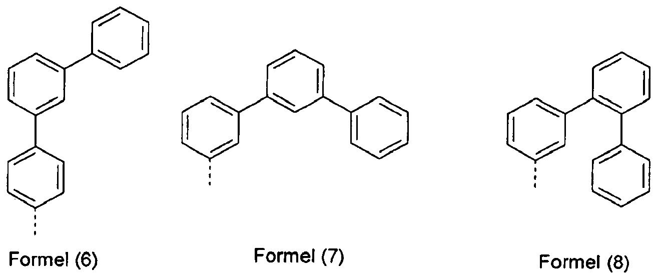

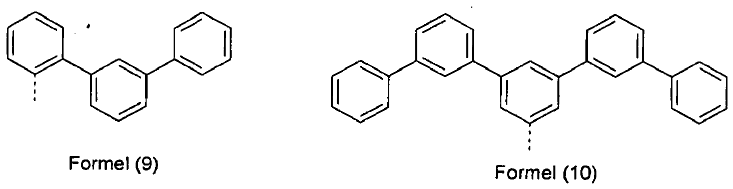

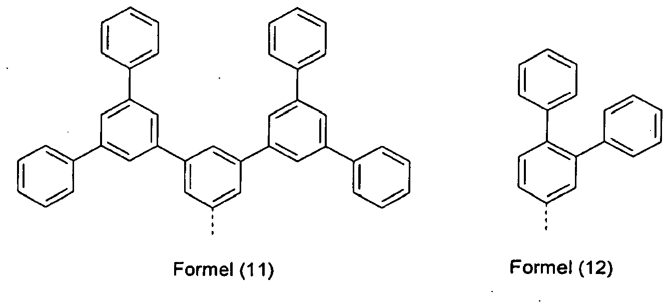

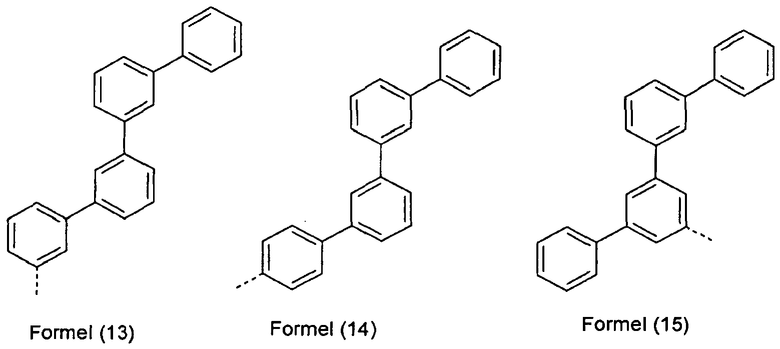

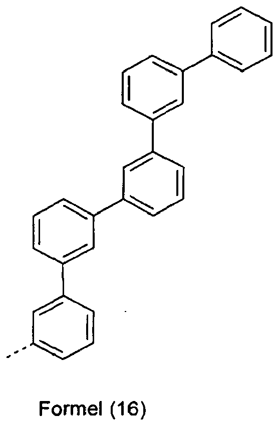

3. Verbindung nach Anspruch 1 oder 2, **dadurch gekennzeichnet, dass** der Rest R, wenn er für eine Gruppe N(Ar)₂ steht, ausgewählt ist aus den Gruppen der Formel (17) oder der Formel (18), 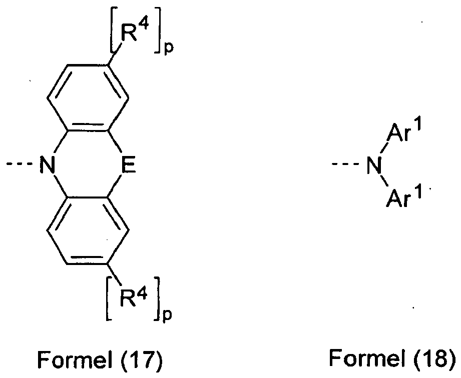 wobei R⁴ die in Anspruch 1 aufgeführte Bedeutung hat und weiterhin gilt:
E steht für eine Einfachbindung, O, S, N(R⁴) oder C(R⁴)₂;
Ar¹ ist gleich oder verschieden bei jedem Auftreten ein aromatisches oder heteroaromatisches Ringsystem mit 5 bis 24 aromatischen Ringatomen oder eine Triarylamingruppe mit 15 bis 30 aromatischen Ringatomen, welche jeweils mit einem oder mehreren Resten R⁴ substituiert sein kann, bevorzugt eine Aryl- oder Heteroarylgruppe mit 6 bis 14 aromatischen Ringatomen oder eine Triarylamingruppe mit 18 bis 30 aromatischen Ringatomen, bevorzugt mit 18 bis 22 aromatischen Ringatomen, welche jeweils mit einem oder mehreren Resten R⁴ substituiert sein kann;
p ist bei jedem Auftreten gleich oder verschieden 0 oder 1.

4. Verbindung nach einem oder mehreren der Ansprüche 1 bis 3, **dadurch gekennzeichnet, dass** alle Gruppen R in Verbindungen der Formel (1) gleich gewählt sind oder dass beide Substituenten R, welche an denselben Ring binden, jeweils gleich gewählt sind, sich jedoch von den Substituenten R am anderen Ring unterscheiden.

5. Verbindung nach einem oder mehreren der Ansprüche 1 bis 4, ausgewählt aus Verbindungen der Formeln (19), (20) und (21), 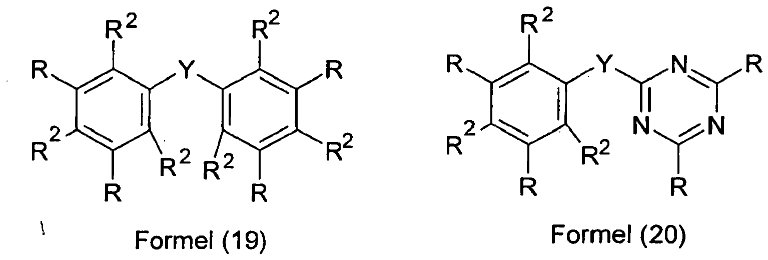 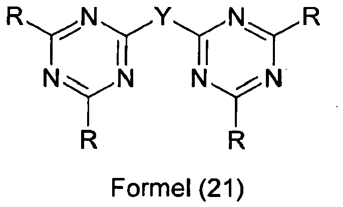 wobei Ar, R³ und R⁴ wie in Anspruch 1 definiert sind und für die weiteren verwendeten Symbole gilt:
Y ist C=O oder C(R¹)₂;
R ist bei jedem Auftreten gleich oder verschieden ein aromatisches oder heteroaromatisches Ringsystem ausgewählt aus der Gruppe bestehend aus Phenyl, o-Biphenyl, m-Biphenyl, p-Biphenyl, o-Terphenyl, m-Terphenyl, p-Terphenyl, 3,5-(Diphenyl)phenyl, m-Quarterphenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Benzimidazolyl, Phenyl-N-benzimidazolyl, N-Phenylbenzimidazolyl oder Phenyl-N-phenylbenzimidazolyl, insbesondere ausgewählt aus den Formeln (2) bis (16) gemäß Anspruch 4 oder eine Gruppe N(Ar)₂, ausgewählt aus den Formeln (17) oder (18) gemäß Anspruch 5, C(=O)Ar oder P(=O)Ar₂;
R¹ ist bei jedem Auftreten gleich oder verschieden H, eine lineare Alkylgruppe mit 1 bis 10 C-Atomen, bevorzugt mit 1 bis 6 C-Atomen, insbesondere Methyl, oder eine verzweigte oder cyclische Alkylgruppe mit 3 bis 10 C-Atomen, bevorzugt mit 3 bis 6 C-Atomen; dabei können mehrere Gruppen R¹ miteinander ein Ringsystem bilden;
R² ist H.

6. Verbindung nach einem oder mehreren der Ansprüche 1 bis 5 gemäß Formel (22), 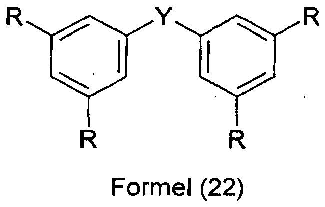 wobei Ar, R³ und R⁴ wie oben definiert sind und für die weiteren verwendeten Symbole gilt:
Y ist C=O, CH₂ oder C(Alkyl)₂, wobei Alkyl eine Alkylgruppe mit 1 bis 6 C-Atomen darstellt, insbesondere Methyl;
R ist bei jedem Auftreten gleich oder verschieden ein aromatisches oder heteroaromatisches Ringsystem ausgewählt aus der Gruppe bestehend aus Phenyl, o-Biphenyl, m-Biphenyl, p-Biphenyl, o-Terphenyl, m-Terphenyl, o-Terphenyl, 3,5-(Diphenyl)phenyl, m-Quarterphenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Benzimidazolyl, Phenyl-N-benzimidazolyl, N-Phenylbenzimidazolyl oder Phenyl-N-phenylbenzimidazolyl, insbesondere ausgewählt aus den Formeln (2) bis (16) gemäß Anspruch 4, oder eine Gruppe N(Ar)₂, bevorzugt ausgewählt aus den Formeln (17) oder (18) gemäß Anspruch 5, oder C(=O)Ar oder P(=O)Ar₂.

7. Verbindung nach einem oder mehreren der Ansprüche 1 bis 6, **dadurch gekennzeichnet, dass** das Symbol Ar gleich oder verschieden bei jedem Auftreten für ein aromatisches Ringsystem mit 6 bis 30 aromatischen Ringatomen steht, insbesondere ausgewählt aus der Gruppe bestehend aus Phenyl, o-Biphenyl, m-Biphenyl, p-Biphenyl, o-Terphenyl, m-Terphenyl, p-Terphenyl, 3,5-(Diphenyl)phenyl, m-Quarterphenyl, 1-Naphthyl, 2-Naphthyl, Anthracenyl, Phenylanthracenyl, 1- oder 2-Naphthylanthracenyl, Binaphthyl, Pyrenyl, Fluoranthenyl, 2-, 3-, 4-, 5-, 6- oder 7-Benzanthracenyl, N-Benzimidazolyl, Phenyl-N-benzimidazolyl, N-Phenylbenzimidazolyl oder Phenyl-N-phenylbenzimidazolyl.

8. Verfahren zur Herstellung einer Verbindung nach einem oder mehreren der Ansprüche 1 bis 7, umfassend die Kupplung von einem substituierten oder unsubstituierten Bis(3,5-dibrombenzophenon) mit einer aromatischen oder heteroaromatischen Boronsäure oder einem entsprechenden Boronsäurederivat unter Metallkatalyse oder mit einem primären oder sekundären aromatischen Amin unter Metallkatalyse oder mit einem Metallcyanid unter Metallkatalyse.

9. Lösung enthaltend mindestens eine Verbindung nach einem oder mehreren der Ansprüche 1 bis 7 und mindestens ein organisches Lösemittel.

10. Verwendung einer Verbindung nach einem oder mehreren der Ansprüche 1 bis 7 in einer elektronischen Vorrichtung, insbesondere in einer organischen Elektrolumineszenzvorrichtung.

11. Organische elektronische Vorrichtung, insbesondere ausgewählt aus der Gruppe bestehend aus organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren, enthaltend mindestens eine Verbindung nach einem oder mehreren der Ansprüche 1 bis 7.

12. Organische Elektrolumineszenzvorrichtung nach Anspruch 11, **dadurch gekennzeichnet, dass** die Verbindung nach einem oder mehreren der Ansprüche 1 bis 7 als Matrixmaterial für eine fluoreszierende oder phosphoreszierende Verbindung in einer emittierenden Schicht oder als Lochtransportmaterial bzw. Lochinjektionsmaterial oder als Elektronenblockiermaterial bzw. Exzitonenblockiermaterial oder als Elektronentransportmaterial oder als Lochblockiermaterial eingesetzt wird.

## Claims

1. Compound of the formula (1), 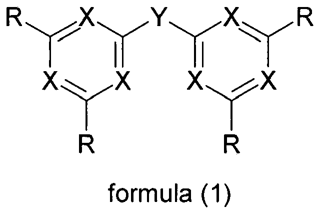 where the following applies to the symbols used:
Y is C=O or C(R¹)₂;
X is on each occurrence, identically or differently, CR² or N, where all symbols X in a ring system either stand for CR² or where all symbols X in a ring system stand for N;
R is on each occurrence, identically or differently, an aromatic or heteroaromatic ring system having 5 to 30 aromatic ring atoms, which may be substituted by one or more radicals R³, or an N(Ar)₂, C(=O)Ar or P(O)(Ar)₂ group;
Ar is on each occurrence, identically or differently, an aromatic or heteroaromatic ring system having 5 to 30 aromatic ring atoms, which may be substituted by one or more non-aromatic radicals R³; two radicals Ar here which are bonded to the same nitrogen or phosphorus atom may also be linked to one another by a single bond or a bridge selected from B(R⁴), C(R⁴)₂, Si(R⁴)₂, C=O, C=NR⁴, C=C(R⁴)₂, O, S, S=O, SO₂, N(R⁴), P(R⁴) and P(=O)R⁴;
R¹ is on each occurrence, identically or differently, H or a linear alkyl group having 1 to 20 C atoms or a branched or cyclic alkyl group having 3 to 20 C atoms; a plurality of radicals R¹ here may form a ring system with one another;
R² is H;
R³ is on each occurrence, identically or differently, H, D, F, Cl, Br, I, CHO, N(Ar)₂, C(=O)Ar, P(=O)(Ar)₂, S(=O)Ar, S(=O)₂Ar, CR²=CR²Ar, CN, NO₂, Si(R⁴)₃, B(OR⁴)₂, B(R⁴)₂, B(N(R⁴)₂)₂, OSO₂R⁴, a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R⁴, where one or more non-adjacent CH₂ groups may be replaced by R⁴C=CR⁴, C=C, Si(R⁴)₂, Ge(R⁴)₂, Sn(R⁴)₂, C=O, C=S, C=Se, C=NR⁴, P(=O)(R⁴), SO, SO₂, NR⁴, O, S or CONR⁴ and where one or more H atoms may be replaced by F, Cl, Br, I, CN or NO₂, or an aromatic or heteroaromatic ring system having 5 to 60 aromatic ring atoms, which may in each case be substituted by one or more radicals R⁴, or an aryloxy or heteroaryloxy group having 5 to 60 aromatic ring atoms, which may be substituted by one or more radicals R⁴, or a combination of these systems; two or more adjacent substituents R³ here may also form a mono- or polycyclic, aliphatic or aromatic ring system with one another;
R⁴ is on each occurrence, identically or differently, H, D or an aliphatic, aromatic and/or heteroaromatic hydrocarbon radical having 1 to 20 C atoms, in which, in addition, H atoms may be replaced by F; two or more adjacent substituents R⁴ here may also form a mono- or polycyclic, aliphatic or aromatic ring system with one another;
the following compounds are excluded from the invention: 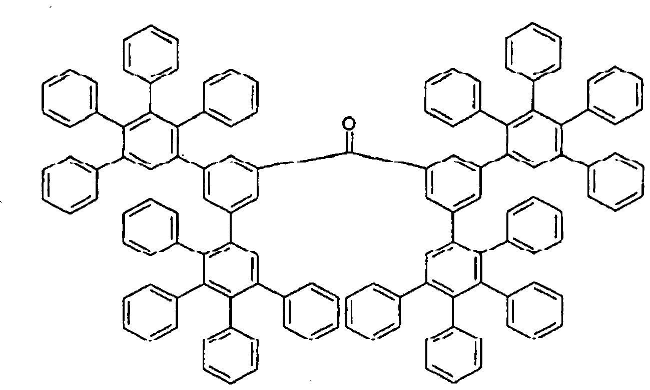 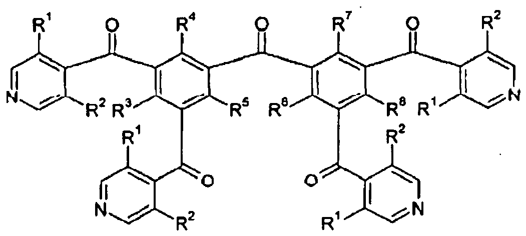 where the following applies to the symbols R¹ to R⁸ used: R¹ to R⁸ are independent of one another and stand for a hydrogen atom, a methyl group, an ethyl group, an n-propyl group, an isopropyl group, an n-butyl group, an isobutyl group, a secondary butyl group or a tertiary butyl group.

2. Compound according to Claim 1, **characterised in that** the group R, if it stands for an aromatic or heteroaromatic ring system, is selected from the groups phenyl, o-biphenyl, m-biphenyl, p-biphenyl, o-terphenyl, m-terphenyl, p-terphenyl, 3,5-(diphenyl)phenyl, m-quaterphenyl, 2-fluorenyl, 2-spirobifluorenyl, 1-naphthyl, 2-naphthyl, 1-, 2- or 9-anthracenyl, phenylanthracenyl, 1- or 2-naphthylanthracenyl, binaphthyl, pyrenyl, fluoranthenyl, 2-, 3-, 4-, 5-, 6- or 7-benzanthracenyl, 2-, 4- or 5-pyrimidinyl, 1,3,5-triazinyl, in particular substituted by aromatic groups, N-benzimidazolyl, phenyl-N-benzimidazolyl, N-phenylbenzimidazolyl, phenyl-N-phenylbenzimidazolyl, thiophene, oxazole, oxadiazole, thiadiazole or benzothiazole, where these groups may each be substituted by one or more substituents R³; R is preferably selected from structures of the formulae (2) to (16), where the dashed bond in each case indicates the linking of this unit and where the groups may each be substituted by one or more radicals R³:

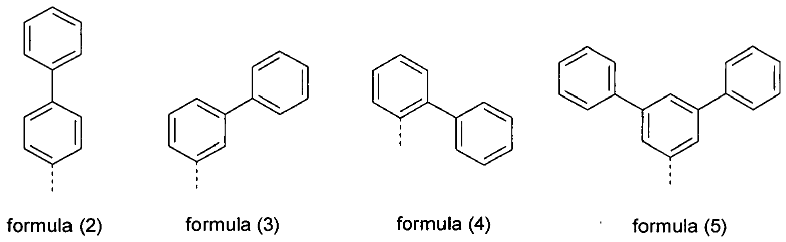

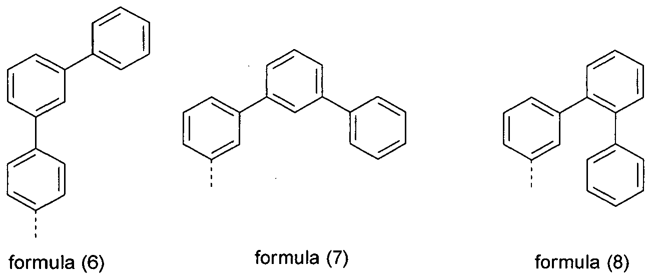

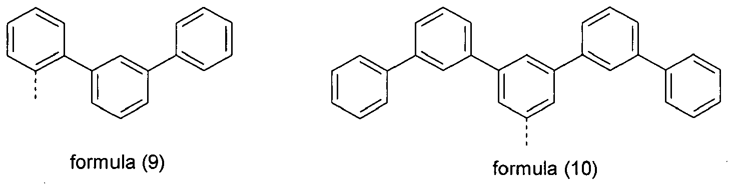

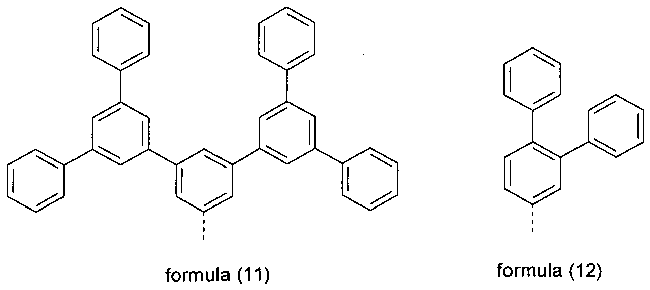

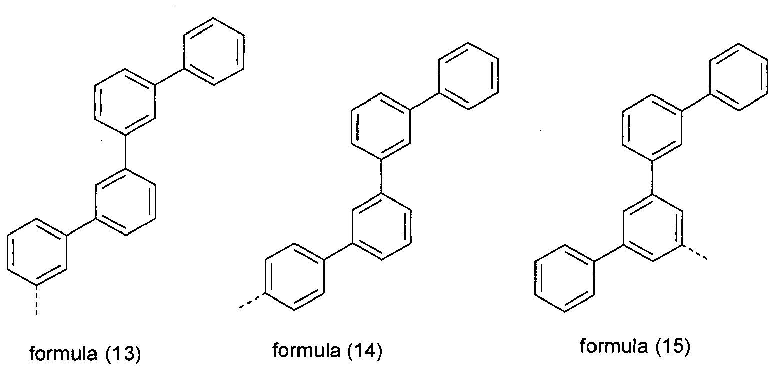

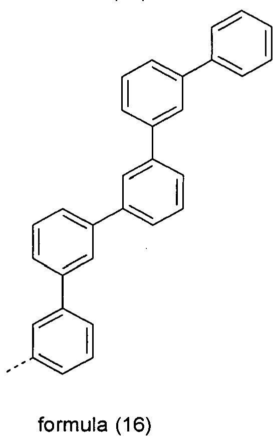

3. Compound according to Claim 1 or 2, **characterised in that** the radical R, if it stands for an N(Ar)₂ group, is selected from the groups of the formula (17) or of the formula (18), 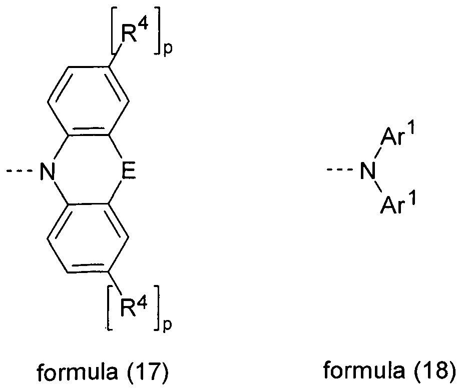 where R⁴ has the meaning indicated in Claim 1 and furthermore:
E stands for a single bond, O, S, N(R⁴) or C(R⁴)₂;
Ar¹ is, identically or differently on each occurrence, an aromatic or heteroaromatic ring system having 5 to 24 aromatic ring atoms or a triarylamine group having 15 to 30 aromatic ring atoms, each of which may be substituted by one or more radicals R⁴, preferably an aryl or heteroaryl group having 6 to 14 aromatic ring atoms or a triarylamine group having 18 to 30 aromatic ring atoms, preferably having 18 to 22 aromatic ring atoms, each of which may be substituted by one or more radicals R⁴;
p is on each occurrence, identically or differently, 0 or 1.

4. Compound according to one or more of Claims 1 to 3, **characterised in that** all groups R in compounds of the formula (1) are selected identically or **in that** both substituents R which are bonded to the same ring are each selected identically, but differ from the substituents R on the other ring.

5. Compound according to one or more of Claims 1 to 4, selected from compounds of the formulae (19), (20) and (21), 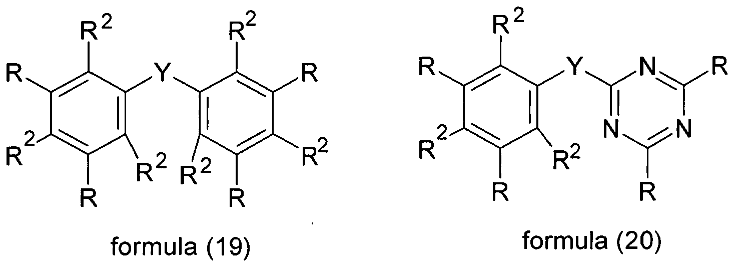 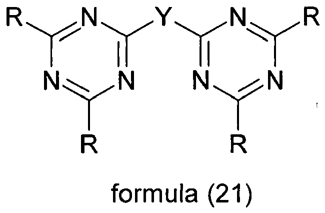 where Ar, R³ and R⁴ are as defined in Claim 1 and the following applies to the other symbols used:
Y is C=O or C(R¹)₂;
R is on each occurrence, identically or differently, an aromatic or heteroaromatic ring system selected from the group consisting of phenyl, o-biphenyl, m-biphenyl, p-biphenyl, o-terphenyl, m-terphenyl, p-terphenyl, 3,5-(diphenyl)phenyl, m-quaterphenyl, 1-naphthyl, 2-naphthyl, anthracenyl, phenylanthracenyl, 1- or 2-naphthylanthracenyl, binaphthyl, pyrenyl, fluoranthenyl, 2-, 3-, 4-, 5-, 6- or 7-benzanthracenyl, N-benzimidazolyl, phenyl-N-benzimidazolyl, N-phenylbenzimidazolyl or phenyl-N-phenylbenzimidazolyl, in particular selected from the formulae (2) to (16) according to Claim 2 or an N(Ar)₂ group selected from the formulae (17) and (18) according to Claim 3, C(=O)Ar or P(=O)Ar₂;
R¹ is on each occurrence, identically or differently, H, a linear alkyl group having 1 to 10 C atoms, preferably having 1 to 6 C atoms, in particular methyl, or a branched or cyclic alkyl group having 3 to 10 C atoms, preferably having 3 to 6 C atoms; a plurality of groups R¹ here may form a ring system with one another;
R² is H.

6. Compound according to one or more of Claims 1 to 5 of the formula (22), 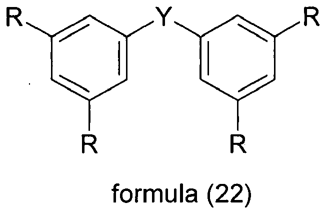 where Ar, R³ and R⁴ are as defined above, and the following applies to the other symbols used:
Y is C=O, CH₂ or C(alkyl)₂, where alkyl represents an alkyl group having 1 to 6 C atoms, in particular methyl;
R is on each occurrence, identically or differently, an aromatic or heteroaromatic ring system selected from the group consisting of phenyl, o-biphenyl, m-biphenyl, p-biphenyl, o-terphenyl, m-terphenyl, p-terphenyl, 3,5-(diphenyl)phenyl, m-quaterphenyl, 1-naphthyl, 2-naphthyl, anthracenyl, phenylanthracenyl, 1- or 2-naphthylanthracenyl, binaphthyl, pyrenyl, fluoranthenyl, 2-, 3-, 4-, 5-, 6- or 7-benzanthracenyl, N-benzimidazolyl, phenyl-N-benzimidazolyl, N-phenylbenzimidazolyl or phenyl-N-phenylbenzimidazolyl, in particular selected from the formulae (2) to (16) according to Claim 2 or an N(Ar)₂ group, preferably selected from the formulae (17) and (18) according to Claim 3, or C(=O)Ar or P(=O)Ar₂.

7. Compound according to one or more of Claims 1 to 6, **characterised in that** the symbol Ar stands, identically or differently on each occurrence, for an aromatic ring system having 6 to 30 aromatic ring atoms, in particular selected from the group consisting of phenyl, o-biphenyl, m-biphenyl, p-biphenyl, o-terphenyl, m-terphenyl, p-terphenyl, 3,5-(diphenyl)phenyl, m-quaterphenyl, 1-naphthyl, 2-naphthyl, anthracenyl, phenylanthracenyl, 1- or 2-naphthylanthracenyl, binaphthyl, pyrenyl, fluoranthenyl, 2-, 3-, 4-, 5-, 6- or 7-benzanthracenyl, N-benzimidazolyl, phenyl-N-benzimidazolyl, N-phenylbenzimidazolyl or phenyl-N-phenylbenzimidazolyl.

8. Process for the preparation of a compound according to one or more of Claims 1 to 7, comprising the coupling of a substituted or unsubstituted bis(3,5-dibromobenzophenone) to an aromatic or heteroaromatic boronic acid or a corresponding boronic acid derivative with metal catalysis or to a primary or secondary aromatic amine with metal catalysis or to a metal cyanide with metal catalysis.

9. Solution comprising at least one compound according to one or more of Claims 1 to 7 and at least one organic solvent.

10. Use of a compound according to one or more of Claims 1 to 7 in an electronic device, in particular in an organic electroluminescent device.

11. Organic electronic device, in particular selected from the group consisting of organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic laser diodes (O-lasers) and organic photoreceptors, comprising at least one compound according to one or more of Claims 1 to 7.

12. Organic electroluminescent device according to Claim 11, **characterised in that** the compound according to one or more of Claims 1 to 7 is employed as matrix material for a fluorescent or phosphorescent compound in an emitting layer or as hole-transport material or hole-injection material or as electron-blocking material or exciton-blocking material or as electron-transport material or as hole-blocking material.

## Revendications

1. Composé de la formule (1) : 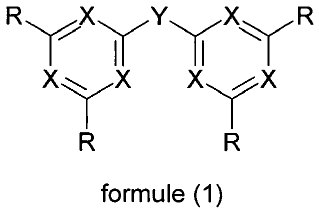 dans laquelle ce qui suit s'applique aux symboles utilisés :
Y est C=O ou C(R¹)₂ ;
X est, pour chaque occurrence, de manière identique ou différente, CR² ou N, où soit tous les symboles X dans un système de cycle représentent CR², soit tous les symboles X dans un système de cycle représentent N ;
R est, pour chaque occurrence, de manière identique ou différente, un système de cycle aromatique ou hétéroaromatique comportant 5 à 30 atomes de cycle aromatique, lequel peut être substitué par un radical ou plusieurs radicaux R³, ou un groupe N(Ar)₂, C(=O)Ar ou P(O)(Ar)₂ ;
Ar est, pour chaque occurrence, de manière identique ou différente, un système de cycle aromatique ou hétéroaromatique comportant 5 à 30 atomes de cycle aromatique, lequel peut être substitué par un radical ou plusieurs radicaux non aromatique(s) R^{3;} deux radicaux Ar ici qui sont liés au même atome d'azote ou de phosphore peuvent également être liés l'un à l'autre par une liaison simple ou un pont choisi parmi B(R⁴), C(R⁴)₂, Si(R⁴)₂, C=O, C=NR⁴, C=C(R⁴)₂, O, S, S=O, SO₂, N(R⁴), P(R⁴) et P(=O)R⁴ ;
R¹ est, pour chaque occurrence, de manière identique ou différente, H ou un groupe alkyle linéaire comportant 1 à 20 atome(s) de C ou un groupe alkyle ramifié ou cyclique comportant 3 à 20 atomes de C ; une pluralité de radicaux R¹ ici peuvent former un système de cycle les uns avec les autres ;
R² est H ;
R³ est, pour chaque occurrence, de manière identique ou différente, H, D, F, Cl, Br, I, CHO, N(Ar)₂, C(=O)Ar, P(=O)(Ar)₂, S(=O)Ar, S(=O)₂Ar, CR²=CR²Ar, CN, NO₂, Si(R⁴)₃, B(OR⁴)₂, B(R⁴)₂, B(N(R⁴)₂)₂, OSO₂R⁴, un groupe alkyle, alcoxy ou thioalcoxy en chaîne droite comportant 1 à 40 atome(s) de C ou un groupe alkyle, alcoxy ou thioalcoxy ramifié ou cyclique comportant 3 à 40 atomes de C, dont chacun peut être substitué par un radical ou plusieurs radicaux R⁴, où un ou plusieurs groupe(s) CH₂ non adjacents peut/peuvent être remplacé(s) par R⁴C=CR⁴, C=C, Si(R⁴)₂, Ge(R⁴)₂, Sn(R⁴)₂, C=O, C=S, C=Se, C=NR⁴, P(=O)(R⁴), SO, SO₂, NR⁴, O, S ou CONR⁴ et où un ou plusieurs atome(s) de H peut/peuvent être remplacé(s) par F, CI, Br, I, CN ou NO₂, ou un système de cycle aromatique ou hétéroaromatique comportant 5 à 60 atomes de cycle aromatique, lequel peut dans chaque cas être substitué par un radical ou plusieurs radicaux R⁴, ou un groupe aryloxy ou hétéroaryloxy comportant 5 à 60 atomes de cycle aromatique, lequel peut être substitué par un radical ou plusieurs radicaux R⁴, ou une combinaison de ces systèmes ; deux substituants R³ adjacents ou plus ici peuvent également former un système de cycle aliphatique ou aromatique monocyclique ou polycyclique l'un avec l'autre ou les uns avec les autres ;
R⁴ est, pour chaque occurrence, de manière identique ou différente, H, D ou un radical hydrocarbone aliphatique, aromatique et/ou hétéroaromatique comportant 1 à 20 atome(s) de C, où, en outre, des atomes de H peuvent être remplacés par F ; deux substituants R⁴ adjacents ou plus ici peuvent également former un système de cycle aliphatique ou aromatique monocyclique ou polycyclique l'un avec l'autre ou les uns avec les autres ;
les composés qui suivent sont exclus de l'invention : 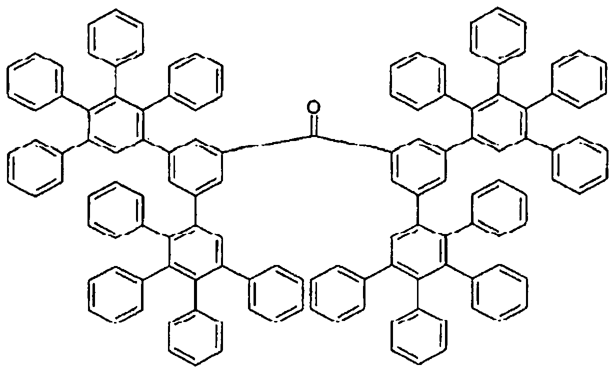 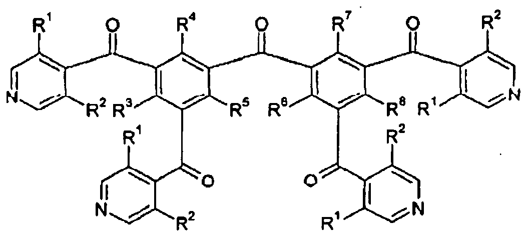 où ce qui suit s'applique aux symboles R¹ à R⁸ utilisés : R¹ à R⁸ sont indépendants les uns des autres et représentent un atome d'hydrogène, un groupe méthyle, un groupe éthyle, un groupe n-propyle, un groupe isopropyle, un groupe n-butyle, un groupe isobutyle, un groupe butyle secondaire ou un groupe butyle tertiaire.

2. Composé selon la revendication 1, **caractérisé en ce que** le groupe R, s'il représente un système de cycle aromatique ou hétéroaromatique, est choisi parmi les groupes phényle, o-biphényle, m-biphényle, p-biphényle, o-terphényle, m-terphényle, p-terphényle, 3,5-(diphényl)phényle, m-quaterphényle, 2-fluorényle, 2-spiro-bifluorényle, 1-naphtyle, 2-naphtyle, 1-, 2- ou 9-anthracényle, phénylanthracényle, 1- ou 2-naphtylanthracényle, binaphtyle, pyrényle, fluoranthényle, 2-, 3-, 4-, 5-, 6- ou 7-benzanthracényle, 2-, 4- ou 5-pyrimidinyle, 1,3,5-triazinyle, en particulier substitués par des groupes aromatiques, N-benzimidazolyle, phényl-N-benzimidazolyle, N-phénylbenzimidazolyle, phényl-N-phénylbenzimidazolyle, thio-phène, oxazole, oxadiazole, thiadiazole ou benzothiazole, où ces groupes peuvent chacun être substitués par un ou plusieurs substituant(s) R^{3;} R est de façon préférable choisi parmi des structures des formules (2) à (16), où le lien en pointillés dans chaque cas indique la liaison de cette unité et où les groupes peuvent chacun être substitués par un radical ou plusieurs radicaux R^{3:}

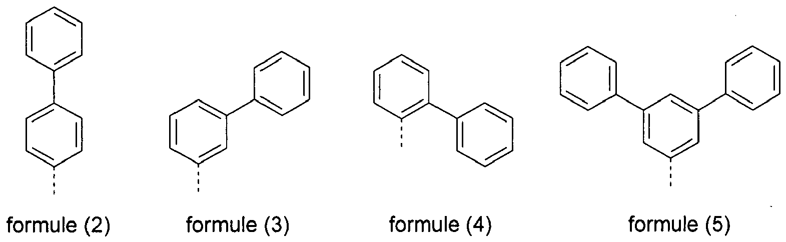

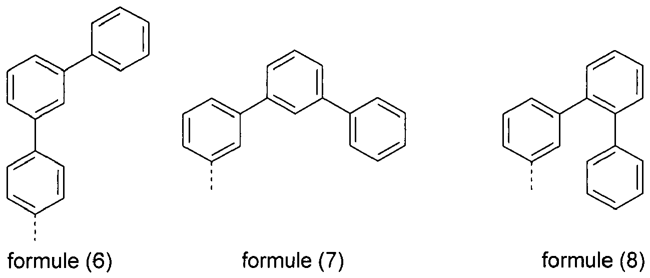

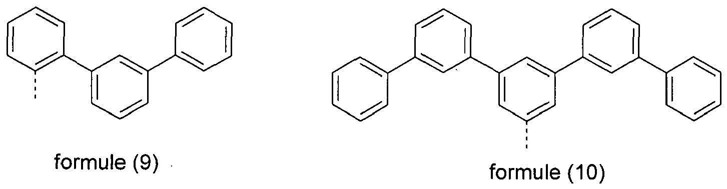

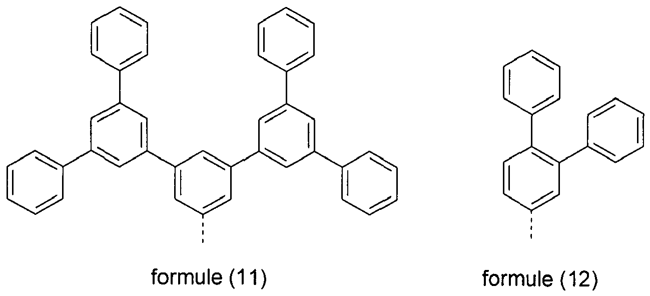

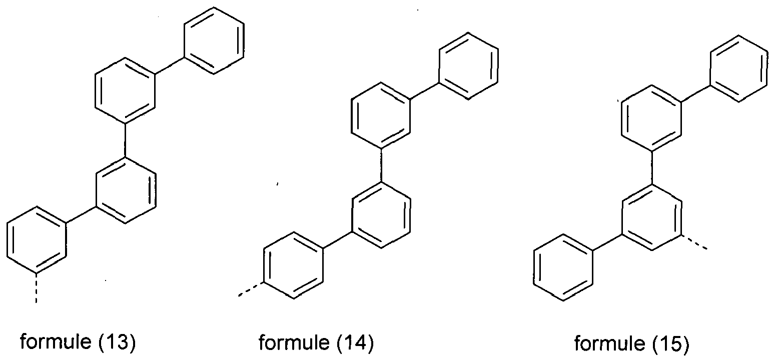

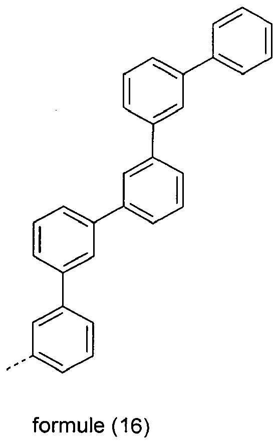

3. Composé selon la revendication 1 ou 2, **caractérisé en ce que** le radical R, s'il représente un groupe N(Ar)₂, est choisi parmi les groupes de la formule (17) ou de la formule (18) : 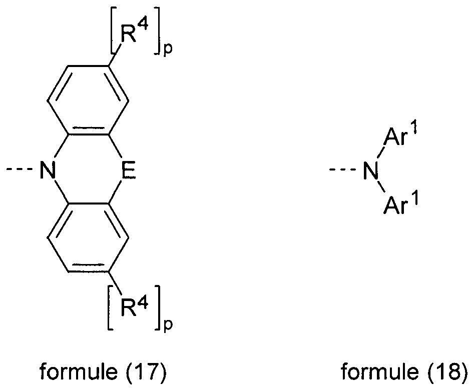 dans lesquelles R⁴ présente la signification indiquée selon la revendication 1 et en outre :
E représente une liaison simple, O, S, N(R⁴) ou C(R⁴)_{2 ;}
Ar¹ est, de manière identique ou différente pour chaque occurrence, un système de cycle aromatique ou hétéroaromatique comportant 5 à 24 atomes de cycle aromatique ou un groupe triarylamine comportant 15 à 30 atomes de cycle aromatique, dont chacun peut être substitué par un radical ou plusieurs radicaux R⁴, de façon préférable un groupe aryle ou hétéroaryle comportant 6 à 14 atomes de cycle aromatique ou un groupe triarylamine comportant 18 à 30 atomes de cycle aromatique, de façon préférable comportant 18 à 22 atomes de cycle aromatique, dont chacun peut être substitué par un radical ou plusieurs radicaux R^{4 ;}
p est, pour chaque occurrence, de manière identique ou différente, 0 ou 1.

4. Composé selon une ou plusieurs des revendications 1 à 3, **caractérisé en ce que** tous les groupes R dans les composés de la formule (1) sont choisis de manière identique ou **en ce que** les deux substituants R qui sont liés au même cycle sont chacun choisis de manière identique, mais diffèrent des substituants R sur l'autre cycle.

5. Composé selon une ou plusieurs des revendications 1 à 4, choisi parmi les composés des formules (19), (20) et (21) : 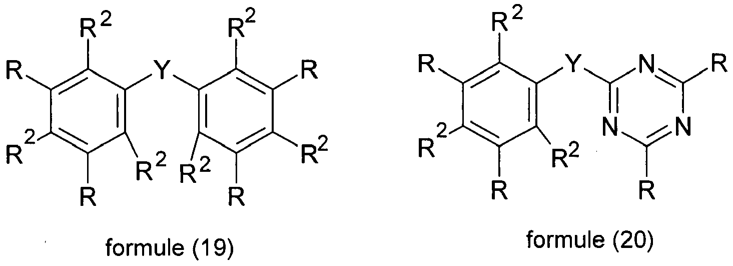 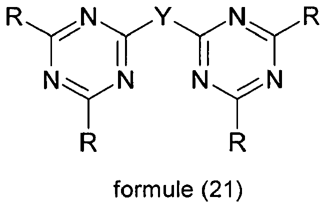 dans lesquelles Ar, R³ et R⁴ sont comme défini selon la revendication 1 et ce qui suit s'applique aux autres symboles utilisés :
Y est C=O ou C(R¹)₂ ;
R est, pour chaque occurrence, de manière identique ou différente, un système de cycle aromatique ou hétéroaromatique choisi parmi le groupe constitué par phényle, o-biphényle, m-biphényle, p-biphényle, o-terphényle, m-terphényle, p-terphényle, 3,5-(diphényl)phényle, m-quaterphényle, 1-naphtyle, 2-naphtyle, anthracényle, phénylanthracényle, 1- ou 2-naphtylanthracényle, binaphthyle, pyrényle, fluoranthényle, 2-, 3-, 4-, 5-, 6- ou 7-benzanthracényle, N-benzimidazolyle, phényl-N-benzimidazolyle, N-phénylbenzimidazolyle ou phényl-N-phénylbenzimidazolyle, en particulier choisi parmi les formules (2) à (16) selon la revendication 2 ou un groupe N(Ar)₂ choisi parmi les formules (17) et (18) selon la revendication 3, C(=O)Ar ou P(=O)Ar_{2 ;}
R¹ est, pour chaque occurrence, de manière identique ou différente, H, un groupe alkyle linéaire comportant 1 à 10 atome(s) de C, de façon préférable comportant 1 à 6 atome(s) de C, en particulier méthyle, ou un groupe alkyle ramifié ou cyclique comportant 3 à 10 atomes de C, de façon préférable comportant 3 à 6 atomes de C ; une pluralité de groupes R¹ ici peuvent former un système de cycle les uns avec les autres ;
R² est H.

6. Composé selon une ou plusieurs des revendications 1 à 5 de la formule (22) : 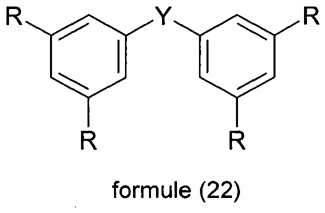 dans laquelle Ar, R³ et R⁴ sont comme défini ci avant, et ce qui suit s'applique aux autres symboles utilisés :
Y est C=O, CH₂ ou C(alkyl)₂, où alkyl représente un groupe alkyle comportant 1 à 6 atome(s) de C, en particulier méthyle ;
R est, pour chaque occurrence, de manière identique ou différente, un système de cycle aromatique ou hétéroaromatique choisi parmi le groupe constitué par phényle, o-biphényle, m-biphényle, p-biphényle, o-terphényle, m-terphényle, p-terphényle, 3,5-(diphényl)phényle, m-quaterphényle, 1-naphtyle, 2-naphtyle, anthracényle, phénylanthracényle, 1- ou 2-naphtylanthracényle, binaphtyle, pyrényle, fluoranthényle, 2-, 3-, 4-, 5-, 6- ou 7-benzanthracényle, N-benzimidazolyle, phényl-N-benzimidazolyle, N-phénylbenzimidazolyle ou phényl-N-phénylbenzimidazolyle, en particulier choisi parmi les formules (2) à (16) selon la revendication 2 ou un groupe N(Ar)₂, de façon préférable choisi parmi les formules (17) et (18) selon la revendication 3, ou C(=O)Ar ou P(=O)Ar₂.

7. Composé selon une ou plusieurs des revendications 1 à 6, **caractérisé en ce que** le symbole Ar représente, de manière identique ou différente pour chaque occurrence, un système de cycle aromatique comportant 6 à 30 atomes de cycle aromatique, en particulier choisi parmi le groupe constitué par phényle, o-biphényle, m-biphényle, p-biphényle, o-terphényle, m-terphényle, p-terphényle, 3,5-(diphényl)-phényle, m-quaterphényle, 1-naphtyle, 2-naphtyle, anthracényle, phénylanthracényle, 1- ou 2-naphtylanthracényle, binaphtyle, pyrényle, fluoranthényle, 2-, 3-, 4-, 5-, 6- ou 7-benzanthracényle, N-benzimidazolyle, phényl-N-benzimidazolyle, N-phénylbenzimidazolyle ou phényl-N-phénylbenzimidazolyle.

8. Procédé pour la préparation d'un composé selon une ou plusieurs des revendications 1 à 7, comprenant le couplage d'un bis(3,5-dibromobenzophénone) substitué ou non substitué sur un acide boronique aromatique ou hétéroaromatique ou un dérivé d'acide boronique correspondant avec une catalyse par métal ou sur un amine aromatique primaire ou secondaire avec catalyse de métal ou sur un cyanure de métal avec catalyse de métal.

9. Solution comprenant au moins un composé selon une ou plusieurs des revendications 1 à 7 et au moins un solvant organique.

10. Utilisation d'un composé selon une ou plusieurs des revendications 1 à 7 dans un dispositif électronique, en particulier dans un dispositif électroluminescent organique.

11. Dispositif électronique organique, en particulier choisi parmi le groupe constitué par des dispositifs électroluminescents organiques (OLED, PLED), des transistors à effet de champ organiques (O-FET), des transistors à film mince organiques (O-TFT), des transistors à émission de lumière organiques (O-LET), des circuits intégrés organiques (O-IC), des cellules solaires organiques (O-SC), des dispositifs à extinction de champ organiques (O-FQD), des cellules électrochimiques à émission de lumière (LEC), des diodes laser organiques (O-laser) et des photorécepteurs organiques, comprenant au moins un composé selon une ou plusieurs des revendications 1 à 7.

12. Dispositif électroluminescent organique selon la revendication 11, **caractérisé en ce que** le composé selon une ou plusieurs des revendications 1 à 7 est utilisé en tant que matériau de matrice pour un composé fluorescent ou phosphorescent dans une couche d'émission ou en tant que matériau de transport de trous ou en tant que matériau d'injection de trous ou en tant que matériau de blocage d'électrons ou en tant que matériau de blocage d'excitons ou en tant que matériau de transport d'électrons ou en tant que matériau de blocage de trous.
